# EUROPEAN PATENT SPECIFICATION

(11) **EP 0 958 282 B1**
(45) Date of publication and mention of the grant of the patent: **21.07.2004**
(21) Application number: 98900635.8
(22) Date of filing: 29.01.1998
(51) Int. Cl.: C07D 213/73, A61K 31/44, C07D 401/12

(54) **2-AMINO-6-(2-SUBSTITUTED-4-PHENOXY)-SUBSTITUTED-PYRIDINES**
2-AMINO-6-(2-SUBSTITUIERTE-4-PHENOXY)-SUBSTITUIERTE-PYRIDINE
PYRIDINES A SUBSTITUTION 2-AMINO-6-(2-SUBSTITUE-4-PHENOXY)

(30) Priority: 10.02.1997 US 37533 P
(43) Date of publication of application: 24.11.1999
(73) Proprietor: Pfizer Products Inc., Groton, CT 06340-5146 (US)
(72) Inventor: LOWE, John, Adams, III, Stonington, CT 06475 (US); NOWAKOWSKI, Jolanta, Old Saybrook, CT 06475 (US); VOLKMANN, Robert, Alfred, Mystic, CT 06355 (US)
(74) Representative: Simpson, Alison Elizabeth Fraser
(86) International application number: PCT/IB1998/000112
(87) International publication number: WO 1998/034919

(56) References cited:
- WO-A-94/14780
- WO-A-95/11231
- WO-A-96/18616
- WO-A-97/36871
- HUSSAINI F A ET AL: "SYNTHESIS OF SUBSTITUTED 2-AMINOPYRIDINES: A VERSATILE APPROACH" JOURNAL OF CHEMICAL RESEARCH. SYNOPSES, no. 3, 1 January 1994, page 86 XP000566748

## Description

The present invention relates to certain 2-amino-6-(2-substituted-4-phenoxy)-substituted-pyridines that exhibit activity as nitric oxide synthase (NOS) inhibitors, to pharmaceutical compositions containing them and to their use in the treatment and prevention of central nervous system disorders, inflammatory disorders, septic shock and other disorders.

There are three known isoforms of NOS - an inducible form (I-NOS) and two constitutive forms referred to as, respectively, neuronal NOS (N-NOS) and endothelial NOS (E-NOS). Each of these enzymes carries out the conversion of arginine to citrulline while producing a molecule of nitric oxide (NO) in response to various stimuli. It is believed that excess nitric oxide (NO) production by NOS plays a role in the pathology of a number of disorders and conditions in mammals. For example, NO produced by I-NOS is thought to play a role in diseases that involve systemic hypotension such as toxic shock and therapy with certain cytokines. It has been shown that cancer patients treated with cytokines such as interleukin 1 (IL-1), interleukin 2 (IL-2) or tumor necrosis factor (TNF) suffer cytokine-induced shock and hypotension due to NO produced from macrophages, i.e., inducible NOS (I-NOS), see Chemical & Engineering News, Dec. 20, p 33, (1993). I-NOS inhibitors can reverse this. It is also believed that I-NOS plays a role in the pathology of diseases of the central nervous system such as ischemia. For example, inhibition of I-NOS has been shown to ameliorate cerebral ischemic damage in rats, see Am. J. Physiol., **268**, p. R286 (1995)). Suppression of adjuvant induced arthritis by selective inhibition of I-NOS is reported in Eur. J. Pharmacol., **273**, p. 15-24 (1995).

NO produced by N-NOS is thought to play a role in diseases such as cerebral ischemia, pain, and opiate tolerance. For example, inhibition of N-NOS decreases infarct volume after proximal middle cerebral artery occlusion in the rat, see J. Cerebr. Blood Flow Metab., **14**, p. 924-929 (1994). N-NOS inhibition has also been shown to be effective in antinociception, as evidenced by activity in the late phase of the formalin-induced hindpaw licking and acetic acid-induced abdominal constriction assays, see Br. J. Pharmacol., **110**, p. 219-224 (1993). Finally, opioid withdrawal in rodents has been reported to be reduced by N-NOS inhibition, see Neuropsychopharmacol., **13**, p. 269-293 (1995).

Other NOS inhibitors and their utility as pharmaceutical agents in the treatment of CNS and other disorders are referred to in United States Provisional Application 60/032,793, filed December 6, 1996, and United States Provisional Application 60/014,343, filed March 29, 1996.

### Summary of the Invention

This invention relates to compounds of the formula 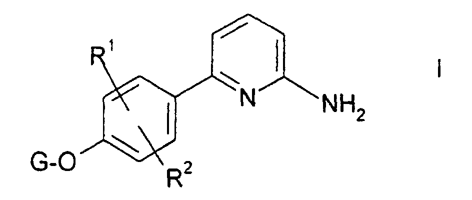 wherein R¹ and R² are selected, independently, from hydrogen, halo, hydroxy, ( C₁-C₆)alkoxy, (C₁-C₇)alkyl, (C₂-C₆)alkenyl, and (C₂ - C₁₀)alkoxyalkyl; and
G is selected from hydrogen, (C₁-C₆)alkyl, (C₁-C₆)alkoxy-(C₁-C₃)alkyl, aminocarbonyl-(C₁-C₃)alkyl-, (C₁-C₃) alkylaminocarbonyl -(C₁-C₃) alkyl-, di-[(C₁-C₃)alkyl]aminocarbonyl-(C₁-C₃)alkyl-, and N(R³)(R⁴)(C₀-C₄)alkyl-, wherein R³ and R⁴ are selected, independently, from hydrogen, (C₁-C₇) alkyl, tetrahydronaphthalene and aralkyl, wherein the aryl moiety of said aralkyl is phenyl or naphthyl and the alkyl moiety is straight, cyclic or branched and contains from 1 to 6 carbon atoms, and wherein said (C₁-C₇) alkyl and said tetrahydronaphthalene and the aryl moiety of said aralkyl may optionally be substituted with from one to three substituents, preferably from zero to two substituents, that are selected, independently, from halo, nitro, hydroxy, cyano, amino, (C₁-C₄)alkoxy, and (C₁-C₄)alkylamino;
or R³ and R⁴ form, together with the nitrogen to which they are attached, a piperazine, piperidine, morpholine, azetidine or pyrrolidine ring or a saturated or unsaturated azabicyclic ring system containing from 6 to 14 ring members, from 1 to 3 of which are nitrogen, from zero to two of which are oxygen, and the rest of which are carbon;
and wherein said piperazine, piperidine, azetidine and pyrrolidine rings and said azabicyclic ring systems may optionally be substituted with one or more substituents, preferably with from zero to two substituents, that are selected, independently, from (C₁-C₆)alkyl, amino, (C₁-C₆) alkylamino, [di-(C₁-C₆)alkyl]amino, phenyl substituted 5 to 6 membered heterocyclic rings containing from 1 to 4 ring nitrogen atoms, benzoyl, benzoylmethyl, benzylcarbonyl, phenylaminocarbonyl, phenylethyl and phenoxycarbonyl, and wherein the phenyl moieties of any of the foregoing substituents may optionally be substituted with one or more substituents, preferably with from zero to two substituents, that are selected, independently, from halo, (C₁-C₃)alkyl, (C₁-C₃)alkoxy, nitro, amino, cyano, CF₃ and OCF₃;
and wherein said piperazine, piperidine, azetidine and pyrrolidine rings and said azabicyclic ring systems may be attached to -(C₀-C₄)alkyl-O- (wherein the oxygen of said -(C₀-C₄)alkyl-O- is the oxygen atom depicted in structural formula I) at a nitrogen atom of the NR³R⁴ ring or at any other atom of such ring having an available bonding site;
or G is a group of the formula A 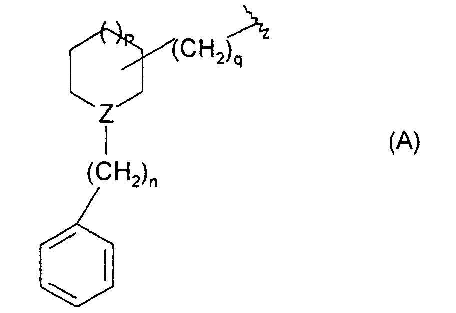 wherein Z is nitrogen or CH, n is zero or one, q is zero, one, two or three and p is zero, one or two;
and wherein the 2-amino piperidine ring depicted in structure I above may optionally be replaced with 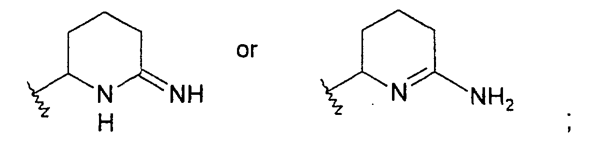 and the pharmaceutically acceptable salts of such compounds

The present invention also relates to the pharmaceutically acceptable acid addition salts of compounds of the formula I. The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1-methylene-bis-(2-hydroxy-3-naphthoate)] salts.

The term "alkyl", as used herein, unless otherwise indicated, includes saturated monovalent hydrocarbon radicals having straight, branched or cyclic moieties or combinations thereof.

The term "one or more substituents", as used herein, refers to a number of substituents that equals from one to the maximum number of substituents possible based on the number of available bonding sites.

The term "halo", as used herein, unless otherwise indicated, includes chloro, fluoro, bromo and iodo.

Examples of compounds of this invention are compounds of the formula I, and their pharmaceutically acceptable salts, wherein G is N(R³)(R⁴)(C₀-C₄) alkyl and N(R³)(R⁴) is amino, dimethylamino, methylbenzylamino, (C₁-C₄)alkylamino, di-[(C₁-C₄)alkyl]amino or one of the following groups:

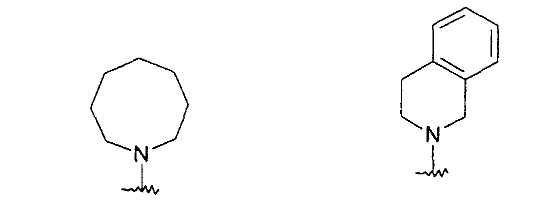

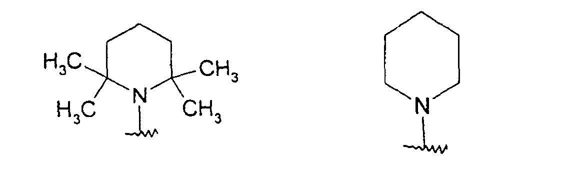

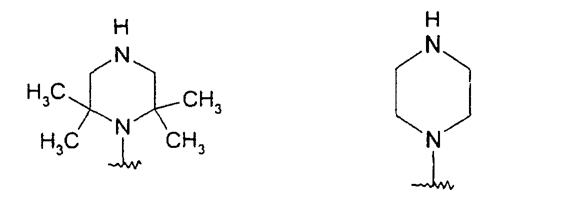

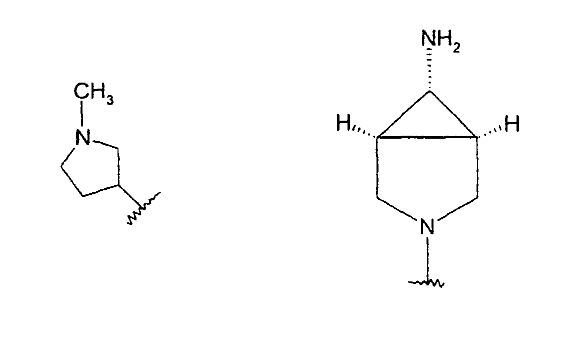

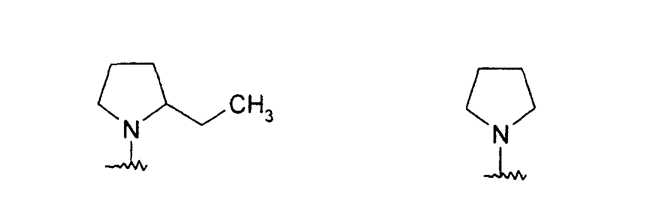

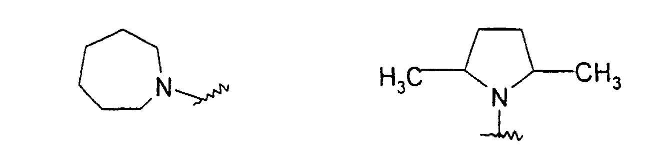

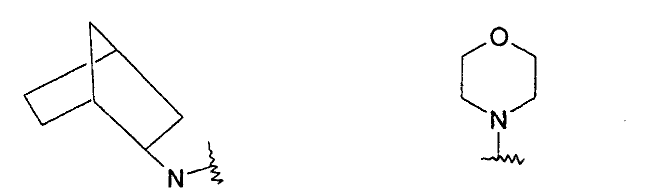

Preferred compounds of the formula I include those wherein R² is hydrogen and R¹ is (C₁ - C₃)alkoxy and is in the ortho position relative to the pyridine ring of formula I.

Other embodiments of this invention relate to compounds of the formula I wherein G is a group of the formula A, as defined above, wherein Z is nitrogen.

Other embodiments of this invention relate to compounds of the formula I wherem R¹ and R² are selected, independently, from (C₁-C₂)alkoxy.

Other embodiments of the invention relate to compounds of the formula I wherein G is a group of the formula A, as defined above, wherein Z is nitrogen, each of p and n is one and q is two.

Other embodiments of this invention relate to compounds of the formula I wherein the 2-aminopyridine ring depicted in formula I above, is present.

The present invention also relates to a pharmaceutical composition for treating or preventing a condition selected from the group consisting of migraine inflammatory diseases (e.g., asthma, psoriasis, eczema, arthritis) stroke, acute and chronic pain, hypovolemic shock, traumatic shock, reperfusion injury, Crohn's disease, ulcerative colitis, septic shock, multiple sclerosis, AIDS associated dementia, neurodegenerative diseases, neuron toxicity, Alzheimer's disease, chemical dependencies and addiction (e.g., dependencies on drugs, alcohol and nicotine), emesis, epilepsy, anxiety, psychosis, head trauma, adult respiratory distress syndrome (ARDS), morphine induced tolerance and withdrawal symptoms, inflammatory bowel disease, osteoarthntis, rheumatoid arthritis, ovulation, dilated cardiomyopathy, acute spinal cord injury, Huntington's disease, Parkinson's disease, glaucoma, macular degeneration, diabetic neuropathy, diabetic nephropathy and cancer in a mammal, including a human, comprising an amount of a compound of the formula I, or a pharmaceutically acceptable salt thereof that is effective in treating or preventing such condition, and a pharmaceutically acceptable carrier.

The present invention also relates to a method of treating or preventing a condition selected from the group consisting of migraine inflammatory diseases (e.g., asthma, psoriasis, eczema, arthritis), stroke, acute and chronic pain, hypovolemic shock, traumatic shock, reperfusion injury, Crohn's disease, ulcerative colitis, septic shock, multiple sclerosis, AIDS associated dementia, neurodegenerative diseases, neuron toxicity, Alzheimer's disease, chemical dependencies and addictions (e.g., dependencies on drugs, alcohol and nicotine), emesis, epilepsy, anxiety, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, Parkinson's disease, glaucoma, macular degeneration, diabetic neuropathy, diabetic nephropathy and cancer in a mammal, including a human, comprising administering to said mammal an amount of a compound of the formula I, or a pharmaceutically acceptable salt thereof, that is effective in treating or preventing such condition.

The present invention also relates to a pharmaceutical composition for inhibiting nitric oxide synthase (NOS) in a mammal, including a human, comprising an NOS inhibiting effective amount of a compound of the formula I, or a pharmaceutically acceptable salt thereof and a pharmaceutically acceptable carrier.

The present invention also relates to a method of inhibiting NOS in a mammal, including a human, comprising administering to said mammal a NOS inhibiting effective amount of a compound of the formula I, or a pharmaceutically acceptable salt thereof.

The present invention also relates to a pharmaceutical composition for treating or preventing a condition selected from the group consisting of migraine, inflammatory diseases (e.g., asthma, psoriasis, arthritis, eczema), stroke, acute and chronic pain, hypovolemic shock, traumatic shock, reperfusion injury, Crohn's disease, ulcerative colitis, septic shock, multiple sclerosis, AIDS associated dementia, neurodegenerative diseases, neuron toxicity, Alzheimer's disease, chemical dependencies and addictions (e.g., dependencies on drugs, alcohol and nicotine), emesis, epilepsy, anxiety, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, glaucoma, macular degeneration, diabetic neuropathy, diabetic nephropathy and cancer in a mammal, including a human, comprising a NOS inhibiting effective amount of a compound of the formula I, or a pharmaceutically acceptable salt thereof and a pharmaceutically acceptable carrier.

The present invention also relates to a method of treating or preventing a condition selected from the group consisting of migraine, inflammatory diseases (e.g., asthma, psoriasis, eczema, arthritis), stroke, acute and chronic pain, hypovolemic shock, traumatic shock, reperfusion injury, Crohn's disease, ulcerative colitis, septic shock, multiple sclerosis. AIDS associated dementia, neurodegenerative diseases, neuron toxicity, Alzheimer's disease, chemical dependencies and addictions (e.g., dependencies on drugs, alcohol or nicotine), emesis, epilepsy, anxiety, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, Parkinson's disease, glaucoma, macular degeneration, diabetic neuropathy, diabetic nephropathy and cancer in a mammal, including a human, comprising administering to said mammal a NOS inhibiting effective amount of a compound of the formula II, or a pharmaceutically acceptable salt thereof.

Compounds of formula I have chiral centers and therefore may exist in different enantiomeric and diastereomeric forms. This invention relates to all optical isomers and all stereoisomers of compounds of the formula I and mixtures thereof, and to all pharmaceutical compositions and methods of treatment defined above that contain or employ them, respectively.

Formula I above includes compounds identical to those depicted but for the fact that one or more hydrogen, carbon or other atoms are replaced by isotopes thereof. Such compounds may be useful as research and diagnostic tools in metabolism pharmacokinetic studies and in binding assays.

This invention also relates to compounds of the formula 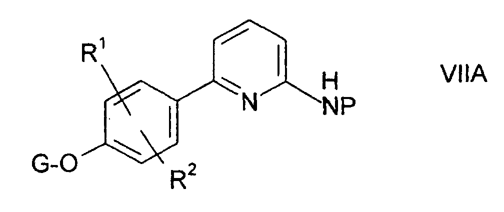 wherein R¹, R² and G are defined as above for compounds of the formula I, and P is a nitrogen protecting group such as trityl, acetyl, benzoyl, trimethylacetyl, t-butoxycarbonyl, benzyloxycarbonyl, or another appropriate nitrogen protecting group, and wherein P can form a ring with the protected nitrogen, in which case the hydrogen that is depicted above as being attached to such nitrogen is absent.

Such compounds are useful as intermediates in the synthesis of the pharmaceutically active compounds of formula I.

This invention also relates to compounds of the formula 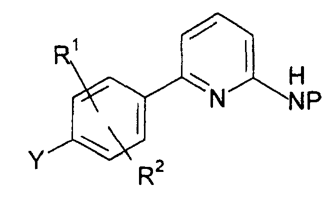 wherein R¹, R² and P are defined as above and Y is fluoro or benzyloxy. Such compounds are useful as intermediates in the synthesis of the pharmaceutically active compounds of formula I.

### Detailed Description of the Invention

The compounds of the formula I may be prepared as described in the following reaction schemes and discussion. Unless otherwise indicated, R¹, R², R³, R⁴, R⁵, R⁶, R⁷, R⁸ and R⁹ and structural formula I in the reaction schemes and discussion that follow are defined as above.

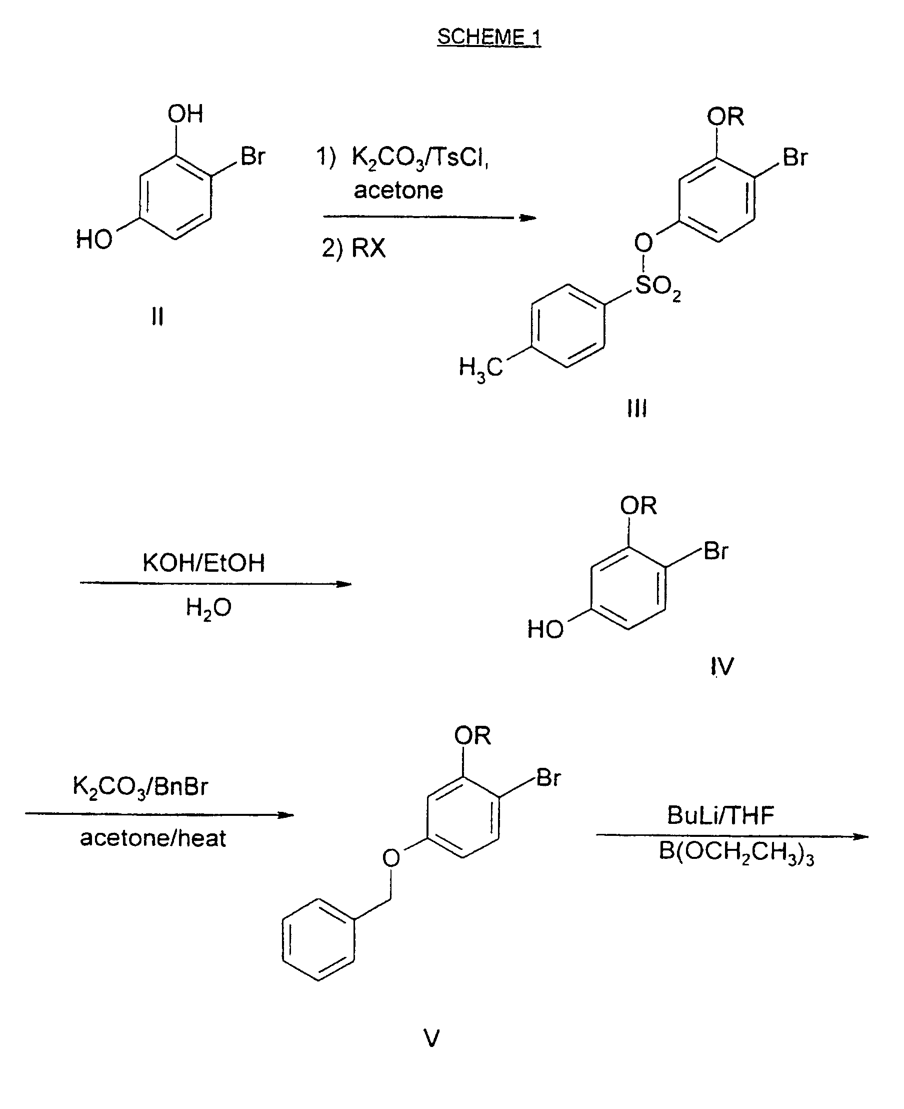

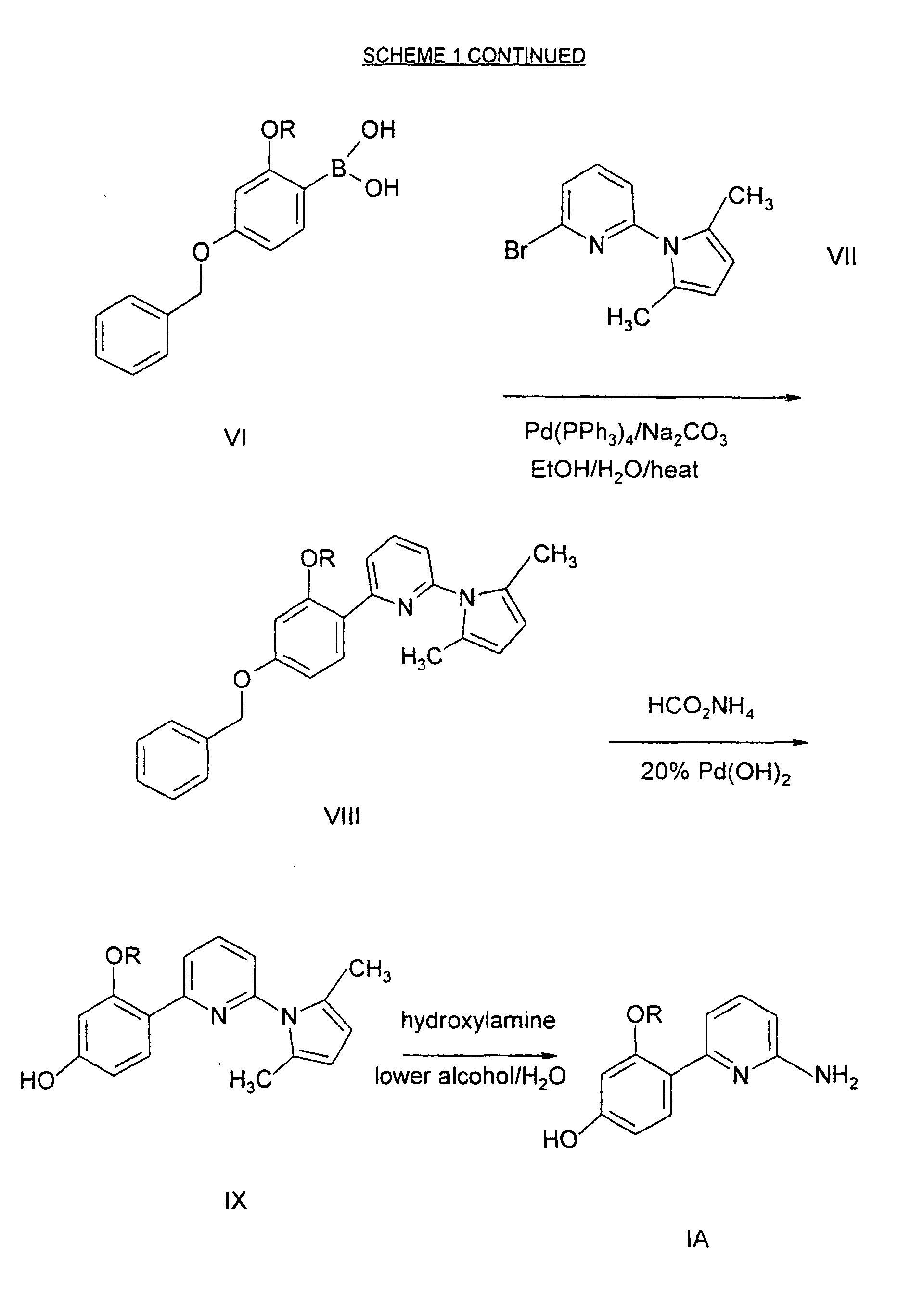

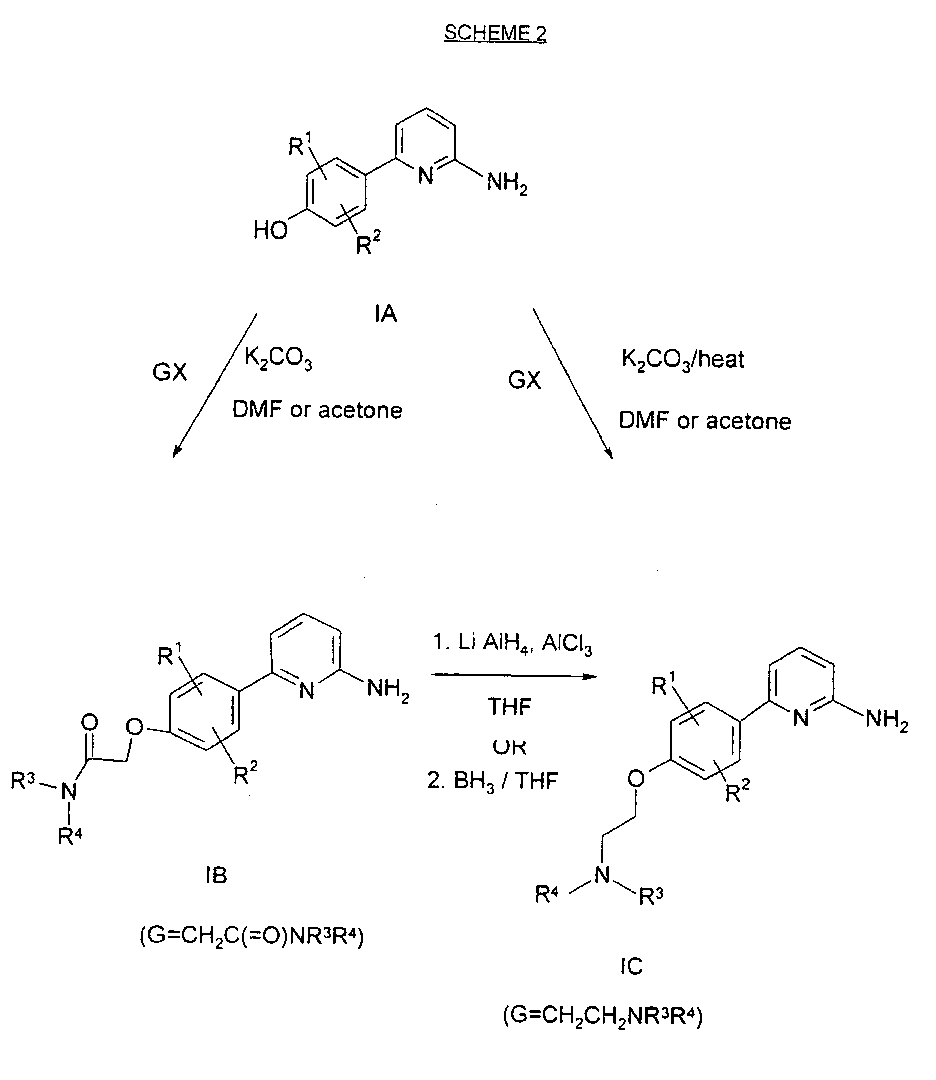

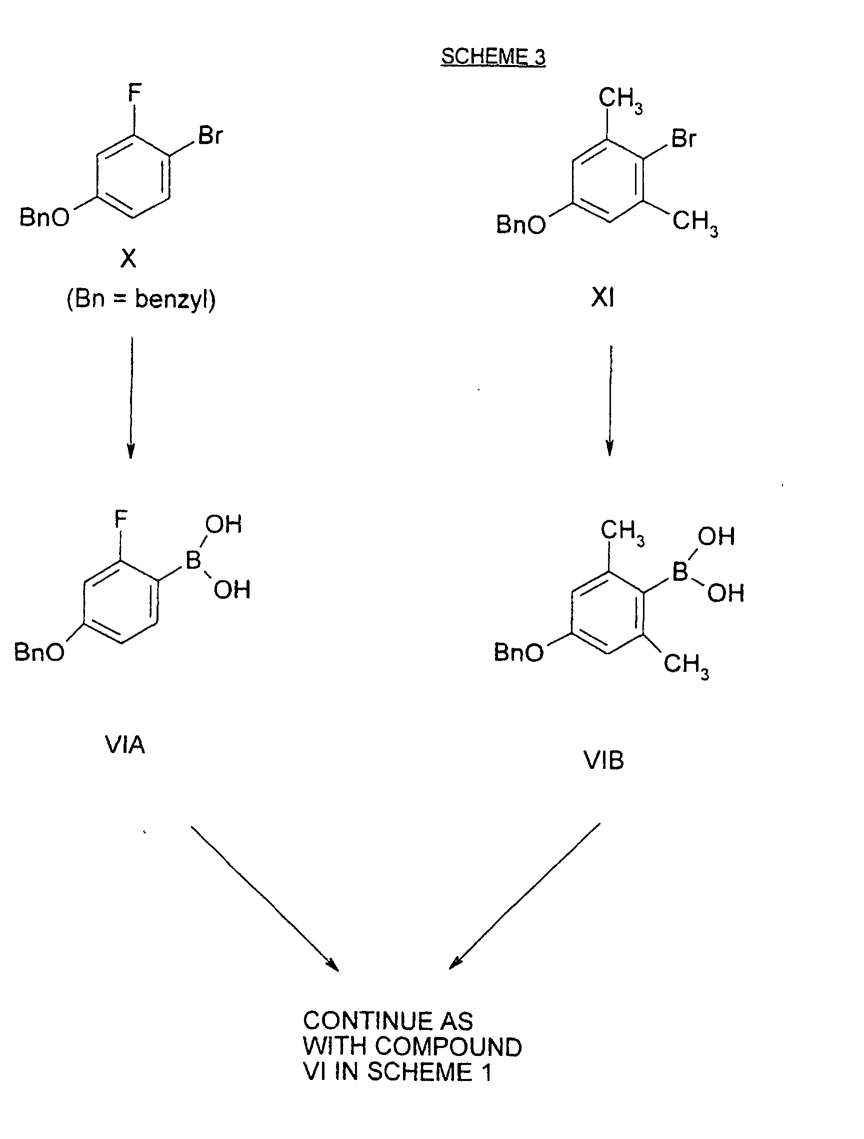

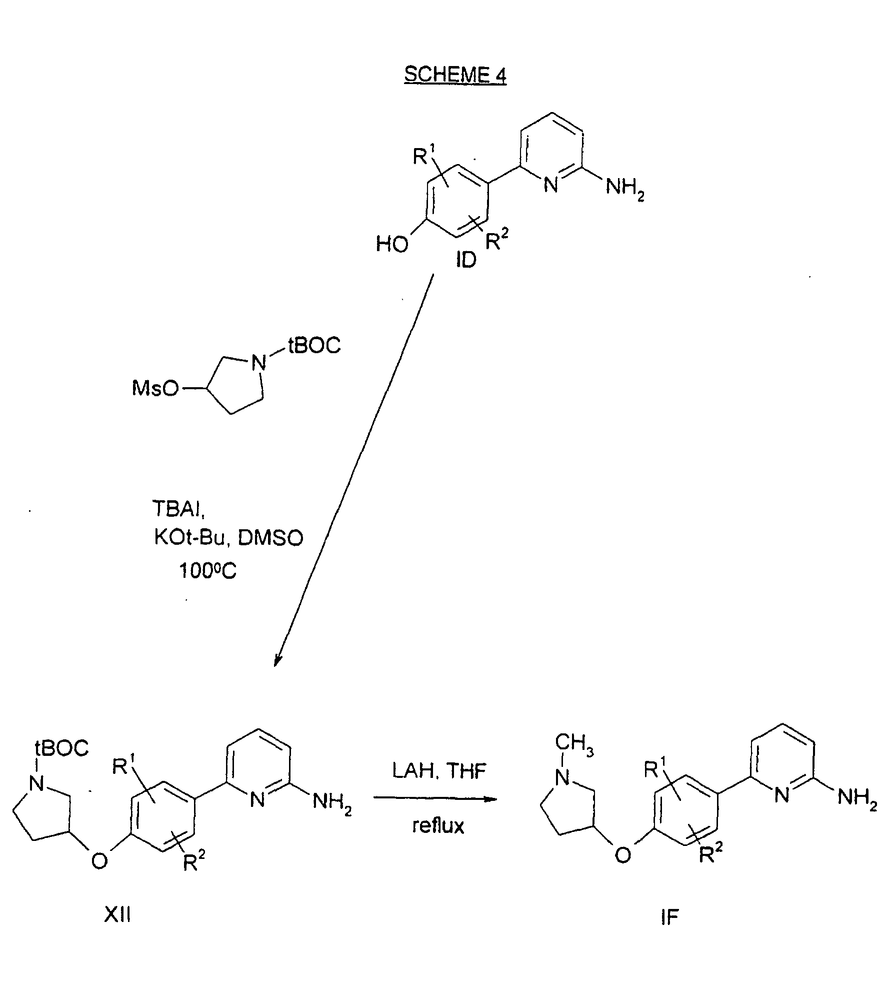

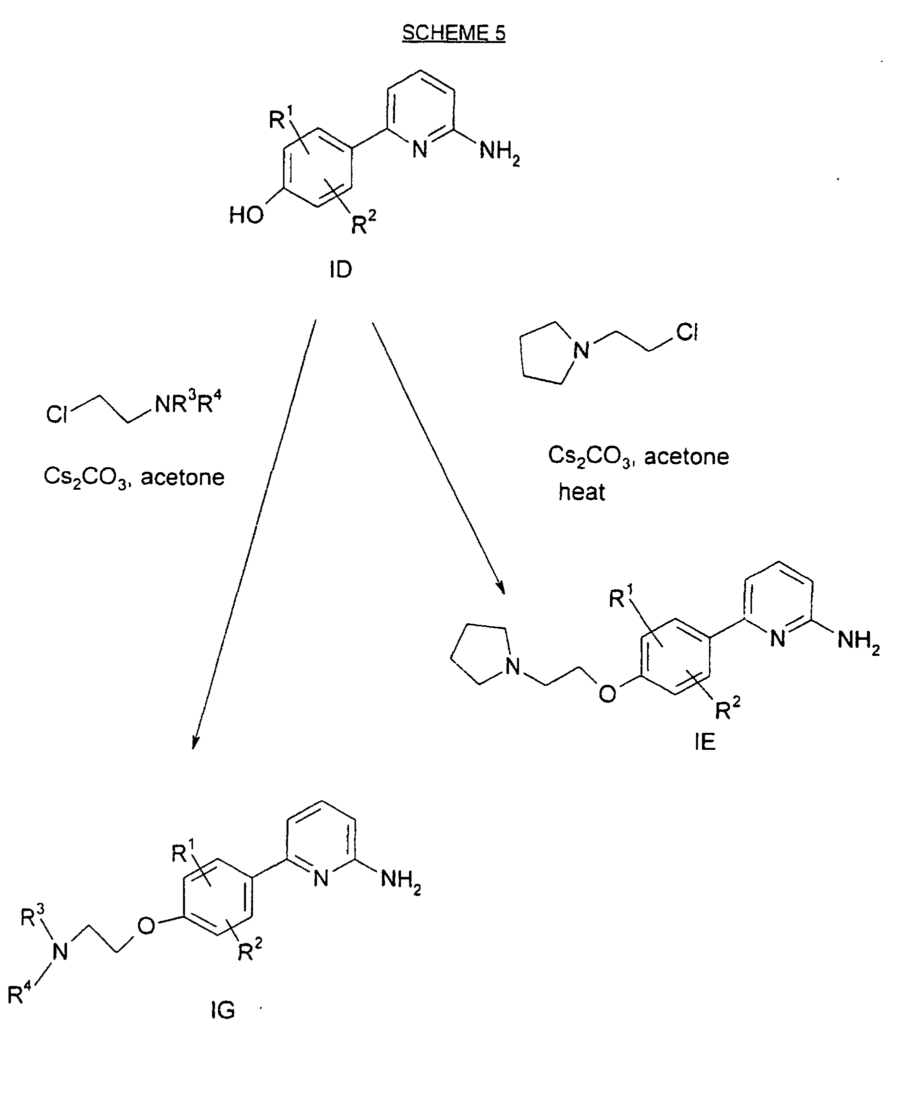

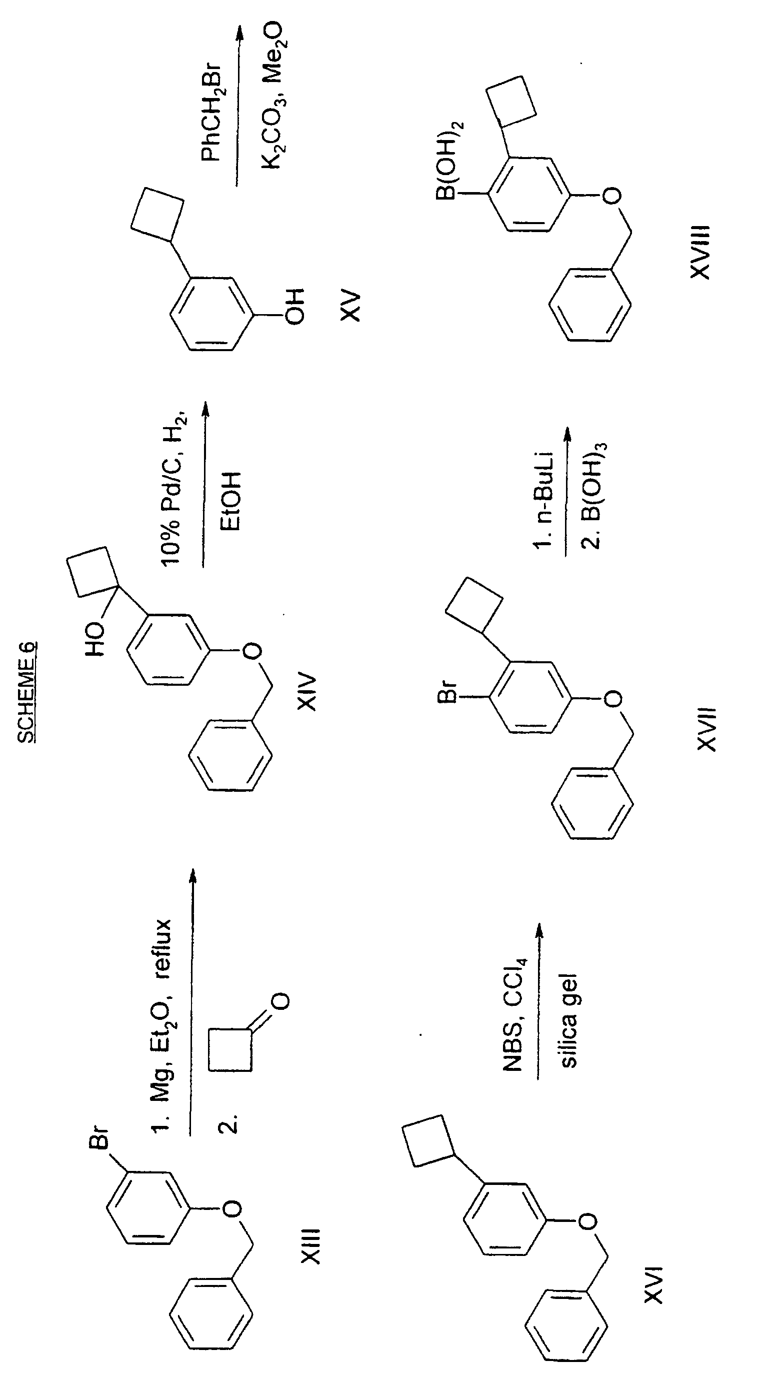

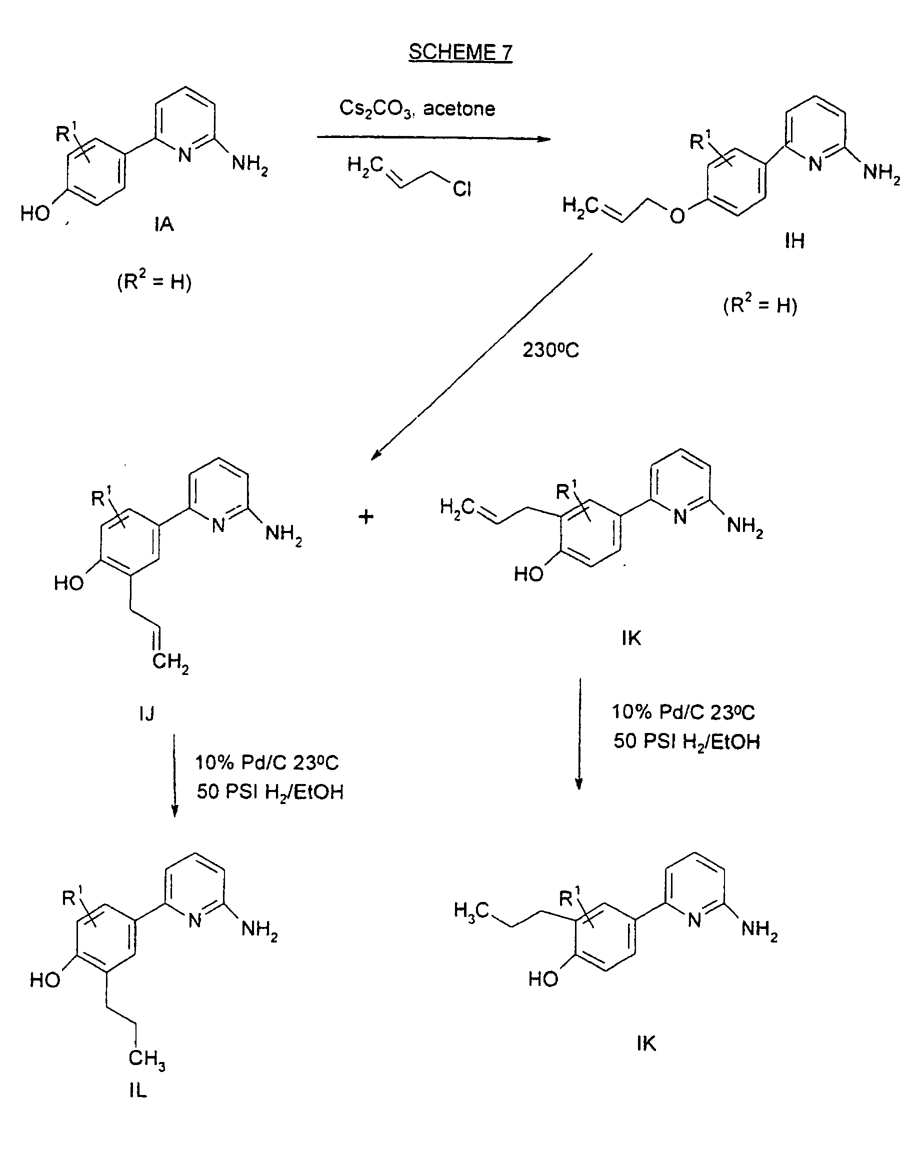

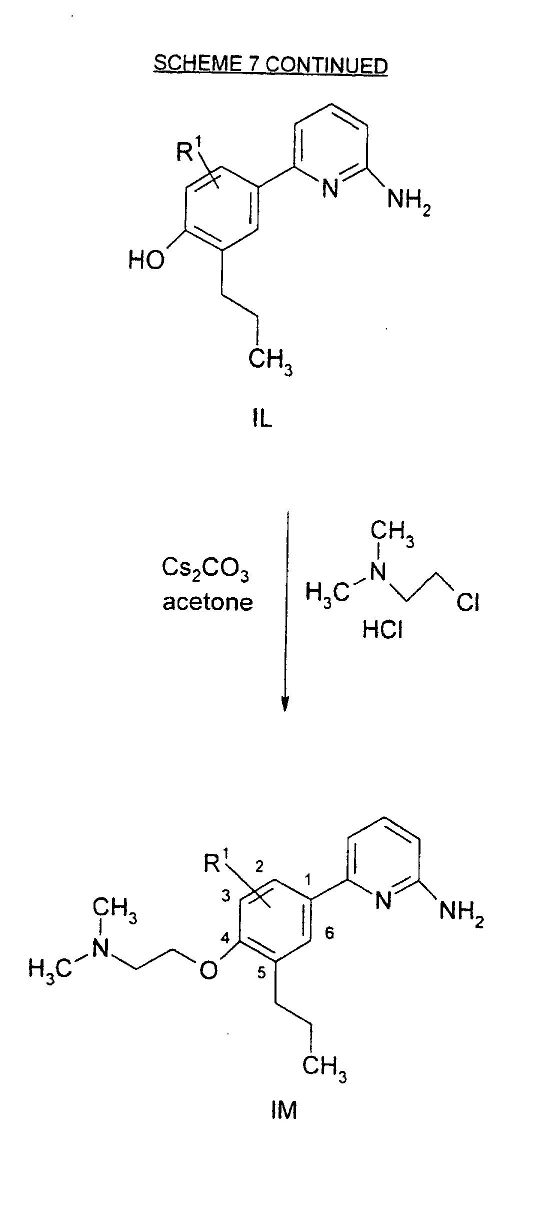

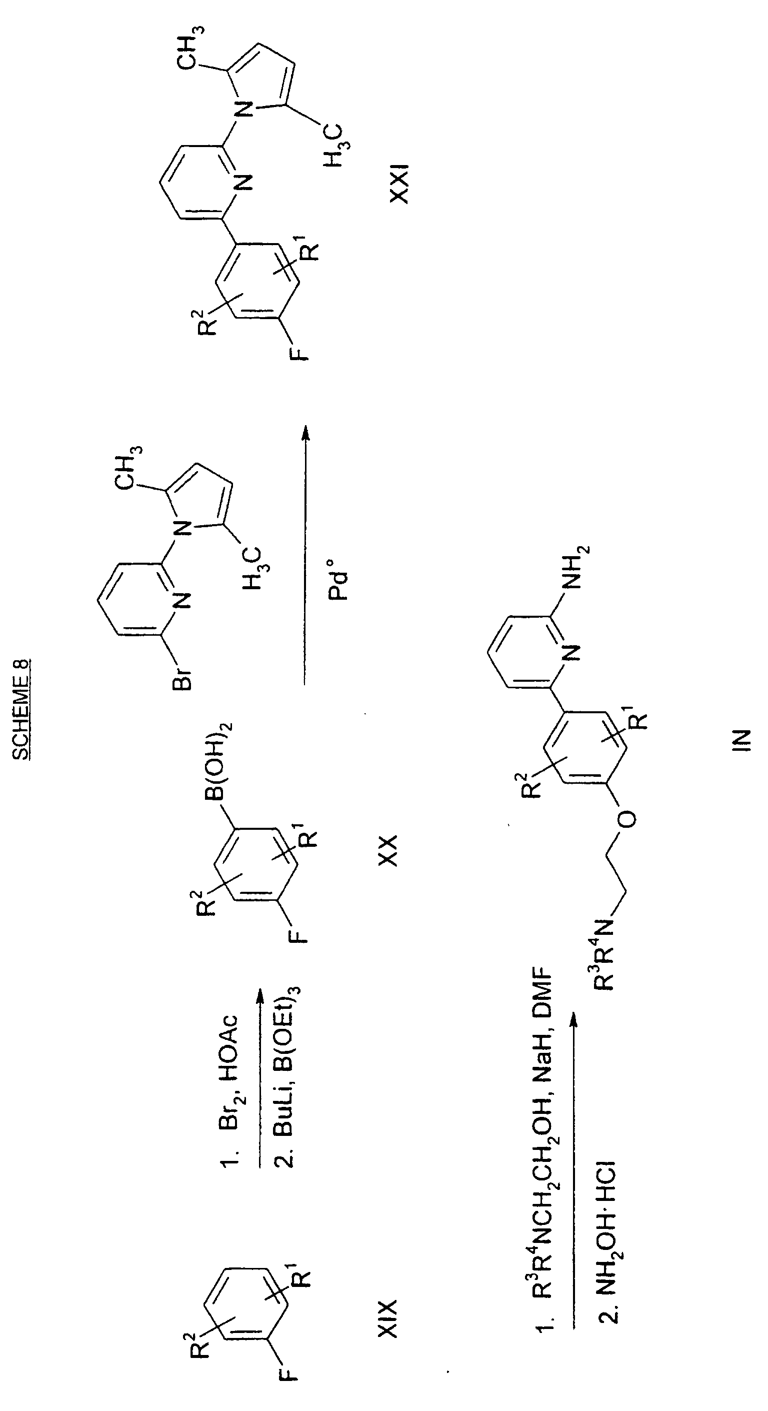

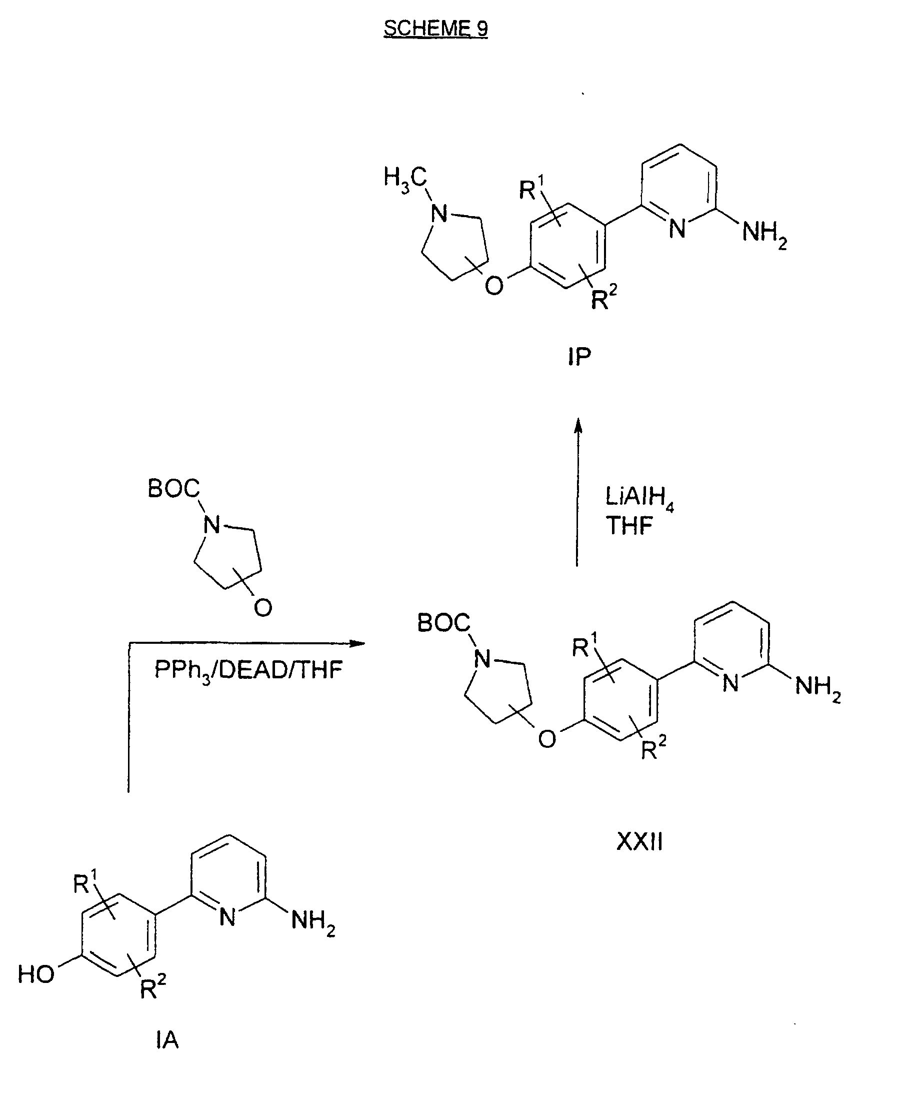

Scheme I illustrates a method for prepanng compounds of the formula I wherem G is hydrogen, R¹ is -OR wherein R is (C₁-C₆)alkyl and R² is hydrogen. These compounds are referred to in Scheme I as compounds of the formula "IA"

Referring to Scheme 1, the compound of formula II is reacted with excess potassium carbonate and one equivalent of tosyl chloride in acetone, at a temperature from about 0°C to about 80°C, preferably at the reflux temperature of the reaction mixture. A compound of the formula RX, wherein R is (C₁-C₆)alkyl and X is iodo, chloro or bromo, is then added to the reaction mixture and the mixture is allowed to react at a temperature ranging from about 0°C to about 80°C, preferably at the reflux temperature of the mixture. This reaction yields a compound of the formula III. The compound of formula III is then converted into the corresponding compound of formula IV by reacting it with potassium hydroxide in ethanol, using water as the solvent. This reaction can be carried out at a temperature from about room temperature to about the reflux temperature of the reaction mixture. Preferably, the reaction mixture is heated to reflux and allowed to react at that temperature.

The compound of formula IV is then reacted with potassium carbonate and benzyl bromide in acetone, at a temperature from about room temperature to about 80°C, to form the corresponding compound of formula V. Preferably, the reaction is conducted at about the reflux temperature. Reaction of the resulting compound of formula V with butyl lithium in tetrahydrofuran (THF) at about -78°C, followed by the addition of triethyl borate and allowing the reaction mixture to warm to ambient temperature, yields the corresponding phenylboronic acid derivative of formula VI.

Reacting the phenylboronic acid derivative of formula VI with 2-bromo-6-(2,5-dimethyl-pyrrol-1-yl)-pyridine (VII), sodium carbonate and tetrakis(triphenylphosphine)palladium(0) in ethanol/water or THF/water, at a temperature from about room temperature to about the reflux temperature of the reaction mixture, preferably at about the reflux temperature, yields the corresponding compound of formula VIII. Alternatively, the reactant of formula VII can be replaced with another compound of the formula 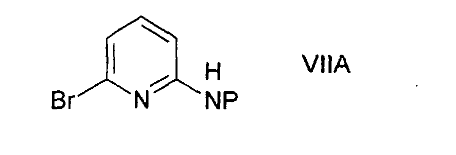 wherein P is a nitrogen protecting group such as trityl, acetyl, benzyl, trimethylacetyl, t-butoxycarbonyl, benzyloxycarbonyl, trichloroethyloxycarbonyl or another appropriate nitrogen protecting group and wherein the hydrogen that is bonded to the protected nitrogen is absent when P is a protecting group that forms a ring with the protected nitrogen, as in the case of P = 2,5-dimethylpyrrolyl. Such protecting groups are well known to those of skill in the art. The above compounds of the formula VIIA are either commercially available, known in the scientific literature or easily obtaining using well known methods and reagents.

The benzyl substituent can be removed from the compound of formula VIII by reacting such compound with ammonium formate in water or a lower alcohol solvent, or in a mixture of one or more of these solvents, at a temperature from about room temperature to about the reflux temperature of the reaction mixture. This reaction is preferably carried out at the reflux temperature in the presence of about 20% palladium hydroxide on carbon. The resulting compound of formula IX is then converted into the desired compound of formula IA by reacting it with hydroxylamine in a solvent selected from water, lower alcohols and mixtures of these solvents, at a temperature from about room temperature to about the reflux temperature of the solvent, preferably at about the reflux temperature.

The procedure of Scheme 1 can also be used to make compounds of the formula I wherein R¹ and R² are other than as specified above and depicted in the scheme. This can be accomplished by using a compound of the formula 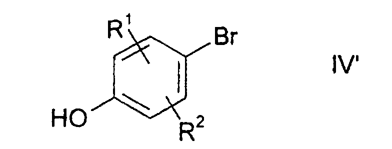 as the starting material and then carrying out the series of reactions, as described above, that are represented in Scheme 1 as reactions IV→V→VI→VII→VIII→IX→IA.

Scheme 2 illustrates a method for preparing compounds of the formula I wherein G is hydrogen into the corresponding compounds of formula I wherein G is other than hydrogen.

Referring to Scheme 2, a compound of the formula IA can be converted into the corresponding compound of formula IC by reacting it with the compound of the formula GX, wherein X is iodo, chloro, or bromo, and G is CH₂CH₂NR³R⁴, and potassium carbonate in either dimethylformamide (DMF) or acetone at a temperature from about room temperature to about the reflux temperature of the mixture, preferably at about the reflux temperature. Compounds of the formulae IC can also be formed, as illustrated in Scheme 2, as by first preparing the corresponding compounds of formula IB and then converting them, if so desired, into the corresponding compounds of formula IC. Compounds of formula IB can be formed by reacting the corresponding compounds of formula IA with a compound of the formula GX, wherein X is defined as above and G is CH₂C(=O)NR³R⁴, and potassium carbonate, in either DMF or acetone, at a temperature from about room temperature to about the reflux temperature of the reaction mixture. This reaction also is preferably carried out at about the reflux temperature.

The resulting compounds of formula of IB can be converted into the corresponding compounds of formula IC by reacting them with lithium aluminum hydride and aluminum chloride in a THF solvent, or with borane in THF. Other aluminum hydride reducing agents can also be used, such as diisobutyl aluminum hydride. Diborane can also be used. This reaction is generally carroid out at temperatures ranging from room temperature to about the reflux temperature of the reaction mixture, and is preferably carried out at the reflux temperature. Other appropriate sovlents include other organic ethers such as ethyl ether, dioxane and glyme, THF is preferred solvent.

Scheme 3 illustrates how certain compounds of the formula I having different substituents R¹ and R² than are depicted in the processes of Scheme 1 can be prepared. Such compounds are prepared by a process similar to that depicted in Scheme 1, with the exception that the processes of Scheme 1 involved in the synthesis of compound VI are replaced with those depicted in Scheme 3. Specifically, when R² is hydrogen and R¹ is fluoro at the ortho position, the compound of formula X is converted to the corresponding phenylboronic acid in a manner analogous to the conversion of compounds of the formula V into those of the formula VI in Scheme 1. The resulting phenylboronic acid derivative is referred to in Scheme 3 as compound VIA. Similarly, as shown in Scheme 3, compounds of the formula I wherein R¹ and R² are both methyl and are both at an ortho position relative to the pyridine ring, may be prepared by converting the compound of formula XI, as shown in Scheme 3, into the corresponding phenylboronic acid derivative designated as compound VIB, in a matter analogous to the conversion of compounds of formula V into those of the formula VI in Scheme 1. The compounds of formulas VIA and VIB can then be transformed into the desired corresponding compounds of the formula I using procedures analogous to those shown in Scheme 1.

Scheme 4 exemplifies methods of preparing compounds of the formula I wherein G is NR³R⁴ and NR³R⁴ forms an N-methylpyrrolin-2-yl ring. Compounds of the formula I wherein G is NR³R⁴ and NR³R⁴ forms other nitrogen containing rings can be prepared in an analogous fashion. Referring to Scheme 4, the compound of formula ID is allowed to react with 3-methanesulfonyloxy-pyrrolidine-1-carboxylic acid tert-butyl ester to form the compound of formula XII. Other nitrogen protecting groups such as -C(=O)OCH₂C₆H₅ and COOR (wherein R is benzyl, phenyl, t-butyl or a similar group) can be used to protect the pyrrolidine nitrogen. Also, the mesylate leaving group can be replaced with another appropriate leaving group. Preferably, a catalytic amount of tetrabutylammonium iodide (TBAI) is added to the reaction mixture. This alkylation reaction is typically carried out in the presence of an alkali metal alkoxide, preferable potassium tert-butoxide, in a high boiling polar organic solvent such as dimethylsulfoxide (DMSO) or DMF, preferably DMSO. The reaction temperature can range from about 50°C to about 100°C, and is preferably about 100°C.

Reduction of the compound of formula XII yields the compound of formula IF. This reduction is preferably accomplished using lithium alluminum hydride as the reducing agent and tetrahydrofuran (THF) or another organic ether (e.g., ethyl ether or glyme) as the solvent. Other aluminum hydride reducing agents can also be used, such as diisobutyl aluminum hydride. Diborane can also be used. The foregoing reaction is generally conducted at a temperature from about room temperature to about the reflux temperature of the reaction mixture, preferably at about the reflux temperature.

As illustrated in Scheme 5, alkylation of the compound of formula ID with 1-(2-chloroethyl)-pyrrolidine yields the compound of formula IE. This reaction is generally conducted in the present of a base such as cesium carbonate, potassium carbonate, or sodium carbonate, preferably cesium carbonate, in a solvent such as acetone, DMSO or acetonitrile, preferably acetone, at a temperature from about room temperature to about the reflux temperature, preferably at about the reflux temperature.

Compounds of the formula I wherein NR³R⁴ do not form a ring can also be prepared by the method illustrated in Scheme 5 and described above for the formation of the compound of formula IE. Structural formula IG, depicted in Scheme 5, includes such compounds.

Scheme 6 illustrates a method of preparing the benzeneboronic acid intermediates use in the syntheses described in Schemes 1 and 3 above wherein the benzene ring of the benzeneboronic acid contains a cycloalkyl substituent. Such intermediates can be used in the processes of Schemes 1 and 3 to form compounds of the formula I wherein one or both of R¹ and R² are cycloalkyl groups. Referring to Scheme 6, the compound of formula XIII is allowed to reflux, in the presence of magnesium metal, in THF or ethyl ether for about 8 hours, after which cyclobutanone is added to the reaction mixture. This reaction yields the compound of formula XIV. Reduction of the compound of formula XIV using, for example, hydrogen gas and 10% palladium on carbon, in a lower alcohol solvent such as ethanol, at a temperature of about room temperature, yields the corresponding compound of formula XV.

Reaction of the compound of formula XV with benzylbromide in the presence of a base such as potassium, cesium or sodium carbonate, in a solvent such as acetone, dichlorothane, chloroform or methylene chloride, at a temperature from about room temperature to about the reflux temperature of the reaction mixture, preferably at about the reflux temperature, yields the corresponding compound of formula XVI.

The compound of formula XVI that was formed in the above step is then brominated by reaction with N-bromosuccinamide (NBS) and silica gel in a chlorinated hydrocarbon solvent such as carbon tetrachloride, methylene chloride or chloroform. This reaction is typically carried out at room temperature. The compound of formula XVII that is produced in this reaction can then be converted into the benzeneboronic acid derivative of formula XVIII in the following manner. First, the compound of formula XVII, in a solvent such as THF, is cooled to a temperature of about -78°C to about -70°C, after which n-butyl lithium is added. After stirring the reaction mixture for about 1 hour, triethyl borate is added and the mixture is allowed to stir for an additional 1-3 hours. The benzeneboronic acid intermediate can then be isolated by methods well known to of those skilled in the art (e.g., quenching with ammonium chloride, adding water followed by concentrated hydrochloric acid, and then extracting with ethyl acetate).

Scheme 7 exemplifies a process for making compounds of the formula I wherein G is alkenyl, as well as compounds of the formula I wherein G is hydrogen and R² is an alkyl or alkenyl group. Referring to Scheme 7, the compound of formula IA is converted into the corresponding compound having the formula IH using an alkylation reaction analogous to that used to convert the compound of formula ID into that of formula IG in Scheme 5. Heating the resulting compound of formula IH to about 230°C yields the corresponding compounds of formulas IJ and IK. Hydrogenation of the compounds of formulas IJ and IK, using methods well know to those of skilled in the art (e.g., using hydrogen gas in ethanol of about 50 pounds per square inch, in the presence of 10% palladium on carbon at about room temperature) yields the corresponding alkyl derivatives of, respectively, formulas IL and IM. Alkylation of the compounds of formulas IL and IM (wherein G is hydrogen), using any of the alkylation methods described in Schemes 2, 4, and 5, and the appropriate alkylating agent, yields the corresponding desired compounds wherein G is other than hydrogen.

Scheme 8 illustrates an alternate method of preparing compounds of the formula I wherein G is NR³R⁴(C₀-C₄) alkyl. Referring to Scheme 8, a compound of the formula XIX is reacted with bromine in acetic acid at a temperature from about 0°C to about 60°C, preferably at about room temperature. This reaction produces the corresponding compound having a bromine substituent para to the fluoro substituent, which can then be converted into the corresponding boronic acid derivative of formula XX as described above for the synthesis of compounds of the formula VI (in Scheme 1) and XVIII (in Scheme 6).

Addition of the 2,5-dimethylpyrroyl protecting group as described above for the synthesis of compounds of the formula VIII (in Scheme 1) yields the corresponding compound of formula XXI. The compound of formula XXI is then reacted with a compound of the formula R³R⁴NOH and an alkali metal hydride, preferably sodium hydride, in a polar, organic solvent such as DMF or DMSO, preferably DMF, at a temperature between about 50°C and about 110°C, preferably at about 100°C, to form a compound that is identical to the corresponding desired compound of formula IN, but for the presence of the 2,5-dimethylpyrrolyl protecting group. Removal of the protecting group, as described above for the preparation of compounds of the formula IA (in Scheme 1) yields the desired compound of formula IN.

Scheme 9 illustrates a method of synthesizing compounds of the formula I wherein G is an optionally substituted pyrrolidin-2-yl or pyrrolidin-3-yl group. Referring to Scheme 9, a compound of the formula IA is reacted with a compound of the formula 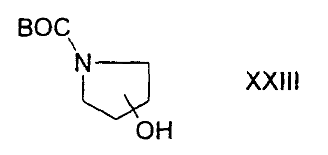 triphenylphosphine and diethylazodicarboxylate or another water soluble azodicarboxylate in THF under standard Mistsunobo reaction conditions. Typically, the reactants are combined at about 0°C and then allowed to warm to room temperature. (If an alkyl substituent on the pyrrolidine nitrogen other than methyl is desired in the final product of formula IP, this can be accomplished by replacing the BOC group of formula XXIII with a group of the formula -C(=O)R, wherein R is the desired alkyl group).

The compound of formula XXII that is formed in the above reaction (or the corresponding -C(=O)R protected compound) can be converted into the desired product having formula IP (or a similar compound wherein the methyl substitutuent depicted in structure IP is replaced with another alkyl group) by reducing it. This reduction can be accomplished by reacting the product from the preceding reaction with lithium aluminum hydride and aluminum chloride in THF or borane in THF as described above for the formation of compounds of the formula IC.

The corresponding compound of formula I wherein the alkyl substituent on the pyrrolidine nitrogen formula IP is replaced with hydrogen can be obtained by reacting the compound of formula XXII with (or an alkyl analogue of XXII, as referred to above) with trifluoroacetic acid or hydrochloric acid in a solvent such as dioxane, or ether, preferably dioxane, at a temperature from about 0°C to about reflux temperature of the reaction mixture, preferably at about the reflux temperature.

The starting materials used in the procedures of Schemes 1-9 are, the syntheses of which are not described above, either commercially available, known in the art or readily obtainable from known compounds using method that will be apparent to those skilled in the art.

The preparation of other compounds of the formula I not specifically described in the foregoing experimental section can be accomplished using combinations of the reactions described above that will be apparent to those skilled in the art.

In each of the reactions discussed or illustrated above, pressure is not critical unless otherwise indicated. Pressures from about 0.5 atmospheres to about 5 atmospheres are generally acceptable, and ambient pressure, i.e., about 1 atmosphere, is preferred as a matter of convenience.

The compounds of formula I ("the active compounds of this invention") which are basic in nature are capable of forming a wide variety of different salts with various inorganic and organic acids. Although such salts must be pharmaceutically acceptable for administration to animals, it is often desirable in practice to initially isolate a compound of the formula I from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the active base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained.

The active compounds of this invention and their pharmaceutically acceptable salts are useful as NOS inhibitors i.e., they possess the ability to inhibit the NOS enzyme in mammals, and therefore they are able to function as therapeutic agents in the treatment of the aforementioned disorders and diseases in an afflicted mammal.

The active compounds of this invention and their pharmaceutically acceptable salts can be administered via either the oral, parental or topical routes. In general, these compounds are most desirably administered in dosages ranging from about 0.01 to about 250 mg per day, in single or divided doses (i.e., from 1 to 4 doses per day), although variations will necessarily occur depending upon the species, weight and condition of the subject being treated and the particular route of administration chosen. However, a dosage level that is in the range of about 0.07 mg to about 21 mg per kg of body weight per day is most desirably employed. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The active compounds of the invention may be administered alone or in combination with pharmaceutically acceptable carriers or diluents by either of the three routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutically-effective compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of an active compound of the present invention in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

Additionally, it is also possible to administer the active compounds of the present invention typically when treating inflammatory conditions of the skin and this may be done by way of creams, jellies, gels, pastes, patches, ointments and the like, in accordance with standard pharmaceutical practice

The ability of compounds of the formulae I to inhibit NOS may be determined using procedures described in the literature. The ability of compounds of the formulae I to inhibit endothelial NOS may be determined by using the procedures described by Schmidt et al. in Proc. Natl. Acad. Sci. U.S.A., 88, pp. 365-369 (1991) and by Pollock et al., in Proc. Natl. Acad. Sci. U.S.A., 88, pp. 10480-10484 (1991). The ability of compounds of the formulae I to inhibit inducible NOS may be determined using the procedures described by Schmidt et al., in Proc. Natl. Acad. Sci. U.S.A., 88 pp. 365-369 (1991) and by Garvey et al. in J. Biol. Chem., 269, pp. 26669-26676 (1994). The ability of the compounds of the formulae I to inhibit neuronal NOS may be determined using the procedure described by Bredt and Snyder in Proc. Natl. Acad. Sci. U.S.A., 87, 682-685 (1990).

The title compounds of Examples 1 and 2 below were tested according to the foregoing procedure and each exhibited an IC₅₀ < 10 µM for inhibition of either inducible or neuronal NOS.

The present invention is illustrated by the following examples. It will be understood, however, that the invention is not limited to the specific details of these examples. Melting points are uncorrected. Proton nuclear magnetic resonance spectra (¹H NMR) and C¹³ nuclear magnetic resonance spectra were measured for solutions in deuterochloroform (CDCl₃) or in CD₃OD or CD₃SOCD₃ and peak positions are expressed in parts per million (ppm) downfield from tetramethylsilane (TMS). The peak shapes are denoted as follows: s, singlet; d, doublet; t, triplet; q, quartet, m, multiplet, b, broad.

### EXAMPLE 1

### 4-(6-AMINO-PYRIDIN-2-YL)-3-METHOXYPHENOL

### A. Toluene-4-sulfonic acid, 4-bromo-3-methoxy-phenyl ester

Under a N₂ atmosphere in 300 mls of acetone was combined 7.00 grams (g) (37.04 mmol) of 4-bromoresorcinol and 32.76 g (237.0 mmol) of potassium carbonate followed by 6.246 g (37.04 mmol) of p-toluenesulfonyl chloride. The reaction was allowed to reflux with stirring for 16 hours at which point 5.96 mls (96.29 mmol) of methyl iodide was added. The solution was heated at 45°C for 48 hours. The reaction mixture was cooled, diluted with 300 mls of dietyl ether, filtered through a pad of Celite@, and concentrated in vacuo to yield 13.0 g of crude product as an orange oil which was chromatographed on 400g of silica gel 60 (EM Science) using 4:1 hexane: ethyl acetate to afford 10.10 g (76%) of the title compound.

¹H NMR (CDCl₃) δ 1.93 (s-6H), 2.30 (s-3H), 3.57 (s-3H), 6 88 (s-2H), 7.47 (d-1H), 762 (dd-1H), 8.17 (d-1H).

### B. 4-Bromo-3-methoxyphenol

Under a nitrogen (N₂) atmosphere was dissolved 10.0 g (27.99 mmol) of the title compound from step A into a solution containing 300 mls of ethanol and 300 mls of water. To this solution was added 21.0 g (318 mmol) of potassium hydroxide and the resultant solution was heated to reflux for 2 hours. The reaction was cooled and concentrated in vacuo to approximately 150 mis and neutralized with acetic acid. This solution was extracted with ethyl ether (3 x 200 mls). The combined extracts were washed with saturated NaCO₃ (2 x 400 mls) followed by 3 percent potassium hydroxide (KOH) (4 x 100 mls). The aqueous layer was acidified with concentrated hydrochloric acid (HCl) and the aqueous layer was extracted with ethyl ether (3 x 200 mls). The organic extracts were washed with brine (1 x 200 mls), dried over magnesium sulfate filtered and concentrated in vacuo to afford 4.60 g (81%) of desired phenol which crystallized upon standing. Recrystallization from hexane/ethyl ether afforded 3.7 g of the title compound as a white crystalline product.

¹H NMR (CDCl₃) δ 1.92 (s-6H), 2.31 (s-3H), 6.89 (s-2H), 7.47 (d-1H), 7.63 (dd-1H), 8.18 (d-1H).

### C. 4-Benzyloxy-1-bromo-2-methoxybenzene

Under a N₂ atmosphere in 50 mls of acetone was combined 3.689 g (18.17 mmol) of 4-bromo-3-methoxyphenol and 7.533 g (54.51 mmol) of potassium carbonate followed by 2.38 mls (19.99 mmol) of benzyl bromide. The reaction was followed to reflux with stirring for 16 hours and concentrated in vacuo. The solid residue was partitioned between ethyl acetate and water. The aqueous layer was extracted with ethyl acetate (1 X 200 mls) and the combined organic extracts were washed with 1M sodium hydroxide (NaOH) (2 X 100 mls) and brine (1 X 100 mls) and dried over sodium sulfate, filtered and concentrated in vacuo to yield 5.38 g (100%) of crude product as a colorless oil.

¹H NMR (CDCl₃) δ 1.37 (s-9H), 1.93 (s-6H), 2.32 (s-3H), 6.08 (bs-1H), 6.96 (s-2H), 7.31 (m-2H), 7,89 (m-1H).

### D. 4-Benzyloxy-2-methoxy-phenylboronic acid

Under a N₂ atmosphere in 75 mls of anhydrous THF was added 5.38 g (18.35 mmol) of 4-benzyloxy-1-bromo-2-methoxybenzene. The solution was cooled to -78°C and 8.07 mls (20.19 mmol) of a 2.5 M solution of butyl lithium was added dropwise and the temperature was maintained below -70°C. The reaction mixture was stirred at -78°C for 1.5 hours at which point 3.43 mls (20.19 mmol) of triethyl borate was added. The reaction was allowed to stir at -78°C for an additional 2.5 hours. The reaction mixture was quenched with 50 mls of saturated ammonium chloride (NH₄Cl) and allowed to warm to ambient temperature. Water (100 mls) was added to this solution, the pH was adjusted to 5.0 with 1 M HCl and the resultant solution was extracted with ethyl acetate (2 x 200 mls). The combined extracts were washed with brine (1 X 100 mls) and dried over sodium sulfate, filtered and concentrated in vacuo to yield crude product as a pink solid which was crystallized with ethyl acetate/hexane to afford 2.68 g (57%) of 4-benzyloxy-2-methoxy-phenylboronic acid as an off-white solid. ¹H NMR (CDCl₃) δ 1.38 (s-9H), 1.93 (s-6H), 2.31 (s-3H), 4.10 (bs-2H), 5.57 (bs-1H), 6.50 (d-1H), 6.77 (d-1H), 6.92 (s-2H), 7.10 (dd-1H).

### E. 2-(4-Benzyloxy-2-methoxy-phenyl)-6-(2,5-dimethyl-pyrrol-1-yl)-pyridine.

Under a nitrogen atmosphere was combined 2.53 g (10.07 mmol) of 2-bromo-6-(2,5-dimethyl-pyrrol-1-yl)-pyridine, 2.60g (10.07 mmol) of benzyloxy-2-methoxy-phenylboronic acid, 4.27 g (40.30 mmol) of sodium carbonate and 292 mg of tetrakis(triphenylphosphine)palladium(0) (0.25 mmol) in 27 mls of ethanol and 3 mls of water. The solution was allowed to reflux for 18 hours at which point the reaction mixture was concentrated in vacuo. The resultant yellow residue was partitioned between ethyl acetate (200 mls) and water (200 mls). The aqueous layer was extracted again with ethyl acetate (200 mls) and the combined organic extracts were washed with brine (1 X 200 mls) and dried over sodium sulfate, filtered and concentrated in vacum to yield crude product as a yellow oil which crystallized upon standing. Recrystallization of this solid from absolute ethanol afforded 3.10 g (80%) of the desired product as a tan solid.

¹H NMR (CDCl₃) δ 0.98 (t-6H), 1.33 (s-9H), 1.57 (m-4H), 1.98 (s-6H), 2.32 (s-3H), 3.30 (m-1H), 4.18 (bs-1H), 5.30 (bs-1H), 6.39 (d-1H, 6.68 (d-1H, 6.92 (s-2H), 7.20 (dd-1H).

¹³C NMR (CDCl₃) 10.13, 20.25, 21.05, 26.61, 28.03, 55.29, 80.03, 110.77, 117.19, 127.69, 128.11, 120.80, 135.79, 136.09, 136.57, 144.30, 153.60

### F. 4-[6-(2,5-Dimethyl-pyrrol-1-yl)-pyridin-2-yl]-3-methoxyphenol

Under a nitrogen atmosphere was combined 3.10 g (8.063 mmol) of 2-(4-benzyloxy-2-methoxy-phenyl)-6-(2,5-dimethyl-pyrrol-1-yl)-pyridine and 15.25 g (241.9 mmol) of ammonium formate in 100 mls of methanol. The resultant slurry was allowed to reflux for 2 hours at which point the reaction mixture was allowed to cool to ambient temperature and passed through a 0.2 uM nylon membrane and the residue was washed with additional methanol. The organic solution was concentrated in vacuo and the resultant yellow residue was partitioned between ethyl acetate (200 mls) and water (200 mls). The aqueous layer was extracted again with ethyl acetate (200 mls) and the combined organic extracts were washed with brine (1 X 200 mls) and dried over sodium sulfate, filtered and concentrated in vacum to yield 2.011 g (85%) of the desired phenol as a tan solid.

¹H NMR (CDCl₃) δ 0.93 (t-6H), 1.60 (m-4H), 1.98 (s-6H), 2.30 (s-3H), 3.08 (m-3H), 3.22 (m-1H), 6.39 (d-1H), 6.61 (d-1H), 6.82 (dd-1H), 6.95 (s-2H)

### G. 4-(6-Amino-pyridin-2-yl)-3-methoxyphenol

Under a nitrogen atmosphere was combined 5.92 g (20.11 mmol) of phenol and 16.77 g (241.3 mmol) of hydroxylamine hydrochloride in 120 mls of ethanol and 20 mls of water. The resultant mixture was allowed to reflux for 16 hours at which point the reaction mixture was allowed to cool to ambient temperature and concentrated in vacuo. The resultant yellow residue was partitioned between ethyl acetate (200 mls) and dilute sodium bicarbonate (200 mls). The aqueous layer was extracted again with ethyl acetate (2 X 200 mls) and the combined organic extracts were washed with brine (1 X 200 mls) and dried over sodium sulfate, filtered and concentrated in vacuum to yield crude product as a brown oil which was chromatographed on 300g of silica gel 60 (EM Science) using 4:1 hexane: ethyl acetate to afford 4.20 g (97%) of products a yellow foam which was crystallized from ethyl acetate/hexane to afford the title compound as a white solid.

¹H NMR (CDCl₃) δ 0.83 (t-6H), 1.33 (t-3H), 1.98 (s-6H), 2.00 (m-4H), 2.20 (m-2H), 2.32 (s-3H), 2.88 (q-2H), 4.08 (m-1H), 6.93 (m-3H), 7.18 (dd-1H), 7.42 (d-1H)

### EXAMPLE 2

### 6-[4-(2-DIMETHYLAMINO-ETHOXY)-2-METHOXY-PHENYL]-PYRIDIN-2-YLAMINE

Under a nitrogen (N₂) atmosphere in 30 mls of acetone was combined 200 mg (0.92 mmol) of phenol and 383 mg (2.78 mmol) of potassium carbonate followed by 146 mg (1.017 mmol) of N-(2-chloroethyl)dimethylamine hydrochloride. The reaction was allowed to reflux with stirring for 16 hours and concentrated in vacuo. The solid residue was partitioned between ethyl acetate and 1M sodium hydroxide (NaOH). The aqueous layer was extracted with ethyl acetate (1 X 200 mls) and the combined organic extracts were washed with 1M NaOH (2 x 100 mls) and brine (1 X 100 mls) and dried over sodium sulfate, filtered and concentrated in vacuo to yield crude product which was chromatographed on 75 g of silica gel 60 (EM Science) using 9:1:0.1 dichloromethane:methanol:ammominum hydroxide to afford 165 mg (62%) of the title compound as an off-white solid. Fifty milligrams of the corresponding hydrochloride salt of the title compound was prepared by dissolving a portion of the title compound in ethyl acetate and adding an ethyl acetate solution saturated with HCl.

### EXAMPLE 3

### 6-[4-(2-DIMETHYLAMINO-ETHOXY)-2,3-DIMETHYL-PHENYL]-PYRIDIN-2-YLAMINE

### A. 3-Fluoro-6-bromo-o-xylene

To a 100 mL round-bottomed flask equipped with N₂ inlet were added 2.50 mL (20 mmol) 3-fluoro-o-xylene, 10 mL acetic acid, and 1.03 mL (20 mmol) bromine. After 12 hours at room temperature, the solution had turned colorless and was poured into water and extracted into petroleum ether. The organic layer was washed with water and 1 N sodium hydroxide solution, dried over sodium sulfate, and evaporated to a liquid, 4 g (100%), as a mixture of isomers.

¹H-NMR (δ, CDCl₃): 2.20, 2.25, 2.30, 2.38 (singlets, 6H), 6.78 (t, J=9, 6.8-7.4 (m, 1H).

¹³C-NMR (δ, CDCl₃): 10.6, 10.7, 19.5, 19.6, 112.2, 112.5, 113.7, 113.9. 125.0, 126.1, 130.2, 138.2, 158.9, 160.0, 161.4, 162.4.

### B. 3-Fluoro-o-xylene-6-boronic acid

To a 125 mL three-necked round-bottomed flask equipped with septum and N₂ inlet were added 4.08 g (20 mmol) 3-fluoro-6-bromo-o-xylene and 20 mL dry tetrahydrofuran. The solution cooled to -70°C, and 9.6 mL (24 mmol) of a 2.5 M solution of butyl lithium in hexane was added slowly over 5 minutes. The reaction was stirred 5 minutes at -70°C, then 4.08 mL (24 mmol) triethyl borate added, and stirring continued at -70°C for 5 minutes. The reaction was then allowed to warm to room temperature and stirred for 16 hours, then poured into dilute hydrochloric acid and extracted with ethyl acetate. The organic layer was washed with brine, dried over sodium sulfate, and evaporated. The residue was triturated with hexane to a white solid, 2.06 g (64%).

¹H-NMR (δ, CDCl₃): 2.22 (s, 3H), 2.30 (s, 3H), 6.7-7.3 (m, 2H).

¹³C-NMR (δ, CDCl₃): 25.4, 26.3, 111.5, 111.7, 112.1, 112.3, 124.9, 126.0, 126.1, 130.8, 130.9, 159.9, 160.6, 162.3, 163.0,.

### C. 2-(2,5-Dimethylpyrrolyl)-6-[4-fluoro-2,3-dimethyl-phenyl]-pyridine

To a 100 mL round-bottomed flask equipped with condenser and N₂ inlet were added 3.08 g (12.27 mmol) 6-bromo-2-(2,5-dimethylpyrrolyl)pyridine, 2.06 g (12.27 mmol) 3-fluoro-oxylene-6-boronic acid, 5.20 g (49.1 mmol) sodium carbonate, 140 mg tetrakistriphenylphosphinepalladium, 36 mL ethanol, and 4 mL water. The reaction was refluxed 4 hours, cooled, and poured into water, then extracted into ethyl acetate. The organic layer was washed with brine, dried over sodium sulfate, and evaporated. The residue was chromatographed on silica gel using hexane/ethyl acetate as eluant to afford 3.2 g (89%) of a solid.

¹H-NMR (δ, CDCl₃): 2.16 (s, 6H), 2.23 (s, 3H), 2.25 (s, 3H), 5.88 (s, 2H), 6.94 (m, 1H), 7.16 (m, 2H), 7.33 (d, J=8, 1H), 7.86 (t, J=8, 1H).

¹³C-NMR (δ, CDCl₃): 11.30, 13.38, 17.31, 106.80, 107.57, 112.15, 112.39, 119.92, 122.96, 123.70, 126.05, 126.42, 128.34, 136.95, 138.10, 139.81, 151.48, 159.99, 162.32.

MS (%): 295 (parent+1, 100).

### D. 2-(2,5-Dimethylpyrrolyl)-6-[4-(2-dimethylamino-ethoxy)-2,3-dimethyl-phenyl]-pyridine

To a 100 mL round-bottomed flask equipped with septum and N₂ inlet were added 0.121 mL (1.2 mmol) 2-dimethylaminoethanol, 4 mL dry dimethylformamide, and 115 mg (2.4 mol) sodium hydride (60% in oil). The reaction was heated for 30 minutes to ensure complete formation of the alkoxide, cooled, and 294 mg (1.0 mmol) 2-(2,5-dimethylpyrrolyl)-6-[4-fluoro-2,3-dimethyl-phenyl)-pyridine added. The reaction was heated at 100°C for 18 hours, cooled, and poured into water, then extracted into ethyl acetate. The organic layer was washed with water and brine, dned over sodium sulfate, and evaporated. The residue was chromatographed on silica gel using methanol/methylene chloride as eluant to afford 260 mg (72%) of an oil.

¹H-NMR (δ, CDCl₃): 2.18 (s, 6H), 2.22 (s, 3H), 2.27 (s, 3H), 2.37 (s, 6H), 2.79 (t, J=6, 2H), 4.11 (t, J=6, 2H), 5.88 (s, 2H), 6.79 (d, J=8, 1H), 7.13 (d, J=8, 1H), 7.22 (d, J=8, 1H), 7.34 (d, J=8, 1H), 7.82 (t, J=8, 1H).

¹³C-NMR (δ, CDCl₃): 12.19, 13.41, 17.61, 45.81, 46.10, 58.39, 66.92, 106.65, 108.81, 119.46, 123.05, 125.98, 127.97, 128.57, 133.22, 135.68, 137.90, 151.34, 156.84, 160.71.

MS (%): 364 (parent+1, 100).

### E. 6-[4-(2-Dimethylamino-ethoxy)-2,3-dimethyl-phenyl]-pyridin-2-ylamine

To a 100 mL round-bottomed flask equipped with condenser and N₂ inlet were added 260 mg (0.716 mmol) 2-(2,5-dimethylpyrrolyl)-6-[4-(2-dimethylamino-ethoxy)-2,3-dimethylphenyl]-pyridine, 500 mg hydroxylamine hydrochloride, 9 mL ethanol, and 1 mL water. The reaction was refluxed 40 hours, cooled, poured into dilute hydrochloric acid, washed with ethyl acetate, adjusted to pH 12 with 6 N sodium hydroxide solution, and extracted twice into methylene chloride. The organic layer was dried over sodium sulfate and evaporated, then converted to the hydrochloride salt using HCl in ether to afford a hygroscopic solid, 182 mg (71%).

¹H-NMR (δ, CDCl₃): 2.16 (s, 3H), 2.18 (s, 3H), 2.32 (s, 6H), 2.73 (d, J=7, 2H), 4.05 (t, J=7, 2H), 4.65 (bs, 2H), 6.33 (d, J=8, 1H), 6.59 (d, J=7, 1H), 6.71 (d, J=8, 1H), 7.10 (d, J=8, 1H), 7.37 (t, J=8, 1H)

¹³C-NMR (δ, CDCl₃): 12.13, 17.25, 46.07, 58.39, 66.92, 106.08, 108.75, 114.40, 125.79, 127.24, 134.23, 135.53, 137.68, 156.39, 157.91, 159.19.

MS (%): 286 (parent+1, 100).

Anal. Calc'd. for C₁₇H₂₃N₃O•2HCl•5/4H₂O: C 53.62, H 7.28, N 11.03 Found: C 53.68, H 7.12, N 10.86.

### EXAMPLE 4

### 6-[4-(2-PYRROLIDINYL-ETHOXY)-2,3-DIMETHYL-PHENYL]-PYRIDIN-2-YLAMINE

Prepared as in Example 3, using 2-pyrrolidinyl-ethanol, in 57% yield, as a hygroscopic solid.

¹H-NMR (δ, CDCl₃): 1.76 (m, 4H), 2.16 (s, 3H), 2.17 (s, 3H), 2.61 (m, 4H), 2.89 (t, J=6, 2H), 4.09 (t, J=6, 2H), 4.62 (bs, 2H), 6.34 (d, J=8, 1H), 6.59 (d, J=7, 1H), 6.71 (d, J=8, 1H), 7.09 (d, J=8, 1H), 7.38 (t, J=8, 1H).

¹³C-NMR (δ, CDCl₃): 12.13, 17.25, 23.52, 54.85, 55.07, 67.78, 106.05, 106.62, 108.73, 114.44, 125.73, 127.24, 134.14, 135.49, 137.68, 156.39, 157.85, 159.22.

MS (%): 312 (parent+1, 100).

Anal Calc'd for C₁₉H₂₅N₃O•2HCl•2H₂O: C 54.29, H 7.43, N 10.00. Found: C 54.48, H 7.60, N 9.64.

### EXAMPLE 5

### 6-[4-(4-(N-METHYL)PIPERIDINYLOXY)-2,3-DIMETHYL-PHENYL]-PYRIDIN-2-YLAMINE

Prepared as in Example 3, using 4-hydroxy-N-methylpiperidine, in 56% yield, mp 110-130°C as the hydrochloride salt.

¹H-NMR (δ, CDCl₃): 1.8-2.0 (m, 4H), 2.16 (s, 6H), 2.24 (s, 3H), 2.6 (m, 4H), 4.3 (m, 1H), 4.62 (bs, 2H), 6.33 (d, J=8, 1H), 6.58 (d, J=8, 1H), 6.71 (d, J=8, 1H), 7.06 (d, J=8, 1H), 7.37 (t, J=8, 1H).

¹³C-NMR (δ, CDCl₃): 12.2, 17.2, 20.9, 30.7, 46.2, 52.4, 106.0, 110.9, 114.3, 127.0, 135.7, 137.6, 140.1, 154.7, 157.8, 159.1.

MS(%): 312 (parent+1, 100).

Anal. Calc'd. for C₁₉H₂₅N₃O•2HCl•3/2H₂O: C 55.48, H 7.35, N 10.21. Found: C 55.72, H 7.32, N 10.66.

### EXAMPLE 6

### 6-[4-(2-DIMETHYLAMINO-ETHOXY)-3-METHOXY-PHENYL]-PYRIDIN-2-YLAMINE

Prepared as in Example 2, using 2-methoxy-4-bromophenol, in 68% yield, mp 225-228°C as the hydrochloride salt.

¹H-NMR (δ, CDCl₃): 2.29 (s, 6H), 2.74 (t, J=6, 2H), 3.87 (s, 3H), 4.10 (t, J=6, 2H), 4.67 (bs, 2H), 6.32 (d, J=8, 1H), 6.88 (d, J=8, 1H), 6.95 (d, J=8, 1H), 7.38 (m, 2H), 7.51 (s, 1H).

¹³C-NMR (δ, CDCl₃): 45.96, 55.86, 58.02, 67.15, 106.54, 110.15, 110.38, 113.04, 119.23, 132.99, 138.27, 148.83, 149.49, 155.66, 158.33.

MS (%): 288 (parent+1, 100).

Anal. Calc'd. for C₁₆H₂₁N₃O₂•2HCl•H₂O·½(C₄H₁₀O): C 52.05, H 7.28, N 10.12. Found: C 51.80, H 6.93, N 10.44.

### EXAMPLE 7

### 6-[4-(2-PYRROLIDINYL-ETHOXY)-3-METHOXY-PHENYL]-PYRIDIN-2-YLAMINE

Prepared as in Example 2, in 65.5% yield, mp 202-210°C as the hydrochloride salt.

¹H-NMR (δ, CDCl₃): 1.75 (m, 4H), 2.59 (m, 4H), 2.92 (t, J=6, 2H), 3.88 (s, 3H), 4.15 (t, J=6, 2H), 4.62 (bs, 2H), 6.33 (d, J=8, 1H), 6.89 (d, J=8, 1H), 6.97 (d, J=8, 1H), 7.39 (m, 2H), 7.52 (s, 1H).

¹³C-NMR (δ, CDCl₃): 23.49, 54.69, 54.78, 55.91, 67.99, 106.50, 110.18, 110.38, 112.98, 119.26, 132.92, 138.27, 148.86, 149.46, 155.69, 158.27.

MS (%): 314 (parent+1, 100)

Anal Calc'd. for C₁₈H₂₃N₃O₂•2HCl•½H₂O: C 54.69, H 6.63, N 10.63 Found: C 54.88, H 6.88, N 10.01.

### EXAMPLE 8

### 6-{4-[2-(6,7-DIMETHOXY-3,4-DIHYDRO-1H-ISOQUINOLIN-2-YL)-ETHOXY]-3-METHOXY-PHENYL}-PYRIDIN-2-YLAMINE

Prepared as in Example 2, in 79% yield, mp 90-100°C as the hydrochloride salt.

¹H-NMR (δ, CDCl₃): 2.80 (m, 4H), 2.98 (t, J=6, 2H), 3.66 (s, 2H), 3.77 (s, 3H), 3.78 (s, 3H), 3.89 (s, 3H), 4.23 (t, J=8, 2H), 4.66 (bs, 2H), 6.31 (d, J=8, 1H), 6.47 (s, 1H), 6.535 (s, 1H), 6.91 (d, J=8, 1H), 6.96 (d, J=8, 1H), 7.37 (m, 2H), 7.52 (s, 1H).

¹³C-NMR (δ, CDCl₃): 28.50, 51.54, 55.84, 55.91, 56.04, 56.57, 67.30, 106.58, 109.42, 110.14, 110.41, 111.33, 113.07, 119.29, 125.95, 126.39, 133.04, 138.29, 147.15, 147.48, 148.80, 149.48, 155.60, 158.34.

MS(%) 436 (parent+1, 100).

Anal. Calc'd. for C₂₅H₂₉N₃O₄•2HCl•5/4H₂O: C 56.55, H 6.36, N 7.91. Found: C 56.59, H 6.19, N 7.70.

### EXAMPLE 9

### 6-{3-METHOXY-4-[2-(4-PHENETHYL-PIPERAZIN-1-YL)-ETHOXY]-PHENYL}-PYRIDIN-2-YLAMINE

Prepared as in Example 2, in 78% yield, mp 167-182°C as the hydrochloride salt.

¹H-NMR (δ, CDCl₃): 2.4-2.6 (m, 10H), 2.75 (m, 2H), 2.825 (t, J=6, 2H), 3.86 (s, 3H), 4.13 (t, J=6, 2H), 4.70 (bs, 2H), 6.32 (d, J=8, 1H), 6.87 (d, J=8, 1H), 6.95 (d, J=8, 1H), 7.15 (m, 3H), 7.21 (m, 2H), 7.37 (m, 2H), 7.51 (s, 1H).

¹³C-NMR (δ, CDCl₃): 32.56, 33.46, 52.98, 53.52, 55.82, 56.91, 60.37, 66.78, 106.47, 110.01, 110.39, 113.04, 119.21, 125.90, 128.25, 128.51, 128.58, 132.96, 138.18, 140.17, 148.73, 149.39, 155.52, 158.29. MS (%): 433 (parent+1, 100)

Anal. Calc'd for C₂₆H₃₂N₄O₂•3HCl•H₂O: C 55.77, H 6.66, N 10.01. Found: C 55.80, H 6.56, N 9.59.

### EXAMPLE 10

### 6-{3-METHOXY-4-[2-(4-METHYL-PIPERAZIN-1-YL)-ETHOXY]-PHENYL}-PYRIDIN-2-YLAMINE

Prepared as in Example 2, in 71% yield, mp 75-95°C as the hydrochloride salt.

¹H-NMR (δ, CDCl₃): 2.19 (s, 3H), 2.4 (m, 4H), 2.6 (m, 4H), 2.78 (t, J=6, 2H), 3.83 (s, 3H), 4.10 (t, J=6, 2H), 4.66 (bs, 2H), 6.295 (d, J=8, 1H), 6.84 (d, J=8, 1H), 6.92 (d, J=8, 1H), 7.33 (m, 2H), 7.48 (s, 1H)

¹³C-NMR (δ, CDCl₃): 45.97, 53.56, 54.98, 55.88, 56.92, 66.93, 106.51, 110.07, 110.43, 113.14, 119.23, 133.02, 138.23, 148.77, 149.46, 155.59, 158.31,.

MS (%): 343 (parent+1, 100).

Anal. Calc'd. for C₁₉H₂₆N₄O₂•3HCl•2H₂O•½(C₄H₁₀O): C 48.05, H 7.30, N 10.67. Found: C 47.85, H 6.98, N 11.01.

### EXAMPLE 11

### 6-{4-[2-(4-DIMETHYLAMINO-PIPERIDIN-1-YL)-ETHOXY]-3-METHOXY-PHENYL}-PYRIDIN-2-YLAMINE

Prepared as in Example 2, in 61 % yield, mp 215-221°C as the hydrochloride salt.

¹H-NMR (δ, CDCl₃): 1.5 (m, 2H), 1.75 (m, 2H), 2.07 (m, 2H), 2.215 (s, 3H), 2.79 (t, J=6, 2H), 3.0 (m, 3H), 3.87 (s, 3H), 4.13 (t, J=6, 2H), 4.62 (bs, 2H), 6.33 (d, J=8, 1H), 6.88 (d, J=8, 1H), 6.96 (d, J=8, 1H), 7.38 (m, 2H), 7.50 (s, 1H).

¹³C-NMR (δ, CDCl₃): 28.17, 30.28, 41.57, 53.69, 55.94, 56.90, 62.04, 67.07, 106.52, 110.18, 110.40, 113.05, 119.26, 132.96, 138.29, 148.80, 149.45, 155.66, 158.27

MS (%): 371 (parent+1, 100).

Anal. Calc'd. for C₂₁H₃₀N₄O₂•3HCl•5/2H₂O: C 48.05, H 7.30, N 10.67 Found: C 48.34, H 7.28, N 10.66.

### EXAMPLE 12

### 6-[4-(2-DIMETHYLAMINO-ETHOXY)-3-ETHOXY-PHENYL]-PYRIDIN-2-YLAMINE

Prepared as in Example 2, (using 2-ethoxy-4-bromophenol), in 72% yield, mp 210-216°C as the hydrochloride salt.

¹H-NMR (δ, CDCl₃): 1.40 (t, J=7, 3H), 2.31 (s, 6H), 2.74 (t, J=6, 2H), 4.10 M, 4H), 4.64 (bs, 2H), 6.34 (d, J=8, 1H), 6.89 (d, J=8, 1H), 6.96 (d, J=8, 1H), 7.38 (m, 2H), 7.51 (s, 1H).

¹³C-NMR (δ, CDCl₃): 14.88, 46.04, 58.06, 63.99, 64.43, 67.65, 106 50, 110.21, 112.10, 113.81, 119.38, 133.12, 138.27, 149.02, 149.22, 155.74, 158.28.

MS (%): 302 (parent+1, 100).

Anal Calc'd. for C₁₇H₂₃N₃O₂•2HCl•½H₂O): C 53.27, H 7.84, N 10.96. Found C 53.57, H 7.16, N 10.71.

### EXAMPLE 13

### 6-[4-(2-PYRROLIDINYL-ETHOXY)-3-ETHOXY-PHENYL]-PYRIDIN-2-YLAMINE

Prepared as in Example 2 (using 2-ethoxy-4-bromophenol), in 69% yield, mp 190-198°C as the hydrochloride salt.

¹H-NMR (δ, CDCl₃): 1.415 (t, J=7, 3H), 1.77 (m, 4H), 2.63 (m, 4H), 2.92 (t, J=6, 2H), 4.15 (m, 4H), 4.59 (bs, 2H), 6.35 (d, J=8, 1H), 6.91 (d, J=8, 1H), 6.97 (d, J=8, 1H), 7.41 (m, 2H), 7.51 (s, 1H).

¹³C-NMR (δ, CDCl₃): 14.91, 23.49, 54.75, 54.79, 64.48, 68.36, 106.47, 110.27, 112.15, 113.65, 119.42, 132.99, 138.29, 148.94, 149.29, 155.80, 158.21.

MS (%): 328 (parent+1, 100).

Anal. Calc'd for C₁₉H₂₅N₃O₂•2HCl•1½H₂O•½(C₄H₁₀O): C 54.31, H 7.60, N 9.05 Found C 54.41. H 7.37, N 9.41

### EXAMPLE 14

### 6-[4-(2-DIMETHYLAMINO-ETHOXY)-2-ISOPROPYL-PHENYL]-PYRIDIN-2-YLAMINE

### A. 1-Isopropyl-3-benzyloxy-benzene

Under a N₂ atmosphere in 300 mL of acetone was combined 20.0 ml (146.0 mmol) of 3-isopropylphenol and 40.35 g (291.9 mmol) of potassium carbonate followed by 17.36 mL (146.0 mmol) of benzyl bromide. The reaction was allowed to reflux with stirring for 16 hours. More (5 ml) benzyl bromide was added and heating was continued for another 24 hours. The reaction mixture was allowed to cool to ambient temperature and solids were removed by filtration and washed with acetone. The filtrate was concentrated *in vacuo*. The solid residue was partitioned between ethyl acetate and water. The aqueous layer was extracted with ethyl acetate (1 X 300 mL) and the combined organic extracts were washed with 1M NaOH (1 X 200 mL) and brine (1 X 150 mL), dried over sodium sulfate, filtered and concentrated *in vacuo* (100°C at 1 mm Hg) to yield 33.80 g (100%) of crude product (the title compound) as a yellow oil.

¹H NMR (CDCl₃) δ1.23 (d-6H; J = 7.06 Hz), 2.87 (m-1H), 5.05 (s-2H), 6.78-6.88 (m-2H), 7.21 (t-1H; J = 7.88 Hz), 7.30-7.45 (m-6H).

### B. 1-Bromo-2-isopropyl-4-benzyloxy-benzene

Under a N₂ atmosphere in 400 mL of carbon tetrachloride was combined 33.50 g (148.0 mmol) of 1-isopropyl-3-benzyloxy-benzene, 27.66 g (155.4 mmol) of NBS (recrystallized from water), followed by 60.0 g of silica gel 60 (EM Science). The reaction was allowed to stir in the absence of light for 48 hours. Silica gel was then removed by filtration and was washed with dichloromethane. The combined filtrate was washed with 1M NaOH (2 X 200 mL) and brine (1 X 200 mL), dried over sodium sulfate, filtered and concentrated *in vacuo* to yield 44.46 g (98 %) of crude product (the title compound) as a yellow liquid.

¹H NMR (CDCl₃) δ1.23 (d-6H; J = 6.84 Hz), 3.28-3.35 (m-1H), 5.02 (s-2H), 6.64 (dd-1H; J = 3.12 Hz; J = 8.72 Hz), 6.89 (d-1H; J = 2.91 Hz) 7.30-7.42 (m-6H).

### C. 4-Benzyloxy-2-isopropyl-benzeneboronic acid

Under a N₂ atmosphere in 300 mL of anhydrous THF was added 44.46 g (145.7 mmol) of 1-bromo-2-isopropyl-4-benzyloxy-benzene. The solution was cooled to -78°C and 64.1 mL (160.2 mmol) of a 2.5 M solution of butyl lithium was added dropwise while maintaining the temperature below -70°C. The reaction mixture was stirred at -78°C for 1.0 hours at which point 27.26 mL (160.2 mmol) of triethyl borate was added. The reaction was allowed to stir at less than -60°C for an additional 2.0 hours. The reaction mixture was allowed to warm to ambient temperature and quenched with 200 mL of saturated NH₄Cl. Water (100 mL) was added to this solution, the pH was adjusted to 3.0 with conc HCl and the resultant solution was extracted with ethyl acetate (1 x 200 mL). The ethyl acetate extract was washed with brine (1 X 100 mL), dried over sodium sulfate, filtered and concentrated *in vacuo* to yield crude product as a pink solid which was triturated with ethyl acetate/hexane to afford 16.80 g (43 %) of the title compound as a tan colored solid.

¹H NMR (CDCl₃) δ1.31 (d-6H; J = 6.85 Hz), 4.12-4.18 (m-1H), 5.13 (s-2H), 6.89 (dd-1H; J = 2.28 Hz; J = 8.50 Hz), 7.05 (d-1H; J = 2.28 Hz), 7.32-7.48 (m-5H), 8.15 (d-1H; J = 8.30 Hz).

### D. 2-(4-Benzyloxy-2-isopropyl-phenyl)-6-(2,5-dimethyl-pyrrol-1-yl)-pyridine

Under a nitrogen atmosphere was combined 15.58 g (62.04 mmol) of 2-bromo-6-(2,5-dimethyl-pyrrol-1-yl)-pyridine, 16.76 g (62.04 mmol) of 4-benzyloxy-2-isopropyl-benzeneboronic acid, 26.31 g (248.2 mmol) of sodium carbonate and 1.80 g of tetrakis(triphenylphosphine)palladium(0) (1.55 mmol) in 243 mL of ethanol and 27 mL of water. The solution was allowed to reflux for 72 hours at which point the reaction mixture was concentrated *in vacuo*. The resultant residue was partitioned between ethyl acetate (300 mL) and water (300 mL). The aqueous layer was extracted again with ethyl acetate (200 mL) and the combined organic extracts were washed with brine (1 X 200 mL), dried over sodium sulfate, filtered and concentrated *in vacuo* to yield crude product as an amber solid which crystallized upon standing. Recrystallization of this solid from absolute ethanol:hexane afforded 21.35 g (87 %) of the title compound as a tan solid.

¹H NMR (CDCl₃) δ1.16 (d-6H; J = 6.85 Hz); 2.16 (s-6H), 3.28-3.31 (m-1H), 5.10 (s-2H), 5.88 (s-2H), 6.85 (dd-1H; J = 2.70, J = 8.51 Hz), 7.00 (d-1H; J = 2.49 Hz), 7.15 (d-1H; J = 7.89 Hz), 7.27 (d-1H; J = 8.51 Hz), 7.33 (dd-1H; J = 1.66 Hz; J = 7.06 Hz), 7.39 (dd-2H, J = 6.23 Hz, J = 7.68 Hz), 7.45 (d-2H; J = 7.27 Hz), 7.84 (dd-1H; J = 7.68 Hz; J = 7.89 Hz).

### E. 4-[6-(2,5-Dimethyl-pyrrol-1-yl)-pyridin-2-yl]-3-isopropyl-phenol

Under a nitrogen atmosphere was combined 21.20 g ( 53.46 mmol) of 2-(4-benzyloxy-2-isopropyl-phenyl)-6-(2,5-dimethyl-pyrrol-1-yl)-pyridine and 67.42 g (1.06.9 mol) of ammonium formate and 2.00 g of palladium hydroxide in 300 mL of methanol. The resultant slurry was allowed to reflux. Over an eight hour period, 10.0 g of catalyst was added. The reaction mixture was allowed to cool to ambient temperature and passed through a pad of celite to remove the catalyst. The celite pad was washed with methanol. The filtrate was concentrated *in vacuo* and the resultant yellow residue was partitioned between ethyl acetate (200 mL) and water (200 mL). The aqueous layer was extracted again with ethyl acetate (200 mL) and the combined organic extracts were washed with brine (1 X 200 mL) and dried over sodium sulfate, filtered and concentrated *in vacuo* to yield 15.58 g (95 %) of desired phenol as a tan solid.

¹H NMR (CDCl₃) δ1.14 (d-6H; J = 6.85 Hz); 2.15 (s-6H), 3.21-3.24 (m-1H), 5.50 (bs-1H), 5.88 (s-2H), 6.61 (dd-1H; J = 2.49; J = 8.30 Hz), 6.80 (d-1H; J = 2.49 Hz), 7.14-7.17 (m-2H), 7.24 (d-1H; J = 0.83 Hz), 7.32 (d-1H; J = 7.68 Hz), 7.84 (dd-1H; J = 0.83 Hz; J = 8.51 Hz).

### F. 4-(6-Amino-pyridin-2-yl)-3-isopropylphenol

Under a nitrogen atmosphere was combined 15.55 g (50.75 mmol) of phenol and 42.32 g (609.0 mmol) of hydroxylamine hydrochloride in 180 mL of ethanol and 30 mL of water. The resultant mixture was allowed to reflux for 16 hours at which point the reaction mixture was allowed to cool to ambient temperature and concentrated *in vacuo.* The resultant brown residue was partitioned between ethyl acetate (300 mL) and dilute sodium bicarbonate (300 mL). The aqueous layer was extracted again with ethyl acetate (4 X 100 mL) and the combined organic extracts were washed with brine (1 X 400 mL), dried over sodium sulfate, filtered and concentrated *in vacuo* to yield crude product as a brown gum. Chromatography on 300g of silica gel 60 (EM Science) starting with 3:2 hexane:ethyl acetate and increasing the ethyl acetate concentration yielded 10.0 g (86%) of aminopyridine as a pink solid which was recrystallized from ethyl acetate/hexane to afford the title compound as a tan solid.

¹H NMR (CD₃OD) δ1.11 (d-6H; J = 6.85 Hz); 3.03-3.10 (m-1H), 4.87 (bs-3H), 6.48-6.53 (m-2H), 6.60-6.63 (m-1H), 6.78 (d-1H; J = 2.28 Hz), 7.01 (d-1H; J = 8.30 Hz), 7.43-7.45 (m-1H).

### G. 6-[4-(2-Dimethylamino-ethoxy)-2-isopropyl-phenyl]-pyridin-2-ylamine

Under a N₂ atmosphere in 175 mL of acetone was combined 3.0 g ( 13.14 mmol) of phenol and 17.13 g (52.56 mmol) of cesium carbonate followed by 2.83 g (19.71 mmol) of N-(2-chloroethyl)dimethylamine hydrochloride. The reaction was allowed to reflux with stirring for 16 hours and concentrated *in vacuo* . The solid residue was partitioned between ethyl acetate and water (H₂O). The aqueous layer was extracted with ethyl acetate (1 X 200 mL) and the combined organic extracts were washed with 1M NaOH (2 X 100 mL) and brine (1 X 100 mL), dried over sodium sulfate, filtered and concentrated *in vacuo* to yield crude product which was chromatographed on 80 g of silica gel 60 (EM Science) using 95:5:.05 dichloromethane:methanol:ammomium hydroxide to afford 3 g ( 76 %) of aminopyridine as a colorless oil. The corresponding hydrochloride salt of the title compound (2.95 g) was prepared by dissolving the title compound in dichloromethane (20 mL) and adding diethyl ether (3 mL) saturated with HCl. The mixture was stirred overnight and the white precipitant was filtered and dried.

¹H NMR (CD₃OD) δ1.19 (d-6H; J = 6.85 Hz), 2.99 (s-6H), 2.98-3.02 (m-1H), 3.61 (t-2H, J = 4.98 Hz), 4.41 (t-2H; J = 4.77 Hz), 6.68 (d-1H; J = 8.26 Hz), 6.81 (d-1H; J = 8.72 Hz), 6.97 (dd-1H, J = 8.51 Hz; J = 2.49 Hz), 7.09 (d-1H; J = 2.49 Hz), 7.26 (d-1H; J = 8.51 Hz), 7.74-7.78 (m-1H).

### EXAMPLE 15

### 4-(6-AMINO-PYRIDIN-YL)-3-CYCLOPROPYL-PHENOL

### A. 1-Cyclopropyl-3-benzyloxy-benzene

Cyclopropylmagnesium bromide (J.O.C., 57, 3499-3503, 1992) (formed in situ, 50 mmol in 35 ml of THF) was added via syringe to a stirred mixture of 1-bromo-3-benzyloxy-benzene ( 7.9 g, 30 mmol), [1,3-bis(diphenylphosphino)propane]nickel (II) dichloride (70 mg) and THF (35 ml).Upon completion of addition, the mixture was stirred at room temperature for 2 hours and then heated to reflux for 72 hours. The reaction mixture was cooled to room temperature and diluted with 100 ml of ethyl ether (Et₂O). The resultant mixture was washed with 5% hydrochloric acid (HCl), brine then dried with magnesium sulfate (MgSO₄) and concentrated in vacuo. The crude product was chromatographed on silica gel using hexanes: methylene chloride (5: 1) to afford 4.0 g (36%) of the title compound.

¹H NMR (CDCl₃) δ: 0.67-0.70 (m, 2H), 0.93-0.96 (m, 2H), 1.87-1.90 ( m, 1H), 5.04 (s, 2H), 6.69-6.71 (m, 2H), 6.77 (d, J=6 Hz, 1H), 7.17 (t, J=8 Hz, 1H), 7.32-7.45 (m, 5H).

### B. 1-Bromo-2-cyclopropyl-4-benzyloxy-benzene

Prepared as in Example 14B using 1-cyclopropyl-3-benzyloxy-benzene, in 84% yield.

¹H NMR ( CDCl₃) δ: 0.62-0.66 (m, 2H), 0.97-1.00 (m, 2H), 2.10-2.14 (m, 1H), 4.99 (s, 2H), 6.54 (d, J=3 Hz, 1H), 6.65 (d, J=4 Hz, 1H), 7.32-7.46 (m, 6H).

### C. 2-Cyclopropyl-4-benzyloxy-benzeneboronic acid

Prepared as in Example ID using 1-bromo-2-cyclopropyl-4-benzyloxy-benzene, in 98% yield as a pink oil. The crude product was not purified but directly converted into 2-(2-cyclopropyl-4-benzyloxy-phenyl)-6-(2,5-dimethyl-pyrrol-1-yl)-pyridine

¹H NMR (CDCl₃) δ: 0.68-0.75 (m, 2H), 0.92-0.98 (m, 2H), 2.09-2.13 (m, 1H), 5.08 (s, 2H), 6.69-6.84 (m, 2H), 7.39-7.45 (m, 5H), 8.08 (d, J=8 Hz, 1H).

### D. 2-(2-Cyclopropyl-4-benzyloxy-phenyl)-6-(2,5-dimethyl-pyrrol-1-yl)-pyridine

Prepared as in Example 1E using 2-cyclopropyl-4-benzyloxy-benzeneboronic acid with 2-bromo-6-(2,5-dimethyl-pyrrol-1-yl)-pyridine, in 50% yield.

¹H NMR (CDCl₃) δ: 0.65-0.67 (m, 2H), 0.82-0.86 (m, 2H), 2.04-2.11 (m, 1H), 2.17 (s, 6H), 5.07 (s, 2H), 5.88 (s, 2H), 6.62 (s, 1H), 6.84 (d, J=4 Hz, 1H), 7.14 (d, J=8 Hz, 1H), 7.32-7.44 (m, 6H), 7.54 (d, J=8 Hz, 1H), 7.83 (t, J=8 Hz, 1H).

MS (%). 395 (parent+1, 100).

### E. 3-Cyclopropyl-4-[6-(2,5-dimethyl-pyrrol-1-yl)-pyridin-2-yl]-phenol

Prepared as in Example 1F using 2-(2-cyclopropyl-4-benzyloxy-phenyl)-6-(2,5-dimethyl-pyrrol-1-yl)-pyridine with ammonium formate and 20% Pd(OH)₂, in 97% yield.

¹H NMR (CDCl₃) δ: 0.60-0.62 (m, 2H), 0.79-0.81 (m, 2H), 1.98-2.00 (m, 1H), 2.11 (s, 6H), 5.83 (s, 2H), 6.42 (s, 1H), 6.65 (d, J=6 Hz, 1H), 7.09 (d, J=8 Hz, 1H), 7.24 (d, J=8 Hz, 1H), 7.51 (d, J=8 Hz, 1H), 7.80 (t, J=8 Hz, 1H).

### F. 4-(6-Amino-pyridin-yl)-3-cyclopropyl-phenol

Prepared as in Example 1G using heating 3-cyclopropyl-4-[6-(2,5-dimethyl-pyrrol-1-yl)-pyridin-2-yl]-phenol with NH₂OH•HCl in aqueous EtOH, in 67% yield.

¹H NMR (CDCl₃) δ: 0.47-0.51 (m, 2H), 0.73-0.77 (m, 2H), 1.90-1.94 (m, 1H), 6.16 (s, 1H), 6.31 (dd, J₁=8 Hz, J₂=2.5 Hz, 1H), 6.41 (d, J=8 Hz, 1H), 6.80 (d, J=8 Hz, 1H), 7.07 (d, J=8 Hz, 1H), 7.46 (t, J=8 Hz, 1H).

¹³C NMR (CDCl₃) δ: 9.57, 13.18, 106.57, 111.21, 112.89, 115.14, 130.46, 138.19, 157.80.

MS (%): 227 (parent+1, 100).

### EXAMPLE 16

### 6-[2-CYCLOPROPYL-4-(2-DIMETHYLAMINO-ETHOXY)-PHENYL]-PYRIDIN-2-YLAMINE

Prepared as in Example 14G using of 4-(6-amino-pyridin-yl)-3-cyclopropyl-phenol and 2-dimethylaminoethyl chloride in a presence of Cs₂CO₃ in a boiling acetone (81% yield).

¹H NMR (CDCl₃, δ): 0.64-0.67 (m, 2H), 0.81-0.83 (m, 2H), 2.06-2.09 (m, 1H), 2.33 (s, 6H), 2.71 (t, J=6 Hz, 2H), 4.05 (t, J=6 Hz, 2H), 6.42 (d, J=8 Hz, 1H), 6.47 (s, 1H), 6.74 (d. J=8 Hz, 1H), 6.82 (d, J=8 Hz, 1H), 7.28 (d, J=8 Hz, 1H), 7.44 (t, J=8 Hz, 1H).

MS (%): 298 (parent+1 , 100).

### EXAMPLE 17

### 6-[2-CYCLOPROPYL-4-(2-PYRROLIDIN-1-YL-ETHOXY)-PHENYL]-PYRIDIN-2-YLAMINE

Prepared as in Example 14G using of 4-(6-amino-pyridin-yl)-3-cyclopropyl-phenol and 1-(2-chloroethyl)-pyrrolidine in a presence of Cs₂CO₃ in a boiling acetone (84 % yield).

¹H NMR (CDCl₃, δ): 0.63-0.66 (m, 2H), 0.80-0.84 (m, 2H), 1.77-1.81 (m, 4H), 2.07-2.10 (m, 1H), 2.59-2.62 (m, 4H), 4.10 (bs, 2H), 6.44 (d, J=8 Hz, 1H), 6.48 (s, 1H), 6.74 (d, J=8 Hz, 1H), 6.82 (d, J=8 Hz, 1H), 7.29 (d, J=8 Hz, 1H), 7.45 (t, J=8 Hz, 1H).

MS (%): 324 (parent+1, 100).

### EXAMPLE 18

### 3-[3-(6-AMINO-PYRIDIN-2YL)-4-CYCLOPROPYL-PHENOXY]-PYRROLIDINE-1-CARBOXYLIC ACID TERT-BUTYL ESTER

Prepared as in Example 29 using of 4-(6-amino-pyridin-yl)-3-cyctopropyl-phenol and 3-methanesulfonyloxy-pyrrolidine-1-carboxylic acid tert-butyl ester in a presence of KOt-Bu in DMSO (69% yield).

¹H NMR (CDCl₃, δ): 0.63-0.67 (m, 2H), 0.82-0.86 (m, 2H), 1.44 (s, 9H), 2.02-2.15 (m, 3H), 3.45-3.60 (m, 4H), 4.49 (bs, 2H), 4.87 (bs, 1H), 6.42-6.44 (m, 2H), 6.67 (d, J=8 Hz, 1H), 6.82 (d, J=8 Hz, 1H), 7.28 (d, J=8 Hz, 1H), 7.45 (t, J=8 Hz, 1H).

MS (%): 396 (parent+1, 100).

### EXAMPLE 19

### 6-[2-CYCLOPROPYL-4-(1-METHYL-PYRROLIDIN-3-YLOXY)-PHENYL]-PYRIDIN-2-YLAMINE

Prepared by a lithium aluminum hydride (LiAlH₄) reduction of 3-[3-(6-amino-pyridin-2yl)-4-cyclopropyl-phenoxy] -pyrrolidine-1-carboxylic acid tert-butyl ester, as described in Example 28, in 50% yield.

¹H NMR (CDCl₃ ) δ: 0.62-0.64 (m, 2H), 0.81-0.85 (m, 2H), 1.95-2.09 (m, 3H), 2.37 (s, 3H), 2.77-3.18 (m, 4H), 4.48 (bs, 2H), 4.81 (bs, 1H), 6.40-6.44 (m, 2H), 6.68 (d, J=8 Hz, 1H), 6.83 (d, J=8 Hz, 1H), 7.28 (d, J=8 Hz, 1H), 7.45 (t, J=8 Hz, 1H)

### EXAMPLE 20

### 4-(6-AMINO-PYRIDIN-2-YL)-3-CYCLOBUTYL-PHENOL

### A. 1-(3-Benzyloxy-phenyl)-cyclobutanol

In a flame-dried flask was placed magnesium and under a N₂ atmosphere added a solution of 1-bromo-3-benzyloxy-benzene (10 53 g, 40 mmol) in 30 ml of anhydrous ethyl ether. A resultant mixture was heated to reflux for 8 hours. The reaction mixture was then cooled to 0°C followed by a dropwise addition of cyclobutanone (J.A.C.S., 90, 3404-3415,1968) (2.96 ml, 40 mmol) in 10 ml of anhydrous ethyl ether. The reaction was stirred at room temperature for 30 minutes and then cooled to 0°C and hydrolyzed with aqueous ammonium chloride (NH₄Cl) (20 ml). The organic extract was dried (MgSO₄) and concentrated in vacuo. The crude product was chromatographed on 300 g silica gel using hexanes-ethyl acetate 3:1 to afford 8.5 g (84%) of the title compound as a yellow oil.

¹H NMR (CDCl₃) δ: 1.60-1.66 (m, 1H), 2.03-2.11 (m, 1H), 2.33-2.36 (m, 2H), 2.50-2.54 (m, 2H), 5.07 (s, 2H), 6.88 (d, J=8 Hz, 1H), 7.09 (d, J=8 Hz, 1H), 7.13 (bs, 1H), 7.28-7.45 (m,3H).

### B. 3-Cyclobutyl-phenol

Under a N₂ atmosphere in 50 ml of ethanol (EtOH) were combined 1-(3-benzyloxy-phenyl)-cyclobutanol (6g, 23.6 mmol) and 10% palladium on carbon (Pd/C) (1.5 g). A resultant mixture was hydrogenated (J.A.C.S., 90, 3404-3415,1968) at 40 psi for 24 hours. The reaction mixture was filtered through a pad of celite and concentrated under vacuo. The crude product was chromatographed on 120 g of silica gel using hexanes-ethyl acetate to afford 2.9 g (83%) of the title comound as a colorless oil.

¹H NMR (CDCl₃) δ: 1.81-1.86 (m, 1H), 1.95-2.02 (m, 1H), 2.08-2.14 (m, 2H), 2.29-2.34 (m, 2H), 3.49 (q, J=8 Hz, 1H), 6.63 (d, J=6 Hz, 1H), 6.69 (bs, 1H), 6.77 (d, J=6 Hz, 1H), 7.15 (t, J=8 Hz, 1H).

### C. 1-Cyclobutyl-3-benzyloxy-benzene

Prepared as in Example 1C using 3-cyclobutyl-phenol, in 98% yield.

¹H NMR (CDCl₃) δ: 1.81-1.86 (m, 1H), 1.98-2.02 (m, 1H), 2.11-2.15- (m, 2H), 2.30-2.34 (m, 2H), 3.52 (q, J=8 Hz, 2H), 5.05 (s, 2H), 6.78-6.86 (m, 3H), 7.21 (t, J=8 Hz, 1H), 7.32-7.45 (m, 5H).

### D. 1-Bromo-2-cyclobutyl-4-benzyloxy-benzene

Prepared as in Example 14B using 1-cyclobutyl-3-benzyloxy-benzene, in 97% yield.

¹H NMR (CDCl₃) δ: 1.81-1.85 (m, 1H), 2.04-2.11 (m, 3H), 2.41-2.44 (m, 2H), 3.73 (q, J=8 Hz, 1H), 5.05 (s, 2H), 6.68 (d, J=8 Hz, 1H), 6.98 (bs, 1H), 7.35-7.46 (m, 6H).

¹³C NMR (CDCl₃) δ: 17.84, 28.60, 40.64, 70.19, 113.09, 114.45, 114.85, 127.45, 127.99, 128.55, 133.02, 136.68, 145.51, 158.17.

### E. 2-Cyclobutyl-4-benzyloxy-benzeneboronic acid

Prepared as in Example 1D using 1-bromo-2-cyclobutyl-4-benzyloxy-benzene, as a beige solid in 58% yield.

¹H NMR (CDCl₃) δ: 1.81-1.85 (m, 1H), 1.98-2.03 (m, 1H), 2.10-2.15 (m, 2H), 2.33-2.36 (m, 2H), 3.86 (q, J=8 Hz, 1H), 6.78 (d, J=8 Hz, 1H), 7.00 (bs, 1H ), 7.38-7.74 (m, 6H).

### F. 2-(2-Cyclobutyl-4-benzyloxy-phenyl)-6-(2,5-dimethyl-pyrrol-1-yl)-pyridine

Prepared as in Example 1E using 2-cyclobutyl-4-benzyloxy-benzeneboronic acid and 2-bromo-6-(2,5-dimethyl-pyrrol-1-yl)-pyridine, in 78% yield.

¹H NMR (CDCl₃) δ: 1.69-1.74 (m, 1H), 1.77-1.82 (m, 1H), 1.96-2.01 (m, 4), 2.16 (s, 6H), 3.91 (q, J=8 Hz, 1H), 5.11 (s, 2H), 5.87 (s, 2H), 6.84 (d, J=8 Hz, 1H), 7.02 (bs, 1H), 7.13 (d, J=8 Hz, 1H), 7.24-7.46 (m, 7H), 7.81 (t, J=8 Hz, 1H).

MS (%): 409 (parent+1, 100).

### G. 3-Cyclobutyl-4-[6-(2,5-dimethyl-pyrrol-1-yl)-pyridin-2-yl]-phenol

Prepared as in Example 1F using 2-(2-cyclobutyl-4-benzyloxy-phenyl)-6-(2,5-dimethyl-pyrrol-1-yl)-pyridine, in 97% yield.

¹H NMR (CDCl₃) δ: 1.71-1.79 (m, 1H), 1.1.79-1.84 (m, 1H), 1.95-1.99 (m, 4H), 2.16 (s, 6H), 5.88 (s, 2H), 6.75 (d, J=8 Hz, 1H), 6.84 (bs, 1H), 7.13 (d, J=8 Hz, 1H), 7.21 (d, J=8 Hz, 1H), 7.30 (d, J=8 Hz, 1H), 7.82 (t, J=8 Hz, 1H).

MS (%): 319 (parent+1, 100).

### H. 4-(6-Amino-pyridin-2-yl)-3-cyclobutyl-phenol

Prepared by heating 2-(2-cyclobutyl-4-benzyloxy-phenyl)-6-(2,5-dimethyl-pyrrol-1-yl)-pyridine with NH₂OH•HCl in aqueous EtOH, as described in Example 1F, as a off-white solid, in 61 % yield.

¹H NMR (CDCl₃) δ: 1.62-1.66 (m, 1H), 1.72-1.78 (m, 1H), 1.92-1.97 (m, 4H), 3.65 (q, J=8 Hz, 1H), 6.37 (d, J=8 Hz, 1H), 6.54 (d, J=8 Hz, 1H), 6.58 (d, J=8 Hz, 1H), 6.79 (bs, 1H), 7.03 (d, J=8 Hz, 1H), 7.39 (t, J=8 Hz, 1H).

MS (%): 241 (parent+1, 100).

### EXAMPLE 21

### 6-[2-CYCLOBUTYL-4-(2-DIMETHYLAMINO-ETHOXY)-PHENYL]-PYRIDIN-2-YLAMINE

Prepared as in Example 14G using 4-(6-amino-pyridin-2-yl)-3-cyclobutyl-phenol and 2-dimethylaminoethyl chloride, as a pale yellow oil in 77% yield.

¹H NMR (CDCl₃) δ: 1.69-1.86 (m, 2H), 2.00-2.06 (m, 4H), 2.33 (bs, 6H), 2.73 (t, J=6 Hz, 2H), 3.80 (q, J=8 Hz, 1H), 4.10 (t, J=6 Hz, 2H), 4.43 (bs, 2H), 6.42 (d, J=8 Hz, 1H), 6.64 (d, J=8 Hz, 1H), 6.75 (d, J=8 Hz, 1H), 6.98 (bs, 1H), 7.21 (d, J=8 Hz, 1H), 7.43 (t, J=8 Hz, 1H)

¹³C NMR (CDCl₃) δ: 17.91, 29.83, 38.26, 45.83, 58.27, 66.11, 105.95, 111.06, 113.43, 114.36, 130.23, 137.45.

MS (%): 312 (parent+1, 100).

### EXAMPLE 22

### 6-[2-CYCLOBUTYL-4-(2-PYRROLIDIN-1-YL-ETHOXY)-PHENYL]-PYRIDIN-2-YLAMINE

Prepared as in Example 14G using 4-(6-amino-pyridin-2-yl)-3-cyclobutyl-phenol and 1-(2-chloroethyl)-pyrrolidine in 69% yield.

¹H NMR (CDCl₃) δ: 1.69-1.86 (m, 5H), 1.99-2.06 (m, 4H), 2.61-2.64 (m, 4H), 2.91 (t, J=6 Hz, 2H), 3.80 (q, J=8 Hz, 1H), 4.14 (t, J=6 Hz, 2H), 4.43 (bs, 2H), 6.41 (d, J=8 Hz, 1H), 6.63 (d, J=8 Hz, 1H), 6.75 (d, J=8 Hz, 1H), 6.97 (bs, 1H), 7.20 (d, J=8 Hz, 1H), 7.43 (t, J=8 Hz, 1H).

¹³C NMR (CDCl₃) δ: 17.91, 23.43, 38.27, 54.63, 55.04, 66.81, 106.26, 115.12, 113.34, 114,36, 130.24, 137.79

MS (%): 338 (parent+1, 100).

### EXAMPLE 23

### 6-[2-CYCLOBUTYL-4-(1-METHYL-PYRROLIDIN-3-YLOXY)-PHENYL]-PYRIDIN-2-YLAMINE

### A. 3-[3-(6-Amino-pyridin-2-yl)-4-cyclobutyl-phenoxy]-pyrrolidine-1-carboxylic acid tert-butyl ester

Prepared as in Example 29 using 4-(6-amino-pyridin-2-yl)-3-cyclobutyl-phenol and 3-methanesulfonyloxy-pyrrolidine-1-carboxylic acid tert-butyl ester, (88% yield).

¹H NMR (CDCl₃) δ: 1.45 (s, 9H), 1.70-1.79 (m, 1H), 1.82-1.87 (m, 1H), 2.00-2.09 (m, 5H), 2.17-2.22 (m, 1H), 3.45-3.60 (m, 4H), 3.79 (q, J=9 Hz, 1H), 4.52 (bs, 2H), 4.92 (bs, 1H), 6.43 (d, J=8 Hz, 1H), 6.66 (d, J=8 Hz, 1H), 6.71 (d, J=8 Hz, 1H), 6.90 (bs, 1H), 7.20-7.24 (m, 1H), 7.44 (t, J=8 Hz, 1H)

### B. 6-[2-Cyclobutyl-4-(1-methyl-pyrrolidin-3-yloxy)-phenyl]-pyridin-2-ylamine

Prepared by a LiAlH₄ reduction of 3-[3-(6-amino-pyridin-2-yl)-4-cyclobutyl-phenoxy]-pyrrolidine-1-carboxylic acid tert-butyl ester, as described in Example 28, in 73 % yield.

¹H NMR (CDCl₃) δ: 1.67-1.71 (m, 1H), 1.78-1.87 (m, 1H), 1.97-2.04 (m, 4H), 2.29-2.38 (m, 1H), 2.39 (s, 9H), 2.43-2.49 (m, 1H), 2.79-2.84 (m, 4H), 3.78 (q, J=9 Hz, 1H), 4.43 (bs, 2H), 4 84-4.88 (m, 1H), 6.42 (d, J=8 Hz, 1H), 6.64-6.68 (m, 2H), 6.90 (sb, 1H), 7.19 (d, J=8 Hz, 1H), 7.42 (t, J=8 Hz, 1H)

¹³C NMR (CDCl₃) δ: 19.09, 29.93, 32.88, 38.12, 42.15, 55.16, 62.41, 76.81, 106.09, 111.68, 114.44, 130.29, 137.68, 145.41.

MS (%): 324 (parent+1, 100)

### EXAMPLE 24

### 4-(6-AMINO-PYRIDIN-2-YL)-3-CYCLOPENTYL-PHENOL

### A. 1-(3-Benzyloxy-phenyl)-cyclopentanol

To a flame-dried flask containing magnesium (Mg) was added a solution of 1-bromo-3-benzyloxy-benzene (10.53 g, 40 mmol) in 40 ml of anhydrous ethyl ether. Under a N₂ atmosphere the resultant mixture was heated to reflux for 8 hours. The reaction mixture was cooled to 0°C, followed by a dropwise addition of cyclopentanone (J.A.C.S., 90, 3404-3415, 1968) (3.54 ml, 40 mmol) in 10 ml of anhydrous ethyl ether. The reaction was stirred at room temperature for 30 minutes, then cooled to 0°C and hydrolyzed by aqueous ammonium chloride (NH₄Cl) (20 ml). The organic extract was dried (MgSO₄) and concentrated in vacuo. The crude product was chromatographed on 300 g silica gel using hexanes-ethyl acetate (EtOAc) 3:1 to afford 4g (37%) of the title compound as a pale yellow oil.

¹H NMR (CDCl₃) δ: 1.79-1.84 (m, 2H), 1.94-2.02 (m, 6H), 5.06 (s, 2H), 6.85 (d, J=8 Hz, 1H), 7.07 (d, J=8 Hz, 1H), 7.15 (bs, 1H), 7.23-7.44 (m, 6H).

### B. 3-Cyclopentyl-phenol

Under a N₂ atmosphere in 30 ml of EtOAc were combined 1-(3-benzyloxy-phenyl)-cyclopentanol (2.8 g, 10.4 mmol), 3 drops of concentrated HCl, and 10% Pd/C (1 g). A resultant mixture was hydrogenated (Tetrahedran Assymetry, 1360, 1993) at 40 psi for 2 hours. The reaction mixture was filtered through a pad of celite and concentrated under vacuo to afford 1.3 g (77%) of the title compound as an oil.

¹H NMR (CDCl₃) δ: 1.56-1.79 (m, 6H), 1.99-2.04 (m, 1H), 2.93 (q, J=8 Hz, 1H), 6.62 (d, J=8 Hz, 1H), 6.71 (bs, 1H), 6.80 (d, J=8 Hz, 1H), 7.13 (d, J=8 Hz, 1H).

### C. 1-Cyclopentyl-3-benzyloxy-benzene

Prepared by heating 3-cyclopentyl-phenol with benzyl bromide and potassium carbonate (K₂CO₃) in acetone, as described in Example 1C, to afford the title compound in 99% yield.

¹H NMR (CDCl₃) δ: 1.54-1.79 (m, 6H), 2.03-2.06 (m, 2H), 2.96 (q, J=8 Hz, 1H), 5.04 (s, 2H), 6.78 (d, J=8 Hz, 1H), 6.84-6.89 (m, 2H), 7.19 (t, J=8 Hz, 1H), 7.30-7.45 (m, 5H).

### D. 1-Bromo-2-cyclopentyl-4-benzyloxy-benzene

Prepared by an NBS bromination of 1-cyclopentyl-3-benzyloxy-benzene, as described in Example 14B, in 76% yield.

¹H NMR (CDCl₃) δ: 1.49-1.53 (m, 2H), 1.66-1.80 (m, 4H), 2.03-2.09 (m, 2H), 3.34 (q, J=8 Hz, 1H), 5.01 (s, 2H), 6.65 (d, J=6 Hz, 1H), 6.90 (s, 1H), 7.31-7.41 (m, 6H).

### E. 2-Cyclopentyl-4-benzyloxy-benzeneboronic acid

Prepared by lithiation of 1-bromo-2-cyclopentyl-4-benzyloxy-benzene with n-BuLi followed by addition of B(OEt)₃, as described in Example 1D, in 80% yield.

¹H NMR (CDCl₃) δ: 1.56-1.80 (m, 6H), 2.02-2.08 (m, 2H), 2.91-2.99 (m, 1H), 5.04 (s, 2H), 6.77 (d, J=8 Hz, 1H), 6.79-6.87 (m, 2H), 7.16-7.46 (m, 5H).

### F. 2-(2-Cyclopentyl-4-benzyloxy-phenyl)-6-(2,5-dimethyl-pyrrol-1-yl)-pyridine

Prepared by a Pd cross-coupling of 2-cyclopentyl-4-benzyloxy-benzeneboronic acid with 2-bromo-6-(2,5-dimethyl-pyrrol-1-yl)-pyridine, as described in Example 1E, in 58 % yield.

¹H NMR (CDCl₃) δ: 1.55-1.60 (m, 4H), 1.74-1.78 (m, 2H), 1.91-1.95 (m, 2H), 2.17 (s, 6H), 3.30 (q, J=8 Hz, 1H), 5.10 (s, 2H), 5.89 (s, 2H), 6.86 (d, J=8 Hz, 1H), 7.03 (s, 1H), 7.16 (d, J=8 Hz, 1H), 7.25-7.47 (m, 7H), 7.84 (t, J=8 Hz, 1H).

MS (%): 423 (parent+1, 100).

### G. 3-Cyclopentyl-4-[6-(2,5-dimethyl-pyrrol-1-yl)-pyridin-2-yl]-phenol

Prepared by a reduction of 2-(2-cyclopentyl-4-benzyloxy-phenyl)-6-(2,5-dimethyl-pyrrol-1-yl)-pyridine with ammonium formate and 20% palladium hydroxide on carbon (Pd(OH)₂ on C), as described in Example 1F, in 48% yield.

¹H NMR (CDCl₃) δ: 1.51-1.55 (m, 4H), 1.74-1.79 (m, 2H), 1.88-1.91 (m, 2H), 2.14 (s, 6H), 3.27 (q, J=8 Hz, 1H), 5.87 (s, 2H), 6.68 (d, J=8 Hz, 1H), 6.85 (bs, 1H), 7.15 (d, J=8 Hz, 1H), 7.23 (d, J=8 Hz, 1H), 7.33 (d, J=8 Hz, 1H), 7.83 (t, J=8 Hz, 1H).

MS (%): 333, (parent+1, 100).

### H. 4-(6-Amino-pyridin-2-yl)-3-cyclopentyl-phenol

Prepared by heating 3-cyclopentyl-4-[6-(2,5-dimethyl-pyrrol-1-yl)-pyridin-2-yl]-phenol with NH₂OH·HCl in aqueous ethanol, as described in Example 1G, in 61% yield.

¹H NMR (CDCl₃) δ; 1.45-1.53 (m,4H), 1.61-1.70 (m, 2H), 1.86-1.93 (m, 2H), 3.08 (q, J=8 Hz, 1H), 4.64 (bs, 2H), 6.35 (d, J=8 Hz, 1H), 6.43 (d, J=8 Hz, 1H), 6.63 (d, J=8 Hz, 1H), 6.74 (bs, 1H), 7.02 (d, J=8 Hz, 1H), 7.45 (t, J=8 Hz, 1H).

MS (%): 255 (parent+1, 100).

### EXAMPLE 25

### 6-[2-CYCLOPENTYL-4-(2-DIMETHYLAMINO-ETHOXY)-PHENYL]-PYRIDIN-2-YLAMINE

Prepared by an alkylation of 4-(6-amino-pyridin-2-yl)-3-cyclopentyl-phenol with 2-dimethylaminoethyl chloride in a presence of Cs₂CO₃ in a boiling acetone, as described in Example 14G, (67% yield).

¹H NMR (CDCl₃) δ: 1.53-1.74 (m, 6H), 1.91-1.95 (m, 2H), 2.32 (s, 6H), 2.71 (t, J=6 Hz. 2H), 3.16 (q, J=8 Hz, 1H), 4.06 (t, J=6 Hz, 2H), 4.43 (bs, 2H), 6.42 (d, J=8 Hz, 1H), 6.66 (d, J=7 Hz, 1H), 6.74 (d, J=8 Hz, 1H ), 6.92 (bs, 1H), 7.20 (d, J=8 Hz, 1H), 7.43 (t, J=8 Hz, 1H).

¹³C NMR (CDCl₃) d 25.98, 35.42, 41.66, 45.92, 58.33, 65.82, 106.10, 110.86, 113.13, 114.61, 130.36, 137.61, 146.31, 157.92, 158.82.

MS (%): 326 (parent+1, 100).

### EXAMPLE 26

### 6-[2-CYCLOPENTYL-4-(2-PYRROLIDIN-1YL-ETHOXY)-PHENYL]-PYRIDIN-2-YLAMINE

Prepared as in Example 14G using 4-(6-amino-pyridin-2-yl)-3-cyclopentyl-phenol and 1-(2-chloroethyl)-pyrrolidine in 43% yield.

¹H NMR (CDCl₃) δ: 1.53-1.95 (m, 12 H), 2.63 (bs, 4H), 2.90 (t, J=6 Hz, 2H), 3.18 (q, J=8 Hz, 1H), 4.12 (t, J=6 Hz, 2H), 4.45 (bs, 2H), 6.41 (d, J=8 Hz, 1H), 6.65 (d, J=7 Hz, 1H), 6.74 (d, J=7 Hz, 1H), 6.91 (bs, 1H), 7.19 (d, J=8 Hz, 1H), 7.42 (t, J=8 Hz, 1H).

¹³C NMR (CDCl₃) δ: 23.47, 25.97, 35.43, 41.67, 54.70, 55.09, 66.84, 106.10, 111.05, 112.99, 114.62, 130.39, 137.61, 146.28, 157.87, 158.77.

MS (%): 352 (parent+1, 100).

### EXAMPLE 27

### 3-[4-(6-AMINO-PYRIDIN-2YL)-3-METHOXY-PHENOXY]-PYRROLIDINE-1-CARBOXYLIC ACID TERT BUTYL ESTER

Under a N₂ atmosphere in 20 mL of anhydrous THF was combined 173 mg ( 0.92 mmol) of (R)-N-BOC-3-hydroxy-pyrrolidine, 200 mg (0.92 mmol) of 4-(6-amino-pyridin-2-yl)-3-methoxy-phenol and 267 mg ( 1.02 mmol) of triphenylphosphine. The reaction was allowed to cool to 0°C and with stirring 160 ul of diethylazodicarboxylate (1.02 mmol) was added. The reaction mixture was allowed to warm to ambient temperature and the reaction was stirred for 18 hours at which point the reaction mixture was concentrated *in vacuo* and redissolved into ethyl acetate (150 mls) . The organic layer was washed with 1M NaOH (2 X 100 mL), with brine (1 X 100 mL), dried over sodium sulfate, filtered and concentrated *in vacuo* to yield crude product which was chromatographed on 40 g of silica gel 60 (EM Science) using 2:1 ethyl acetate:hexane to afford 397 mg of crude product (the title compound) which was carried directly into the next step.

### EXAMPLE 28

### 6-[4-(1-METHYL-PYRROLIDIN-3-YLOXY)-2-METHOXY-PHENYL]-PYRlDIN-2-YLAMINE

Under a N₂ atmosphere in 15 mL of anhydrous THF was added 357 mg (0.92 mmol) of crude aminopyridine 3-[4-(6-amino-pyridin-2yl)-3-methoxy-phenoxy]-pyrrolidine-1-carboxylic acid tert butyl ester and 2.31 ml (2.31 mmol) of a 1.0 M solution of lithium aluminum hydride. The reaction mixture was heated to reflux for 2 hours and then cooled to ambient temperature. The reaction mixture was carefully quenched with 88 ul of water, 88 ul of 1N NaOH and 264 ul of water. The aluminum salts were filtered and washed with ethyl acetate and the filtrate was concentrated in vacuo to yield 290 mg of crude product as a greenish-yellow oil which was chromatographed on 25 g of silica gel 60 (EM Science) using 95:5:.05 dichloromethane:methanol:ammomium hydroxide to afford 85 mg ( 31 %) of the title compound as colorless oil, which was converted to 79 mg of HCl salt by dissolving in dichloromethane and adding 1 ml of an ether solution saturated with HCl and concentrating and triturating with ethyl acetate.

¹H NMR (CDCl₃) δ 1.98-2.03 (m-1H), 2.28-2.44 (m-2H), 2.38 (s-3H), 2.74-2.86 (m-3H), 3.78 (s-3H), 4.42 (bs-2H), 4.84-4.87 (m-1H), 6.37 (dd-1H, J = 0.83; J = 8.09), 6.45-6.51 (m-2H), 7.12 (dd-1H; J = 0.83, J = 7.68 Hz), 7.40-7.44 (m-1H), 7.63 (d-1H; J = 8.51H z).

### EXAMPLE 29

### 4-[4-(6-AMINO-PYRIDIN-2YL)-3-METHOXY-PHENOXY]-PIPERIDINE-1-CARBOXYLIC ACID TERT BUTYL ESTER

Under a N₂ atmosphere in 15 mL of anhydrous DMSO was combined 57 mg (0.51 mmol) of potassium t-butoxide followed by 100 mg ( 0.46 mmol) of 4-(6-amino-pyridin-2-yl)-3-methoxy-phenol. N-BOC-4-hydroxy-piperidine mesylate (142 mg, 0.51 mmol) was then added and the resultant mixture was heated to 105°C for 4.5 hours. Another 142 mg (0.51 mmol) of mesylate was then added and the reaction was heated for an additional 75 minutes. The reaction was allowed to cool to ambient temperature and water (100 mls) was added.The aqueous solution was extracted with ethyl acetate (2X150 mls). The organic layer was washed with water (2X100 mls), 1M NaOH (2 X 100 mL), with brine (1 X 100 mL), dried over sodium sulfate, filtered and concentrated *in vacuo* to yield crude product which was chromatographed on 30 g of silica gel 60 (EM Science) using 2:1 ethyl acetate:hexane to afford 210 mg of crude product (the title compound) which was carried directly into the next step.

### EXAMPLE 30

### 6-[2-METHOXY-4-(1-METHYL-PIPERIDIN-4-YLOXY)-PHENYL]-PYRIDIN-2-YLAMINE

Lithium aluminum hydride reduction 4-[4-(6-amino-pyridin-2yl)-3-methoxy-phenoxy]-piperidine-1-carboxylic acid tert butyl ester as described above for the reduction of 3-[4-(6-amino-pyridin-2yl)-3-methoxy-phenoxy]-pyrrolidine-1-carboxylic acid tert butyl ester provided, after silica gel chromatography (95:5:0.05: CH₂Cl₂: MeOH: NH₄OH), 65 mg(45%-for two steps) of the title compound.

¹H NMR (CDCl₃) δ 1.81-2.03 (m-4H), 2.29 (s-3H), 2.26-2.30 (m-2H), 2.68 (m-2H), 3.79 (s-3H), 4.33-4.43 (m-3H), 6.37 (dd-1H; J = 0.62 Hz; J = 8.10 Hz), 6.51-6.57 (m-2H), 7.11 (dd-1H; J = 0.62 Hz; J = 7.68 Hz), 7.41 (t-1H; J = 7.68 Hz), 7.61 (d-1H; J = 8.52 Hz).

### EXAMPLE 31

### 6-[4-(ALLYLOXY)-2-METHOXY-PHENYL]-PYRIDIN-2-YLAMINE

Under a N₂ atmosphere in 75 mL of acetone was combined 3.00 g (13.87 mmol) of 4-(6-amino-pyridin-2-yl)-3-methoxy-phenol and 9.04 g (27.75 mmol) of cesium carbonate followed by 3.39 mL (41.62 mmol) of allyl chloride. The reaction was allowed to heat at 45°C with stirring for 16 hours and concentrated *in vacuo*. The solid residue was partitioned between ethyl acetate (200 mL) and water (200 mL). The organic layer was washed with brine (1 X 100 mL), dried over sodium sulfate, filtered and concentrated *in vacuo* to yield product as a yellow solid which was triturated with hexane anf filtered to afford 3.24 g (91 %) of crude product (the title compound) as a pale yellow solid.

¹H NMR (CDCl₃) δ 3.80 (s-3H), 4.45 (bs-2H), 4.55 (d-2H, J = 5.19 Hz), 5.28 (d-1H; J = 10.58 Hz), 5.41 (d-1H; J = 17.22 Hz), 6.05 (m-1H), 6.38 (d-1H; J = 8.09 Hz), 6.55 (m-2H), 7.11 (d-1H; J = 7.68 Hz),7.42 (t-1H; J = 7.67 Hz)., 7.64 (d-1H; J = 8.30 Hz).

### EXAMPLE 32-33

### 4-(6-AMINO-PYRIDIN-2-YL)-3-METHOXY-6-ALLYL-PHENOL AND 4-(6-AMINO-PYRIDIN-2-YL)-3-METHOXY-2-ALLYL-PHENOL

Under a N₂ atmosphere in a round bottom flask equipped with a stir bar was added 4-(6-amino-pyridyl-2-yl)-3-methoxyphenol and allyl ether. The reaction vessel was evacuated under reduced pressure and was then purged with nitrogen gas. The reaction vessel was immersed in an oil bath heated to 230°C and was allowed to stir for 20 minutes at this temperature. Analysis after cooling by TLC( 2:1 ethyl acetate: hexane) revealed some starting ether. The reaction vessel was immersed in an oil bath heated to 230°C for an additional 20 minutes The resultant brown oil was taken up in a methanol/ ethyl acetate solution and combined with 15 g of silica gel 60 (EM Science). This mixture was concentrated *in vacuo* and the resultant brown powder was placed on the head of a silica gel (150 g) column and chromatographed using 3.2 ethyl acetate: hexane to afford 1.4 g of crude 6-allyl phenol contaminated with some 2-allyl phenol. Crude 6-allyl phenol was rechromatographed using 1:1 ethyl acetate: hexane to afford 1.05 g (33 %) of 6-allyl phenol as a pale yellow solid.

¹H NMR (CDCl₃) δ 3.32 (d-2H; J = 6.22 Hz), 3.38 (s-3H), 4.68 (bs-2H), 5.03 (m-1H), 5.10 (m-1H), 5.95 (m-1H), 6.17 (s-1H), 6.37 (m-1H), 6.95 (m-1H), 7.28 (s-1H), 7.44 (m-1H).

¹H NMR (CDCl₃) 3.44 (s-3H), 3.46 (d-2H; J = 5.82 Hz), 4.59 (bs-2H), 5.03 (m-2H), 6.02 (m-1H), 6.38 (m-2H), 7.07 (d-1H; J = 7.68 Hz), 7.24 (m-1H), 7.42 (m-1H).

### EXAMPLE 34

### 4-(6-AMINO-PYRIDIN-2-YL)-3-METHOXY-6-PROPYL-PHENOL

Under a N₂ atmosphere in a Parr bottle was dissolved 1.20 g (4.682 mmol) of 4-(6-amino-pyridin-2-yl)-3-methoxy-6-allyl-phenol in 25 mL of absolute ethanol. The ethanol solution was hydrogenated (50 PSI) for 45 minutes at ambient temperature. The reaction mixture was then filtered through a pad of celite which was washed with additional methanol. The combined filtrates were concentrated *in vacuo* to afford 1.20 g (99%) of the desired product.

¹H NMR (CD₃OD) δ 0.94 (t-3H: J = 7.47 Hz), 1.58 (m-2H), 2.52 (m-2H), 3.73 (s-3H), 6.42 (dd-1H; J =0.83 Hz; J = 8.30 Hz), 6.47 (s-1H), 6.88 (dd-1H; J = 0.83 Hz; J = 7.47 Hz), 7.19 (s-1H), 7.40 (dd-1H, J = 7.47 Hz; J = 8.09 Hz).

### EXAMPLE 35

### 6-[4-(2-DIMETHYLAMINO-ETHOXY)-2-METHOXY-5-PROPYL-PHENYL]-PYRIDIN-YLAMINE

Under a N₂ atmosphere in 20 mL of acetone was combined 150 mg (0.58 mmol) of 4-(6-amino-pyridin-2-yl)-3-methoxy-6-propyl-phenol and 819 mg (2.32 mmol) of cesium carbonate followed by 125 mg (0.87 mmol) of N-(2-chloroethyl)dimethylamine hydrochloride. The reaction was allowed to reflux with stirring for 16 hrs and concentrated *in vacuo*. The solid residue was partitioned between ethyl acetate (150 ml) and H₂O. The organic extract was washed with brine (1 X 100 mL), dried over sodium sulfate, filtered and concentrated *in vacuo* to yield crude product which was chromatographed on 25 g of silica gel 60 (EM Science) using 9:1 dichloromethane:methanol to afford 131 mg ( 69 %) of aminopyridine as a pale yellow solid. One hundred forty-five mg of the corresponding hydrochloride salt of the title compound was prepared by dissolving the title compound in dichloromethane and adding diethyl ether saturated with HCl. The cloudy solution was concentrated *in vacuo*, isopropyl alcohol was added, and the solution was again concentrated *in vacuo* to provide a solid which was triturated with ethyl acetate.

¹H NMR (CDCl₃) δ 0.93 (t-3H; J = 7.47 Hz), 1.60 (m-2H), 2.40 (s-6H), 2.55 (m-2H), 2.74 (t-2H, J = 6.02 Hz), 3.82 (s-3H), 4.14 (t-2H; J = 6.02 Hz), 4.48 (bs-2H), 6.39 (d-1H; J = 8.09 Hz), 6.50 (s-1H), 7.14 (d-1H; J = 7.67 Hz), 7.43 (t-1H; J = 7.68 Hz), 7.51 (s-1H).

The title compounds of Example 36-42 were prepared using the procedures described in Example 27-30.

### EXAMPLE 36

### 6-[2-ISOPROPYL-4-(PYRROLIDIN-3-YLOXY)-PHENYL]-PYRIDIN-2-YLAMINE

¹H NMR (CDCl₃) δ 1.13 (d-6H; J = 6.86 Hz), 1.92-2.11 (m-2H), 2.43 (bs-2H), 2.84-3.22 (m-5H), 4.53 (bs-2H), 4.81-4.84 (m-1H), 6.38 (dd-1H; J = 0.62 Hz, J = 8.10 Hz), 6.60-6.69 (m-2H), 6.83 (d-1H; J = 2.49 Hz), 7.17 (d-1H; J= 8.52 Hz), 7.41 (t-1H; J = 7.47 Hz).

### EXAMPLE 37

### 6-[2-ISOPROPYL-4-(PIPERIDIN-3-YLOXY)-PHENYL]-PYRIDIN-2-YLAMINE

¹H NMR (CDCl₃) δ 1.14 (d-6H, J = 6.85 Hz), 1.22-1.27 (m-1H), 1.40-1.55 (m-1H), 1.71-1.84 (m-2H), 1.97-2.02 (m-1H), 2.20 (bs-1H), 2.72-2.78 (m-3H), 3.15-3.22 (m-2H), 4.14-4.32 (m-2H), 4.47 (bs-2H), 6.42 (dd-1H; J = 0.83 Hz, J = 8.33 Hz), 6.65 (dd-1H; J = 0.83 Hz; J = 7.48 Hz), 6.75 (dd-1H; J = 2.71 Hz; J = 8.51 Hz), 6.89 (d-1H; J = 2.50 Hz), 7.18 (d-1H; J = 8.31 Hz), 7.44 (dd-1H; J = 7.48 Hz, J = 8.10 Hz).

### EXAMPLE 38

### 6-[2-ISOPROPYL-4-(1-METHYL-AZETIDIN-3-YLOXY)-PHENYL]-PYRIDIN-2-YLAMINE

¹H NMR (CDCl₃) δ 1.12 (d-6H, J = 6.85 Hz), 2.40 (s-3H), 3.10 (m-2H), 3.16-3.22 (m-1H), 3.83 (m-2H), 4.47 (bs-2H), 4.73-4.79 (m-1H), 6.40 (d-1H; J = 8.09 Hz), 6.55 (dd-1H; J = 2.50 Hz; J = 8.30 Hz). 6.63 (d-1H; J = 7.47 Hz), 6.79 (d-1H; J = 2.70 Hz), 7.17 (d-1H, J = 8.30 Hz), 7.42 (t-1H; J = 7.68 Hz).

### EXAMPLE 39

### 6-[2-ISOPROPYL-4-(1-METHYL-PIPERIDIN-4-YLOXY)-PHENYL]-PYRIDIN-2-YLAMINE

¹H NMR (CDCl₃) δ 1.15 (d-6H; J = 6.85 Hz), 1.82-1.90 (m-1H), 2.00-2.05 (m-1H), 2.31 (s-3H), 2.29-2.33 (m-2H), 2.70 (m-2H), 3.16-3.23 (m-1H), 4.34-4.45 (m-3H), 6.42 (dd-1H; J = 0.62 Hz; J = 8.10 Hz), 6.65 (dd-1H; J = 0.62 Hz; J = 7.47 Hz), 6.74 (dd-1H; J = 2.70 Hz; J = 8.51 Hz), 6.88 (d-1H; J = 2.70 Hz), 7.18 (d-1H; J = 8.52 Hz), 7.44 (dd-1H; J = 7.27 Hz; J = 8.10 Hz).

### EXAMPLE 40

### 6-[2-ISOPROPYL-4-(1-METHYL-PYRROLIDIN-3-YLOXY)-PHENYL]-PYRIDIN-2-YLAMINE

¹H NMR (CDCl₃) δ 1.12 (d-6H; J = 6.85 Hz), 1.98-2.02 (m-1H), 2.28-2.47 (m-2H), 2.38 (s-3H), 2.80-2.84 (m-3H), 3.15-3.20 (m-1H), 4.49 (bs-2H), 4.83-4.85 (m-1H), 6.38-6.41 (m-1H), 6.62-6.66 (m-2H), 6.85 (d-1H; J = 2.50 Hz), 7.17 (d-1H; J = 8.31 Hz), 7.39-7.43 (m-1H).

### EXAMPLE 41

### 6-[2-ISOPROPYL-4-(1-METHYL-PYRROLIDIN-3-YLOXY)-PHENYL]-PYRIDIN-2-YLAMINE

¹H NMR (CDCl₃) δ 1.11 (d-6H; J = 6.85 Hz), 1.94-2.02 (m-1H), 2.24-2.46 (m-2H), 2.37 (s-3H), 2.77-2.83 (m-3H), 3.14-3.21 (m-1H), 4.45 (bs-2H), 4.80-4.85 (m-1H), 6.38-6.40 (m-1H), 6.62-6.65 (m-2H), 6.84 (d-1H; J = 2.70 Hz), 7.14-7.17 (m-1H), 7.41 (dd-1H; J = 7.47 Hz; J = 8.02 Hz).

### EXAMPLE 42

### 6-[2-ISOPROPYL-4-(2-METHYL-2-AZA-BICYCLO[2.2.1]HEPT-5-YLOXY)-PHENYL]-PYRIDIN-2-YLAMINE

¹H NMR (CDCl₃) δ 1.14 (d-6H), 1.48-1.96 (m-4H), 2.40 (s-3H), 2.44-2.88 (m-2H), 3.03 - 3.06 (m-1H), 3.16-3.23 (m-2H), 4.43 (bs-2H), 4.64 (m-1H), 6.43 (dd-1H; J = 0.83 Hz, J = 8.30 Hz), 6.64-6.70 (m-2H), 6.86 (d-1H; J = 2.49 Hz), 7.17-7.20 (m-1H), 7.41-7.45 (dd-1H; J = 7.47 Hz, J = 8.09 Hz).

The title compounds of Examples 43-75 were prepared using procedures analogous to those described in Example 2.

### EXAMPLE 43

### 6-[4-(2-DIMETHYLAMINO-ETHOXY)-2-METHOXY-PHENYL]-PYRIDIN-2-YLAMINE

¹H NMR (CDCl₃) δ 2.34 (s-6H), 2.74 (t-2H), 3.79 (s-3H), 4.10 (t-2H), 4.49 (bs-2H), 6.38 (dd-1H; J = 8.09 Hz, 0.62 Hz), 6.54-6.58 (m-2H), 7.12 (dd-1H; J = 7.47 Hz, 0.83 Hz), 7.42 (t-1H; J = 7.68 Hz), 7.65 (m-1H).

### EXAMPLE 44

### 6-{4-[2-(BENZYL-METHYL-AMINO)-ETHOXY]-2-METHOXY-PHENYL}-PYRIDIN-2-YLAMINE

¹H NMR (CDCl₃) δ 2.34 (s-3H), 2.84 (t-2H, J = 6.01 Hz), 3.62 (s-2H), 3.79 (s-3H), 4.10 (t-2H; J = 6.01 Hz), 4.51 (bs-1H), 6.36 (d-2H, J = 8.09 Hz), 6.52-6.57 (m-2H), 7.12 (d-2H; J = 7.47 Hz), 7.22-7.36 (m-5H), 7.42 (t-1H; J = 7.89 Hz), 7.65 (d-1H; J = 8.30).

### EXAMPLE 45

### 6-[2-METHOXY-4-(2-PYRROLIDIN-1-YL-ETHOXY)-PHENYL]-PYRIDIN-2-YLAMINE

¹H NMR (CDCl₃) δ 1.78-1.82(m-4H), 2.60-2.65 (m-4H), 2.90 (t-2H; J = 5.82 Hz), 3.79 (s-3H), 4.13 (t-2H, J = 6.02 Hz), 4.44 (bs-2H), 6.37 (d-1H; J = 8.10), 6.55 (s-1H), 6.55-6.57 (m-1H), 7.11 (d-1H; J = 7.48 Hz), 7.39-7.43 (m-1H), 7.64 (d-1H; J = 7.89 Hz),

### EXAMPLE 46

### 2-(6-AMINO-PYRIDIN-2-YL)-5-(2-DIMETHYLAMINO-ETHOXY)-PHENOL

¹H NMR (CDCl₃) δ 2.34 (s-6H), 2.77 (t-2H), 4.09 (t-2H), 6.38-6.47 (m-2H), 7.06 (dd-1H; J = 2.49 Hz, J = 7.68 Hz), 7.46-7.51 ( m-1H), 7.67-7.71 (m-1H).

### EXAMPLE 47

### 2-[4-(6-AMINO-PYRIDIN-2-YL)-3-METHOXY-PHENOXY]-ACETAMIDE

¹H NMR (CD₃OD) δ 3.80 (s-3H), 4.53 (s-2H), 4.87 (bs-4H), 6.45 (d-1H; J = 8.09 Hz), 6.61 (dd-1H; J = 2.08 Hz, J = 8.51 Hz), 6.72 (d-1H; J = 1.87 Hz), 6.87 (d-1H; J = 7.47 Hz), 7.40-7.43 (m-2H).

### EXAMPLE 48

### 6-[4-(2-AMINO-ETHOXY)-2-METHOXY-PHENYL]-PYRIDIN-2-YLAMINE

¹H NMR (CD₃OD) δ 3.08 (t-2H; J = 5.19 Hz), 3.78 (s-3H), 4.87 (bs-4H), 6.45 (dd-1H; J = 0.62 Hz; J = 8.30 Hz), 6.60 (dd-1H; J = 2.28 Hz; J = 8.30 Hz), 6.65 (d-1H; J = 2.28 Hz), 6.87 (dd-1H; J = 0.83; J = 7.47 Hz), 7.40-7.44 (m-2H).

### EXAMPLE 49

### 6-{4-[2-(3,4-DIHYDRO-1H-ISOQUINOLIN-2-YL)-ETHOXY]-2-METHOXY-PHENYL}-PYRIDIN-2-YLAMINE

¹H NMR (CDCl₃) δ 2.86-2.93 (m-4H), 2.98 (t-2H; J = 6.01), 3.77 (s-2H), 3.80 (s-3H), 4.22 (t-2H; J = 6.01 Hz), 6.36 (d-1H; J = 8.09 Hz), 6.57-6.61 (m-2H), 7.01-7.14 (m-5H), 7.42 (t-1H; J = 7.89 Hz), 7.68 ( d-1H; J = 8.50).

### EXAMPLE 50

### 2-[4-(6-AMINO-PYRIDIN-2-YL)-3-METHOXY-PHENOXY]-ETHANOL

¹H NMR (CDCl₃) δ 2.02 (bs-1H), 3.81 (s-3H), 3.81-3.84 (m-2H), 4.05-4.07 (m-2H), 4.55 (bs-1H), 6.40 (dd-1H; J = 0.62 Hz; J = 8.09 Hz), 6.53-6.58 (m-2H), 7.11-7.12 (m-1H), 7.44 (t-1H; J = 7.89 Hz), 7.64 (dd-1H; J = 2.49 Hz; J = 6.64 Hz).

### EXAMPLE 51

### 6-{2-METHOXY-4-[2-(2,2,6,6-TETRAMETHYL-PIPERIDIN-1-YL)-ETHOXY]-PHENYL}-PYRIDIN-2-YLAMINE

¹H NMR (CDCl₃) δ 0.86-1.65 (m-18 H), 2.73 (t-2H, J = 8.30), 3.33 (t-2H, J = 8.71 Hz), 3.82 (s-3H), 6.39 (d-1H; J = 8.30 Hz), 6.52-6.58 (m-2H), 7.13 ( d-1H; J = 7.47 Hz), 7.43 (t-1H, J = 7.47 Hz), 7.65 (d-1H; J = 8.51 Hz).

### EXAMPLE 52

### 6-{4-[2-(2,5-DIMETHYL-PYRROLIDIN-1-YL)-ETHOXY]-2-METHOXY-PHENYL}-PYRIDIN-2-YLAMINE

¹H NMR (CDCl₃) δ 1.12 (d-6H; J = 6.23 Hz), 1.44-1.51 (m-2H), 2.07-2.15 (m-2H), 2.94-3.11 (m-2H), 3.27 (bs-2H), 3.80 (s-3H), 4.15-4.23 (m-2H), 4.52 (bs-2H), 6.38 (d-1H; J = 8.10 Hz), 6.53-6.58 (m-2H), 7.11 (d-1H; J = 7.47 Hz), 7.43 (t-1H; J = 7.26 Hz), 7.64 (d-1H; J = 8.51 Hz).

### EXAMPLE 53

### 6-{4-[2-(2,5-DIMETHYL-PYRROLIDIN-1-YL)-ETHOXY]-2-METHOXY-PHENYL}-PYRIDIN-2-YLAMINE

¹H NMR (CDCl₃) δ 1.19 (d-6H; J = 6.22 Hz), 1.41-1.44 (m-2H), 1.82-1.89 (m-2H), 2.76-2.78 (bs-2H), 3.02 (t-2H; J = 6.64 Hz), 3.80 (s-3H), 4.09 (t-2H; J = 6.64 Hz), 4.53 (bs-2H), 6.38 (d-1H; J = 8.09 Hz), 6.50-6.57 (m-2H), 7.11 (d-1H; J = 7.47 Hz), 7.43 (t-1H; J = 7.26 Hz), 7.64 (d-1H; J = 8.51 Hz)

### EXAMPLE 54

### 2-[4-(6-AMINO-PYRIDIN-2-YL)-3-METHOXY-PHENOXY]-1-(2,2,6,6-TETRAMETHYL-PIPERIDIN-1-YL)-ETHANONE

LR/MS : M+H = 398 (theoretical = 398)

### EXAMPLE 55

### 6-[2-METHOXY-4-(1-METHYL-PYRROLIDIN-2-YLMETHOXY)-PHENYL]-PYRIDIN-2-YLAMINE

¹H NMR (CDCl₃) δ 1.23-2.35 (m-4H), 2.35 (s-3H), 2.65(m-1H), 2.90-2.99 (m-1H), 3.80 (s-3H), 4.46-4.50 (m-2H), 4.76 (bs-2H), 6.40 (dd-1H; J = 0.62 Hz; J = 8.10 Hz), 6.58-6.61 (m-2H), 7.08 (dd-1H; J = 0.81 Hz; J = 7.68 Hz). 7.41-7.46 (m-1H), 7.61 (dd-1H; J = 1.24; J = 8.10 Hz).

### EXAMPLE 56

### 6-[4-(2-DIMETHYLAMINO-ETHOXY)-2-PROPOXY-PHENYL]-PYRIDIN-2-YLAMINE

¹H NMR (CDCl₃) δ 0.97 (t-3H, J = 7.47), 1.71-1.80 (m-2H), 2.33 (s-6H), 2.72 (t-2H; J = 5.60 Hz), 3.90 ( t-2H; J = 6.43 Hz), 4.07 (t-2H; J = 5.60 Hz), 4.45 (bs-2H), 6.36 (dd-1H; J = 0.41 Hz; J = 7.89 Hz), 6.54-6.57 (m-2H), 7.19 (d-1H; J = 7.68 Hz), 7.39 (t-1H; J = 7.47n Hz), 7.70 (d-1H; J = 8.10 Hz).

### EXAMPLE 57

### 6-{4-[2-(BENZYL-METHYL-AMINO)-ETHOXY]-2-PROPOXY-PHENYL}-PYRIDIN-2-YLAMINE

¹H NMR (CDCl₃) δ 0.99 (t-3H; J = 7.47), 1.74-1.82 (m-2H), 2.34 (s-3H), 2.84 (t-2H; J = 6.02 Hz), 3.62 (s-3H), 3.91 ( t-2H; J = 6.52 Hz), 4.11 (t-2H; J = 5.81 Hz), 4.47 (bs-2H), 6.37 (d-1H; J = 7.89 Hz), 6.51-6.56 (m-2H), 7.21-7.44 (m-2H), 7.70 (d-1H; J = 8.10 Hz).

### EXAMPLE 58

### 6-[4-(2-ETHOXY-ETHOXY)-2-METHOXY-PHENYL]-PYRIDIN-2-YLAMINE

¹H NMR (CDCl₃) δ 1.23 (t-3H; J = 7.06 Hz), 3.55-3.61 (m-2H), 3.79 (s-3H), 3.76-3.79 (m-2H), 4.12-4.15 (m-2H), 4.49 (bs-1H), 6.37 (d-1H; J = 8.09 Hz), 6.54-6.56 (m-2H), 7.11 (d-1H; J = 7.47 Hz), 7.41 (dd-1H; J = 8.10 Hz; J = 1.46 Hz), 7.63 (dd-1H; J = 0.63 Hz, J = 7.87 Hz).

### EXAMPLE 59

### 6-[4-(2-DIMETHYLAMINO-ETHOXY)-2-ISOPROPOXY-PHENYL]-PYRIDIN-2-YLAMINE

¹H NMR (CDCl₃) δ 1.26 (d-6H, J = 6.02 Hz), 2.33 (s-6H), 2.72 (t-2H; J = 5.81 Hz), 4.07 (t-2H, J = 5.81 Hz), 4.41-4.47 (m-3H), 6.35 (d-1H; J = 8.09 Hz), 6.53-6.57 (m-2H), 7.20-7.23 (m-1H), 7.39 (t-1H; J = 7.68 Hz), 7.68 (d-1H; J = 8.50 Hz).

### EXAMPLE 60

### 6-[4-(2-ETHOXY-ETHOXY)-2-ISOPROPOXY-PHENYL]-PYRIDIN-2-YLAMINE

¹H NMR (CDCl₃) δ 1.21-1.27 (m-9H), 3.58 (q-2H; J = 6.85 Hz), 3.75-3.78 (m-2H), 4.08-4.13 (m-1H), 4.39-4.47 (m-3H), 6.35 (d-1H; J = 8.09 Hz), 6.55-6.58 (m-2H), 7.22 (d-1H; J = 6.88 Hz), 7.37-7.41 (m-1H), 7.69 (d-1H; J = 7.88 Hz).

### EXAMPLE 61

### 6-[2-METHOXY-4-(3-METHYL-BUTOXY)-PHENYL]-PYRIDIN-2-YLAMINE

¹H NMR (CDCl₃) δ 0.96 (d-6H; J = 6.65 Hz), 1.68 (q-2H; J = 6.86 Hz), 1.80-1.87 (m-1H), 3.81 (s-3H), 4.01 (t-2H; J = 6.65 Hz), 4.42 (bs-2H), 6.37 (dd-1H; J = 0.83 Hz; J = 8.10 Hz), 6.51 (d-1H; J = 2.31 Hz), 6.55 (dd-1H; J = 2.28 Hz; J = 8.52 Hz), 7.13 (dd-1H; J = 0.64 Hz; J = 7.48 Hz), 7.42 (t-1H; J = 7.79 Hz), 7.65 (d-1H; J = 8.51 Hz).

### EXAMPLE 62

### 6-[4-(2-DIMETHYLAMINO-ETHOXY)-2-ETHOXY-PHENYL]-PYRIDIN-2-YLAMINE

¹H NMR (CDCl₃) δ 1.37 Hz ( t-3H; J = 7.05 Hz), 2.34 (s-6H), 2.73 (t-2H; J = 5.60 Hz), 4.02 (q-2H; J = 7.05 Hz), 4.08 (t-2H, J = 5.60 Hz), 4.53 (bs-2H), 6.36-6.38 (m-1H), 6.55-6.58 (m-2H), 7.21 (d-1H; J = 7.68 Hz), 7.39-7.43 (m-1H), 7.71 (d-1H; J = 8.30 Hz).

### EXAMPLE 63

### 6-{4-[2-(BENZYL-METHYL-AMINO)-ETHOXY]-2-ETHOXY-PHENYL}-PYRIDIN-2-YLAMINE

¹H NMR (CDCl₃) δ 1.39 (t-3H; J = 7.06 Hz), 2.35 (s-3H), 2.84 (t-2H; J = 6.02 Hz), 3.62 (s-3H), 4.03 (q-2H, J = 6.84 Hz), 4.12 Hz (t-2H; J = 6.02 Hz), 4.43 (bs-2H), 6.38 (d-1H; J = 8.09 Hz), 6.51 (d-1H; J = 2.08 Hz), 6.55-6.57 (m-1H), 7.23-7.35 (m-5H), 7.42 (t-1H; J = 7.68 Hz), 7.73 (d-1H; J = 8.50 Hz).

### EXAMPLE 64

### 6-[2-ETHOXY-4-(3-METHYL-BUTOXY)-PHENYL]-PYRIDIN-2-YLAMINE

¹H NMR (CDCl₃) δ 0.97 (d-6H, J = 6.64 Hz), 1.39 (t-3H; J = 7.05 Hz), 1.60-1.75 (m-2H), 1.81-1.87 (m-1H), 3.99-4.06 (m-4H), 4.49 (bs-2H), 6.36 (d-1H; J = 7.89 Hz), 6.51 (d-1H; J = 2.08 Hz), 6.57 (dd-1H; J = 2.28 Hz; J = 8.50 Hz), 7.23 (d-1H; J = 7.47 Hz), 7.41 (t-1H; J = 7.68 Hz), 7.73 ( d-1H; J = 8.50 Hz).

### EXAMPLE 65

### 1-(6-AMINO-3-AZA-BICYCLO[3.1.0]HEX-3-YL)-2-[4-(6-AMINO-PYRIDIN-2-YL)-3-ETHOXY-PHENOXY]-ETHANONE

¹H NMR (CD) δ 1.38 (t-3H; J = 6.85 Hz), 2.00-2.20 (m-2H), 2.60-3.90 (m- 6H), 4.13-4.14 (m-2H), 4.77-4.87 (m-4H), 6.62-6.97 (m-4H), 7.44 (d-1H; J = 8.72 Hz), 7.90-7.95 (m-1H).

### EXAMPLE 66

### 6-[2-ETHOXY-4-(2-PYRROLIDIN-1-YL-ETHOXY)-PHENYL]-PYRIDIN-2-YLAMINE

¹H NMR (CDCl₃) δ 1.37 (t-3H; J = 7.05 Hz), 1.76-1.84 (m-4H), 2.57-2.63 (m-4H), 2.89 (t-2H; J = 5.81 Hz), 4.02 (q-2H; J = 5.85 Hz), 4.12 (t-2H, J = 5.81 Hz), 4.44 (bs-2H), 6.36 (d-1H, J = 8.09 Hz), 6.53-6.58 (m-2H), 7.22 (d-1H; J = 7.47 Hz), 7.40 (t-1H; J = 7.68 Hz), 7.71 (d-1H; J = 8.51 Hz).

### EXAMPLE 67

### 3-{2-[4-(6-AMINO-PYRIDIN-2-YL)-3-ETHOXY-PHENOXY]-ETHYL}-3-AZA-BICYCLO[3,1,0]HEX-6-YLAMINE

¹H NMR (CDCl₃) δ 1.37-1.41 (m-5H), 1.78 (bs-2H), 2.47 (d-2H; J = 8.71 Hz), 2.55 (s-1H), 2.76-2.81 (m-2H), 3.05-3.08 (m-2H), 4.00-4.05 (m-4H), 4.47 (bs-2H), 6.35-6.38 (m-1H), 6.52-6.55 (m-2H), 7.20-7.25 (m-1H), 7.39-7.43 (m-1H), 7.69-7.72 (m-1H).

### EXAMPLE 68

### 1-(6-AMINO-3-AZA-BICYCLO[3,1,0]HEX-3-YL)-2-[4-(6-AMINO-PYRIDIN-2-YL)-3-METHOXY-PHENOXY]-ETHANONE

¹H NMR (CD₃OD)-HCl salt δ 2.07-2.20 (m-2H), 2.47 (s-1H), 3.52-3.56 (m-1H), 3.64 (s-3H), 3.73-3.77 (m-1H), 3.88-3.93 (m-2H), 4.77-4.92 (m-2H), 6.71 (d-1H; J = 8.51 Hz), 6.81 (s-1H), 6.89 (d-1H, J = 8.92 Hz), 6.99 (d-1H; J = 7.47 Hz), 7.50 (d-1H; J = 8.71 Hz), 7.93 (d-1H; J = 7.47 Hz).

### EXAMPLE 69

### 3-{2-[4-(6-AMINO-PYRIDIN-2-YL)-3-METHOXY-PHENOXY]-ETHYL}-3-AZA-BICYCLO[3,1,0]HEX-6-YLAMINE

¹H NMR (CDCl₃) δ 1.39 (s-2H), 2.50 (d-2H; J = 8.50 Hz), 2.57 (s-1H), 2.82 (t-2H; J = 6.01 Hz), 3.10 (d-2H; J = 8.90 Hz), 3.81 (s-3H), 4.04 (t-2H; J = 5.61 Hz), 4.45 (bs-1H), 6.39 (d-1H; J = 8,09 Hz), 6.51-6.56 (d-2H), 7.11(d-1H; J = 7.47 Hz), 7.43 (t-1H; J = 7.68 Hz), 7.63 (d-1H; J = 8.30 Hz).

### EXAMPLE 70

### 6-[2-ISOPROPOXY-4-(2-PYRROLIDIN-1-YL-ETHOXY)-PHENYL]-PYRIDIN-2-YLAMINE

¹H NMR (CDCl₃) δ 1.26 (d-6H; J = 6.02 Hz), 1.77-1.84 (m-4H), 2.61-2.65 (m-4H), 2.90 (t-2H; J = 5.81 Hz), 4.41-4.48 (m-3H), 6.35 (d-1H; J = 8.09 Hz), 6.53-6.58 (m-2H), 7.21 (d-1H; J = 7.68 Hz), 7.39 (t-1H; J=7.88 Hz), 7.69 (d-1H; J =8.50 Hz).

### EXAMPLE 71

### 6-{4-[2-(BENZYL-METHYL-AMINO)-ETHOXY]-2-ISOPROPOXY-PHENYL}-PYRIDIN-2-YLAMINE

¹H NMR (CDCl₃) δ 1.27 (d-6H; J = 6.02 Hz), 2.34 (s-3H), 2.83 (t-2H; J = 6.01 Hz), 3.61 (s-2H), 4.10 (t-2H; J = 6.02 Hz), 4.41-4.48 (m-3H), 6.36 (d-1H; J = 8.09 Hz), 6.51-6.57 (m-2H), 7.23-7.34 (m-5 H), 7.41 (t-1H; J = 8.09 Hz), 7.70 (d-1H; J = 8.50 Hz).

### EXAMPLE 72

### 6-[4-(2-DIMETHYLAMINO-ETHOXY)-2-METHOXY-5-PROPYL-PHENYL]-PYRIDIN-2-YLAMINE

¹H NMR (CDCl₃) δ 2.34 (s-6H), 2.74 (t-2H), 3.79 (s-3H), 4.10 (t-2H), 4.49 (bs-2H). 6.38 (dd-1H; J = 8.09 Hz, 0.62 Hz), 6.54-6.58 (m-2H), 7.12 (dd-1H; J = 7.47 Hz, 0.83 Hz), 7.42 (t-1H; J = 7.68 Hz), 7.65 (m-1H).

### EXAMPLE 73

### 6-[5-ALLYL-4-(2-DIMETHYLAMINO-ETHOXY)-2-METHOXY-PHENYL]-PYRIDIN-2-YLAMINE

¹H NMR (CDCl₃) δ 2.38 (s-6H), 2.80 (t-2H; J = 5.81 Hz), 3.33 (d-2H, J = 6.65 Hz), 3.80 (s-3H), 4.13 (t-2H, J = 5.82 Hz), 4.54 (bs-2H), 4.96-5.06 (m-2H), 5.91-6.00 (m-1H), 6.37 (dd-1H; J = 0.62 Hz; J = 8.10 Hz), 6.50 (s-1H), 7.10 (dd-1H; J = 0.62 Hz; J = 8.31 Hz), 7.41 (t-1H; J = 8.10 Hz), 7.49 (s-1H).

### EXAMPLE 74

### 6-[5-ALLYL-2-METHOXY-4-(2-PYRROLIDIN-1-YL-ETHOXY)-PHENYL]-PYRIDIN-2-YLAMINE

¹H NMR (CDCl₃) δ 1.79-1.82 (m-4H), 2.58-2.68 (m-4H), 2.92-2.96 (m-2H), 3.32-3.34 (m-2H), 3.78 (s-3H), 4.14-4.17 (m-2H), 4.41 (bs-2H), 4.94-5.04 (m-2H), 5.90-6.00 (m-1H), 6.35 (dd-1H; J = 0.83 Hz; J = 7.88 Hz), 6.49 (s-1H), 7.10 (dd-1H; J = 0.83 Hz; J = 7.68 Hz), 7.40 (m-1H), 7.48 (s-1H).

### EXAMPLE 75

### 6-[3-ALLYL-4-(2-DIMETHYLAMINO-ETHOXY)-2-METHOXY-PHENYL]-PYRIDIN-2-YLAMINE

¹H NMR (CDCl₃) δ 2.38 (s-6H), 2.80 (t-2H; J = 5.81 Hz), 3.45 (s-3H), 3.45-3.47 (m-2H), 4.12 (t-2H; J = 5.81 Hz), 4.47 (bs-2H), 4.92-4.99 (m-2H), 5.94-6.01 (m-1H), 6.40 (d-1H; J = 8.09 Hz), 6.71 (d-1H: J = 8.50 Hz), 7.15 (d-1H; J = 7.47 Hz), 7.44 (t-1H, J = 7.47 Hz), 7.50 (d-1H; J = 8.72 Hz).

The title compounds of Examples 76-94 were prepared using procedures analogous to those descnbed in Examples 1 and 27-30.

### EXAMPLE 76

### 6-[2-METHOXY-4-(PYRROLIDIN-3-YLOXY)-PHENYL]-PYRIDIN-2-YLAMINE

¹H NMR (CDCl₃) δ 1.92-2.14 (m-3H), 2.85-3.20 (m-3H), 3.79 (s-3H), 4.44 (bs-2H), 4.83-4.86 (m-1H), 6.37 (dd-1H; J = 8.09), 6.47-6.52 (m-2H), 7.12 (d-1H; J = 7.68 Hz), 7.39-7.46 (m-1H), 7.65 (d-1H; J = 8.30 Hz).

### EXAMPLE 77

### 6-[2-METHOXY-4-(1-METHYL-PYRROLIDIN-3-YLOXY)-PHENYL]-PYRIDIN-2-YLAMINE

¹H NMR (CDCl₃) δ 1.96-2.43 (m-3H), 2.38 (s-3H), 2.73-2.86 (m-3H), 3.78 (s-3H), 4.40 (bs-2H), 4.83-4.89 (m-1H), 6.38 (d-1H; J = 8.09), 6.46-6.51 (m-2H), 7.12 (d-1H; J = 7.47 Hz), 7.39-7.44 (m-1H), 7.63 (d-1H; J = 8.50 Hz).

### EXAMPLE 78

### 6-[2-ETHOXY-4-(PYRROLIDIN-3-YLOXY)-PHENYL]-PYRIDIN-2-YLAMINE

Bis HCl salt: ¹H NMR (CD₃OD) δ 1.39-1.43 (m-3H), 2.33-2.39 (m-2H), 3.46-3.51 (m-1H), 3.57-3.65 (-3H), 4.16 (q-2H), 5.33 (bs-1H), 6.73-6.77 (m-1H), 6.90-6.93 (m-1H), 6.97-7.00 (m-1H), 7.50-7.53 (m-1H), 7.91-7.96 (m-1H).

### EXAMPLE 79

### 6-[2-Isopropoxy-4-(pyrrolidin-3-yloxy)-phenyl]-pyridin-2-ylamine

¹H NMR (CDCl₃) δ 1.28 (d-6H; J = 6.02 Hz), 1.97-2.13 (m-2H), 2.82-3.23 (m-4H), 4.41-4.48 (m-3H), 4.85(m-1H), 6.38 (d-1H, J = 7.88 Hz), 6.47-6.52 (m-2H), 7.21-7.25 (m-2H), 7.41 (t-1H; J = 7.89 Hz), 7.68 (d-1H; J = 8.50 Hz).

### EXAMPLE 80

### 6-[2-METHOXY-4-(PIPERIDIN-4-YLOXY)-PHENYL]-PYRIDIN-2-YLAMINE

¹H NMR (CD₃OD) δ 2.04-2.20 (m-4H), 3.27-3.39 (m-2H), 3.58-3.61 ( m-2H), 3.91 (s-3H), 4.84 (m-1H), 6.80-6.98 (m-4H), 7.48-7.52 (m-1H), 7.83-7.93 (m-1H).

### EXAMPLE 81

### 6-[2-METHOXY-4-(2,2,6,6-TETRAMETHYL-PIPERIDIN-4-YLOXY)-PHENYL]-PYRIDIN-2-YLAMINE

¹H NMR (CDCl₃) δ 1.23-1.38 (m-14H), 2.11-2.15 (m-2H), 3.81 (s-3H), 4.43 (m-1H), 4.70-4.75 (m-1H), 6.40 (d-1H; J = 8.08 Hz), 6.51 (d-1H; J = 2.28 Hz), 6.57 (dd-1H; J = 2.29 Hz; J = 8.51 Hz), 7.14 (d-1H; J = 7.47 Hz), 7.44 (t-1H; J = 7.67 Hz), 7.66 (d-1H; J = 8.50 Hz).

### EXAMPLE 82

### 6-[2-ISOPROPOXY-4-(PYRROLIDIN-3-YLOXY)-PHENYL]-PYRIDIN-2-YLAMINE

¹H NMR (CDCl₃) δ 1.27 (d-6H; J = 6.01 Hz), 1.93-2.16 (m-2H), 2.85-3.20 (m-4H), 4.41-4.47 (m-3H), 4.81-4.84 (m-1H), 6.36 (dd-1H, J = 0.83 Hz; J = 8.10 Hz), 6.46 (d-1H; J = 2.08 Hz), 6.51 (dd-1H; J = 1.66 Hz; J = 7.90 Hz), 7.21-7.25 (m-1H), 7.37-7.42 (m-1H), 7.69 (d-1H; J = 8.51 Hz).

### EXAMPLE 83

### 3-[4-(6-AMINO-PYRIDIN-2-YL)-3-METHOXY-PHENOXY]-AZETIDINE-1-CARBOXYLIC ACID TERT-BUTYL ESTER

¹H NMR (CDCl₃) δ 1.43 (s-9H), 3.79 (s-3H), 3.97-4.00 (m-2H), 4.26-4.30 (m-2H), 4.45 (bs-2H), 4.89 (m-1H), 6.28 (dd-1H; J = 2.29 Hz; J = 8.54 Hz), 6.38 (d-1H; J = 8.10 Hz), 6.44 (d-1H; J = 2.28 Hz), 7.10 (d-1H; J = 7.68 Hz), 7.42 (t-1H; J = 7.90 Hz), 7.62 (d-1H; J = 8.51 Hz).

### EXAMPLE 84

### 6-[4-(AZETIDIN-3-YLOXY)-2-METHOXY-PHENYL]-PYRIDIN-2-YLAMINE

¹H NMR (CD₃OD) HCl salt: δ 3.93 (s-3H), 4.15-4.19 (m-2H), 4.57-4.62 (m-2H), 5.26-5.29 (m-1H), 6.57 (dd-1H; J = 2.78 Hz, J = 8.50 Hz), 6.72 (d-1H; J = 2.07 Hz),6.89-6.99 (m-2H), 7.52 (dd-1H; J = 2.28 Hz; J = 8 51 Hz), 7.90-7.95 (m-1H)

### EXAMPLE 85

### 6-[2-METHOXY-4-(1-METHYL-AZETIDIN-3-YLOXY)-PHENYL]-PYRIDIN-2-YLAMINE

¹H NMR (CDCl₃) δ 2.41 (s-3H), 3.09-3.14 (m-2H), 3.79 (s-3H), 3.79-3.87 (m-2H), 4.44 (bs-2H), 4.76-4.81 (m-1H), 6.34-6.44 ( m-2H), 6.52 ( d-1H; J = 2.07 Hz), 7.09-7.12 (m-1H), 7.40-7.44 (m-1H), 7.61-7.65 (m-1H).

### EXAMPLE 86

### 6-[2-ISOPROPOXY-4-(PYRROLIDIN-3-YLOXY)-PHENYL]-PYRIDIN-2-YLAMINE

¹H NMR (CDCl₃) δ 1.27 (d-6H; J = 6.02 Hz), 2.00-2.15 (m-2H), 3.03-3.26 (m-4H), 3.90 (bs-1H), 4.40-4.47 (m-3H), 4.87 (m-1H), 6.38 (dd-1H; J = 0.83 Hz; J = 8.10 Hz), 6.47 -6.52 (m-2H), 7.20 (dd-1H; J = 0.83 Hz, J = 7.68 Hz), 7.24 (d-1H, J = 1.04 Hz), 7.41 (t-1H; J = 8.10 Hz), 7.67 (d-1H; J = 8 31 Hz).

### EXAMPLE 87

### 6-[2-ISOPROPOXY-4-(PYRROLIDIN-3-YLOXY)-PHENYL]-PYRIDIN-2-YLAMINE

¹H NMR (CDCl₃) δ 1.25 (d-6H, J = 6.02 Hz), 1.91-2.13 (m-2H), 2.35 (bs-1H), 2.86-3.19 (m-4H), 4.39-4.45 (m-3H), 4.80-4.83 (m-1H), 6.34-6.36 (m-1H), 6.44 (d-1H; J = 2.28 Hz), 6.49 (dd-1H; J = 2.28 Hz, J = 8.51 Hz), 7.19-7.24 (m-1H), 7.36-7.41 (m-1H), 7.67 (dd-1H; J = 3.53 Hz; J = 8.51 Hz).

### EXAMPLE 88

### 6-[2-METHOXY-4-(PYRROLIDIN-3-YLOXY)-PHENYL]-PYRIDIN-2-YLAMINE

¹H NMR (CD₃OD) HCl salt: δ 2.00-2.10 (m-1H), 2.15-2.25 (m-1H), 3.21-3.64 (m-5H), 3.94 (s-3H), 5.34 (m-1H), 6.78-7.00 (m-4H), 7.54 (d-1H; J = 8.51 Hz), 7.93 (dd-1H; J = 7.68 Hz; J = 8.39 Hz).

### EXAMPLE 89

### 6-[2-METHOXY-4-(1-METHYL-PYRROLIDIN-3-YLOXY)-PHENYL]-PYRIDIN-2-YLAMINE

¹H NMR (CDCl₃) δ 1.98-2.03 (m-1H), 2.27-2.44 (m-2H), 2.38 (s-3H), 2.74-2.86 (m-3H), 3.78 (s-3H), 4.45 (bs-2H), 4.82-4.87 (m-1H), 6.36(dd-1H; J = 0.83 Hz; J = 8.09 Hz), 6.45-6.51 (m-2H), 7.11 (dd-1H; J = 0.62 Hz; J = 7.47 Hz), 7.41 (t-1H; J = 7.83 Hz), 7.63 (d-1H; J = 8.30 Hz).

### EXAMPLE 90

### 6-[2-METHOXY-4-(1-METHYL-PYRROLIDIN-3-YLOXY)-PHENYL]-PYRIDIN-2-YLAMINE

¹H NMR (CDCl₃) δ 1.98-2.03 (m-1H), 2.28-2.44 (m-2H), 2.38 (s-3H), 2.74-2.86 (m-3H), 3.78 (s-3H), 4.43 (bs-2H), 4.84-4.87 (m-1H), 6.37 (dd-1H; J = 0.83 Hz; J = 8.09 Hz), 6.46-6.51 (m-2H), 7.12 (dd-1H; J = 0.83 Hz; J = 7.68 Hz), 7.41 (t-1H; J = 7.68 Hz), 7.63 (d-1H; J = 8.51 Hz).

### EXAMPLE 91

### 6-[2-METHOXY-4-(2-METHYL-2-AZA-BICYCLO[2,2,1]HEPT-5-YLOXY)-PHENYL]-PYRIDIN-2-YLAMINE

¹H NMR (CDCl₃) δ 1.48-1.98 (m-4H), 2.40 (s-3H), 2.61-2.75 (m-2H), 3.05-3.18 (m-2H), 3.80 (s-3H), 4.40 (bs-2H), 4.66-4.70 (m-1H), 6.38 (dd-1H; J = 0.83 Hz; J = 8.09 Hz), 6.50-6.53 (m-2H), 7.13 (dd-1H; J = 0.62 Hz; J = 7.47 Hz), 7.42 (t-1H; J = 7.88 Hz), 7.62-7.64 (m-1H).

### EXAMPLE 92

### 6-[2-METHOXY-4-(1-METHYL-PIPERIDIN-4-YLOXY)-PHENYL]-PYRIDIN-2-YLAMINE

¹H NMR (CDCl₃) δ 1.81-2.03 (m-4H), 2.29 (s-3H), 2.26-2.30 (m-2H), 2.68 (m-2H), 3.79 (s-3H), 4.33-4.43 (m-3H), 6.37 (dd-1H; J = 0.62 Hz; J = 8.10 Hz), 6.51-6.57 (m-2H), 7.11 (dd-1H; J = 0.62 Hz; J = 7.68 Hz), 7.41 (t-1H; J = 7.68 Hz), 7.61 (d-1H; J = 8.52 Hz).

### EXAMPLE 93

### 6-[4-(1-ETHYL-PIPERIDIN-4-YLOXY)-2-METHOXY-PHENYL]-PYRIDIN-2-YLAMINE

¹H NMR (CDCl₃) δ 1.09 (t-3H; J = 7.26 Hz), 1.80-2.31 (m-6H), 2.41 (q-2H), 2.74 (m-2H), 3.79 (s-3H), 4.33-4.42 (m-3H), 6.36 (d-1H; J = 8.09 Hz), 6.51-6.57 (m-2H), 7.11 (d-1H; J = 7.47 Hz), 7.39-7.43 (m-1H), 7.62 -7.64 (m-1H).

### EXAMPLE 94

### 6-[5-ALLYL-2-METHOXY-4-(1-METHYL-PYRROLIDIN-3-YLOXY)-PHENYL]-PYRIDIN-2-YLAMINE

¹H NMR (CDCl₃) δ 2.02-2.05 (m-1H), 2.29-2.34 (m-1H), 2.42 (s-3H), 2.64-2.74 (m-3H), 3.07-3.11 (m-1H), 3.32-3.34 (m-2H), 3.79 (s-3H), 4.45 (bs-2H), 4.86-4.89 (m-1H), 4.95-5.06 (m-2H), 5.91-5.98 (m-1H), 6.36-6.38 (m-2H), 7.09 (dd-1H; J = 0.83 Hz; J = 7.67 Hz), 7.41 (dd-1H; J = 7.68 Hz; J = 8.09 Hz), 7.48 (s-1H).

The title compounds of Examples 95 - 108 were prepared using procesures analogous to those described in Example 14.

### EXAMPLE 95

### 6-[4-(2-DIMETHYLAMINO-ETHOXY)-2,6-DIMETHYL-PHENYL]-PYRIDIN-2-YLAMINE

¹H NMR (CDCl₃) δ 2.03 (s-6H), 2.33 (s-6H), 2.73 (t-2H; J = 5.81 Hz), 4.06 (t-2H; J = 5.81 Hz), 4.54 (bs-2H), 6.39 (dd-1H; J = 0.83 Hz; J = 8.30 Hz), 6.51 (dd-1H; J = 0.62 Hz, J = 7.26 Hz), 6.61 (s-2H), 7.41-7.46 (m-1H).

### EXAMPLE 96

### 6-[2,6-DIMETHYL-4-(3-PIPERIDIN-1-YL-PROPOXY)-PHENYL]-PYRIDIN-2-YLAMINE

¹H NMR (CDCl₃) δ 1.45-1.60 (m-2H), 1.68-1.81 (m-4H), 2.08 (s-6H), 2.52-2.85 (m-6H), 4.01 (t-2H), 4.53 (bs-1H), 6.42 (d-1H), 6.53 (d-1H), 6.60 (s-2H), 7.49 (t-1H).

### EXAMPLE 97

### 6-[2,6-DIMETHYL-4-(2-PYRROLIDIN-1-YL-ETHOXY)-PHENYL]-PYRIDIN-2-YLAMINE

¹H NMR (CDCl₃) δ 1.81-1.90 (m-4H), 2.10 (s-6H), 2.66-2.74 (m-4H), 2.96 (t-2H), 4.14(t-2H), 4.52 (bs-1H), 6.42 (d-1H), 6.56(d-1H), 6.65 (s-2H), 7.47 (t-1H).

### EXAMPLE 98

### 6-{2,6-DIMETHYL-4-[3-(4-METHYL-PIPERAZIN-1-YL)-PROPOXY]-PHENYL}-PYRIDIN-2-YLAMINE

¹H NMR (CDCl₃) δ 1.92-1.99 (m-2H), 2.05 (s-6H), 2.32 (s-3H), 2.52-2.56 (m-6H), 3.99 (t-2H; J = 6.22 Hz), 4.48 (bs-2H), 6.42 (dd-2H, J = 0.83 Hz, J = 8.30 Hz), 6.53 (dd-2H; J = 0.52 Hz; J = 7.26 Hz), 6.61 (s-2H), 7.44-7.48 (m-1H).

### EXAMPLE 99

### 6-[2,6-DIMETHYL-4-(2-MORPHOLIN-4-YL-ETHOXY)-PHENYL}-PYRIDIN-2-YLAMINE

¹H NMR (CDCl₃) δ 2.05 (s-6H), 2.56-2.58 (m-4H), 2.78 (t-2H; J = 5.65 Hz), 3.71-3.74 (m-4H), 4.10 (t-2H; J = 5.60 Hz), 4.54 (bs-2H), 6.41-6.44 (d -1H), 6.53 (d-1H; J = 7.26 Hz), 6.61 (s-2H), 7.44-7.48 (m-1H).

### EXAMPLE 100

### 6-{4-[2-(BENZYL-METHYL-AMINO)-ETHOXY]-2,6-DIMETHYL-PHENYL}-PYRIDIN-2-YLAMINE

¹H NMR (CDCl₃) δ 2.05 (s-6H), 2.33 (s-3H), 2.83 (t-2H, J = 6.01 Hz), 3.63 (s-2H), 4.09 (t-2H; J = 6.01 Hz), 4.49 (bs-2H), 6.42(d-1H), 6.54 (dd-1H; J = 0.62 Hz; J = 7.22 Hz), 6.61 (s-2H), 7.22-7.35 (m-5H), 7.44-7.48 (m-1H).

### EXAMPLE 101

### 2-[4-(6-AMINO-PYRIDIN-2-YL)-3,5-DIMETHYL-PHENOXY]-ACETAMIDE

¹H NMR (CDCl₃) δ 2.08 (s-6H), 4.49 (s-2H), 4.61 (bs-2H), 5.98 (bs-2H), 6.40-6.60 (m-2H), 6.67 (s-2H), 7.45-7.55 (m-1H).

### EXAMPLE 102

### 6-[4-(2-AMINO-ETHOXY)-2,6-DIMETHYL-PHENYL]-PYRIDIN-2-YLAMINE

¹H NMR (CD₃OD) δ 2.02 (s-6H), 3.01 (t-2H; J = 5.18 Hz), 4.00 (t-2H; J = 5.18 Hz), 6.43 (dd-1H; J = 0.83 Hz, J = 7.26 Hz), 6.51 (dd-1H; J = 0.83 Hz; J = 8.52 Hz), 6.67 (s-2H), 7.50 ( dd-1H; J = 7.26 Hz; J = 8.52 Hz).

### EXAMPLE 103

### 6-[2-ISOPROPYL-4-(2-PYRROLIDIN-1-YL-ETHOXY)-PHENYL]-PYRIDIN-2-YLAMINE

¹H NMR **23** (CD₃OD) δ 1.19 (d-6H; J = 6.85 Hz), 2.99 (s-6H), 2.98-3.02 (m-1H), 3.61 (t-2H; J = 4.98 Hz), 4.41 (t-2H; J = 4.77 Hz), 6.68 (d-1H; J = 8.26 Hz), 6.81 (d-1H; J = 8.72 Hz), 6.97 (dd-1H; J = 8.51 Hz; J = 2.49 Hz), 7.09 (d-1H; J = 2.49 Hz), 7.26 (d-1H; J = 8.51 Hz), 7.74-7.78 (m-1H).

### EXAMPLE 104

### 2-(2,5-DIMETHYL-PYRROLIDIN-1-YL)-6-[2-ISOPROPYL-4-(2-PYRROLIDIN-1-YL-ETHOXY)-PHENYL]-PYRIDINE

¹H NMR (CDCl₃) δ 1.17 (d-6H), 1.29 (d-6H), 1.67-1.82 (m-6H), 2.00-2.05 (m-2H), 2.63-2.66 (m-4H), 2.92 (t-2H), 3.51-3.52 (m-1H), 4.05-4.16 (m-4H), 6.30 (d-1H; J = 8.30 Hz), 6.54 (dd-1H; J = 0.62 Hz: J = 7.25 Hz), 6.74-6.77 (m- 1H), 6.95 (dd-1H; J = 1.04 Hz; J = 2.49 Hz), 7.24-7.27 (m-1H), 7.40-7.44 (m-1H).

### EXAMPLE 105

### 6-{4-[2-(3,5-DIMETHYL-PIPERIDIN-1-YL)-ETHOXY]-2-ISOPROPYL-PHENYL)-PYRIDIN-2-YLAMINE

¹H NMR (CDCl₃) δ 0.95 (d-6H; J = 6.64 Hz), 1.15 (d-6H; J = 6.84 Hz), 1.16-1.40 (m-4H), 1.50-2.80 (m-6H), 3.17-3.24 (m-1H), 4.09-4.11 (m-2H), 4.43 (bs-2H), 6.43 (dd-1H; J = 2.70 Hz, J = 8.09 Hz), 6.65 (d-1H; J = 7.26 Hz), 6.76 (dd-1H; J = 2.49 Hz; J = 8.30 Hz), 6.89 (d-1H; J = 2.49 Hz), 7.19-7.22 (m-1H), 7.44 (t-1H; J = 7.89 Hz).

### EXAMPLE 106

### 6-[4-(2-DIMETHYLAMINO-ETHOXY)-2-ISOPROPYL-PHENYL]-PYRIDIN-2-YLAMINE

¹H NMR (CDCl₃) δ 1.12 (d-6H; J = 6.85 Hz), 2.32 (s-6H), 2.72 (t-2H; J = 5.82 Hz), 3.17-3.21 (m-1H), 4.07 (t-2H; J = 5.61 Hz), 4.56 (bs-2H), 6.37 (d-1H; J = 8.10 Hz), 6.61 (d-1H; J = 7.27 Hz), 6.73 (dd-1H; J = 2.70 Hz; J = 8.52 Hz), 6.91 (d-1H; J = 2.70 Hz), 7.18 (d-1H; J = 8.51 Hz), 7.40 (dd-1H; J = 7.27 Hz; J = 7.68 Hz).

### EXAMPLE 107

### 6-[2-TERT-BUTYL-4-(2-DIMETHYLAMINO-ETHOXY)-PHENYL]-PYRIDIN-2-YLAMINE

¹H NMR (CDCl₃) δ 1.19 (s-9H), 2.34 (s-6H), 2.73 (t-2H; J = 5.60 Hz), 4.07 (t-2H, J = 5.81 Hz), 4.44 (bs -2H), 6.39 (d-1H; J = 8.09 Hz), 6.61 (d-1H, J = 7.26 Hz), 6.70 (dd-1H; J = 2.70 Hz; J = 8.51 Hz), 6.98 (d-1H; J = 8.51 Hz), 7.07 (d-1H; J = 2.49 Hz), 7.36-7.40 (m-1H).

### EXAMPLE 108

### 6-[2-TERT-BUTYL-4-(2-PYRROLIDIN-1-YL-ETHOXY)PHENYL]-PYRIDIN-2-YLAMINE

¹H NMR (CDCl₃) δ 1.18 (s-9H), 1.80-1.83 (m-4H), 2.65-2.67 (m-4H), 2.93 (t-2H; J = 5.81 Hz), 4.13 (t-2H; J = 5.81 Hz), 4.47 (bs -2H), 6.38 (d-1H; J = 8.09 Hz), 6.60 (d-1H; J = 7.47 Hz), 6.70 (dd-1H; J = 2.49 Hz; J = 8.30 Hz), 6.98 (d-1H; J = 8.30 Hz), 7.05 (d-1H; J = 2.49 Hz), 7.37 (t-1H; J = 7.68 Hz).

## Claims

1. A compound of the formula 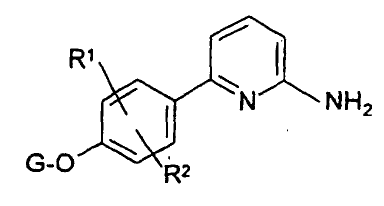 wherein R¹ and R² are selected, independently, from hydrogen, (C₁-₆)alkyl, (C₂-C₆)alkenyl, (C₁-C₆)alkoxy-(C₁-C₃)alkyl, halo, hydroxy, (C₁-C₆)alkoxy, (C₁ - C₇)alkyl, and (C₂ - C₁₀)alkoxyalkyl; and
G is selected from hydrogen, aminocarbonyl-(C₁-C₃)alkyl-, (C₁-C₃) alkylaminocarbonyl-(C₁-C₃) alkyl-, di-[(C₁-C₃)alkyl]aminocarbonyl-(C₁-C₃) alkyl-, and N(R³)(R⁴)(C₀-C₄)alkyl-, wherein R³ and R⁴ are selected, independently, from hydrogen, (C₁-C₇) alkyl, tetrahydronaphthalene and aralkyl, wherein the aryl moiety of said aralkyl is phenyl or naphthyl and the alkyl moiety is straight cyclic or branched and contains from 1 to 6 carbon atoms, and wherein said (C₁-C₇) alkyl and said tetrahydronaphthalene and the aryl moiety of said aralkyl may optionally be substituted with from one to three substituents, preferably from zero to two substituents, that are selected, independently, from halo, nitro, hydroxy, cyano, amino, (C₁-C₄) alkoxy, and (C₁-C₄) alkylamino;
or R³ and R⁴ form, together with the nitrogen to which they are attached, a piperazine, piperidine, morpholine, azetidine or pyrrolidine ring or a saturated or unsaturated azabicyclic ring system containing from 6 to 14 ring members, from 1 to 3 of which are nitrogen, from zero to two of which are oxygen, and the rest of which are carbon;
and wherein said piperazine, piperidine, azetidine and pyrrolidine rings and said azabicyclic ring systems may optionally be substituted with one or more substituents, preferably with from zero to two substituents, that are selected, independently, from (C₁-C₆)alkyl, amino, (C₁-C₆) alkylamino, [di-(C₁-C₆)alkyl]amino, phenyl substituted 5 to 6 membered heterocyclic rings containing from 1 to 4 ring nitrogen atoms, benzoyl, benzoylmethyl, benzylcarbonyl, phenylaminocarbonyl, phenylethyl and phenoxycarbonyl, and wherein the phenyl moieties of any of the foregoing substituents may optionally be substituted with one or more substituents, preferably with from zero to two substituents, that are selected, independently, from halo, (C₁-C₃)alkyl, (C₁-C₃)alkoxy, nitro, amino, cyano, CF₃ and OCF₃;
and wherein said piperazine, piperidine, azetidine and pyrrolidine rings and said azabicyclic ring systems may be attached to -(C₀-C₄)alkyl-O- (wherein the oxygen of said -(C₀-C₄)alkyl-O- is the oxygen atom depicted in structural formula I) at a nitrogen atom of the NR³R⁴ ring or any other atom of the ring having an available bonding site;
or G is a group of the formula A 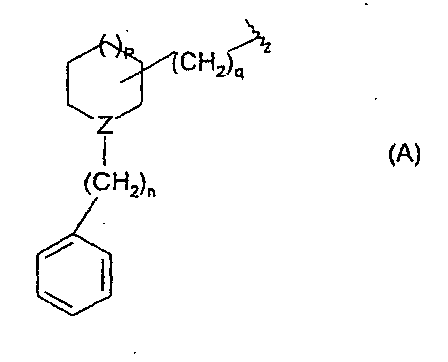 wherein Z is nitrogen or CH, n is zero or one, q is zero, one, two or three and p is zero, one or two;
and wherein the 2-aminopyridine ring depicted in structure I above may optionally be replaced with a 2-amino piperidine ring, as shown below 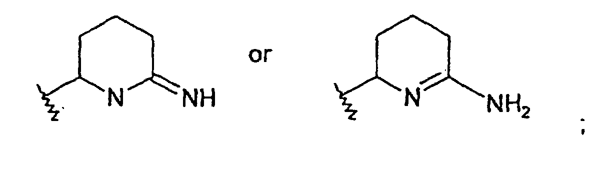 or a pharmaceutically acceptable salt of such compound

2. A compound according to claim 1, wherein G is NR³R⁴(C₀-C₄)alkyl and NR³R⁴ is a piperidine, piperazine or pyrrolidine ring.

3. A compound according to claim 1 wherein R¹ and R² are selected from hydrogen and (C₁-C₂)alkyl.

4. A compound according to claim 1 wherein G is NR³R⁴(C₀-C₄)alkyl and NR³R⁴ is a group of the formula

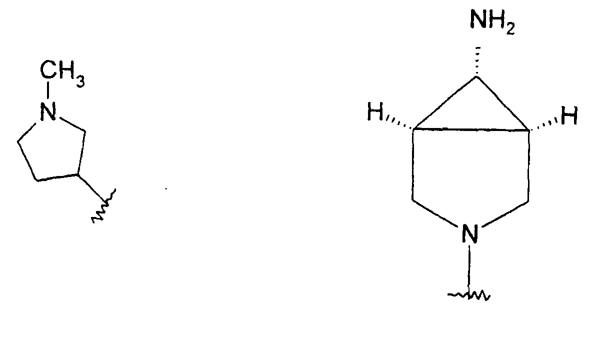

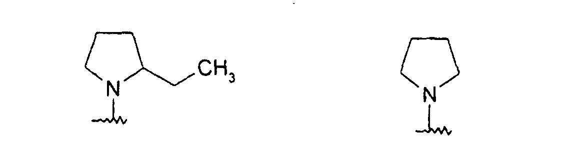

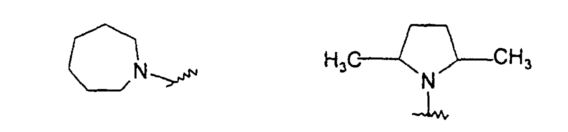

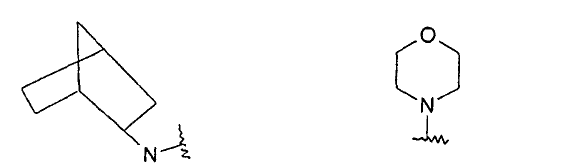

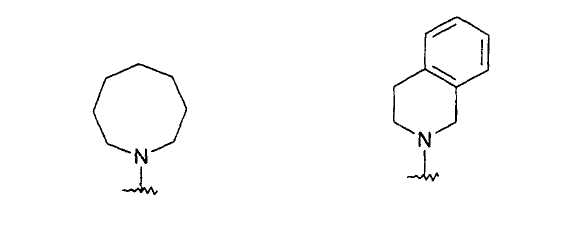

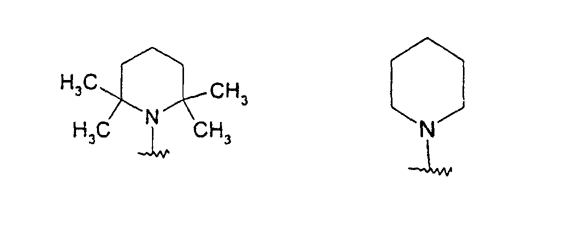

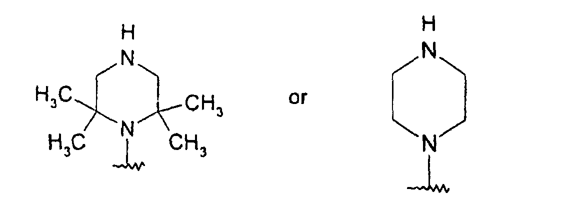

5. A compound according to claim 1 wherein G is a group of the formula A and Z is nitrogen.

6. A compound according to claim 1 wherein G is a group of the formula A, Z is nitrogen, each of n and p is one and q is two.

7. A pharmaceutical composition for treating or preventing a condition selected from the group consisting of migraine inflammatory diseases, stroke, acute and chronic pain, hypovolemic shock, traumatic shock, reperfusion injury, Crohn's disease, ulcerative colitis, septic shock, multiple sclerosis, AIDS associated dementia, neurodegenerative diseases, neuron toxicity, Alzheimer's disease, chemical dependencies and addictions, emesis, epilepsy, anxiety, psychosis, head trauma, adult respiratory distress syndrome (ARDS), morphine induced tolerance and withdrawal symptoms; inflammatory bowel disease, osteoarthritis, rheumatoid arthritis, ovulation, dilated cardiomyopathy, acute spinal cord injury, Huntington's disease, Parkinson's disease, glaucoma, macular degeneration, diabetic neuropathy, diabetic nephsopathy and cancer in a mammal, comprising an amount of a compound according to claim 1 that is effective in treating or preventing such condition and a pharmaceutically acceptable carrier.

8. The use of an effective amount of a compound according to Claim 1 for the preparation of a medicament for the treatment or prevention of a condition selected from the group consisting of migraine, inflammatory diseases, stroke, acute and chronic pain, hypovolemic shock, traumatic shock, reperfusion injury, Crohn's disease, ulcerative colitis, septic shock, multiple sclerosis, AIDS associated dementia, neurodegenerative diseases, neuron toxicity, Alzheimer's disease, chemical dependencies and addictions, emesis, epilepsy, anxiety, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, Parkinson's disease, glaucoma, macular degeneration, diabetic neuropathy, diabetic nephropathy and cancer in a mammal.

9. A pharmaceutical composition for inhibiting nitric oxide synthase (NOS) in a mammal, according to claim 1, comprising a NOS inhibiting effective amount of a compound according to claim 1, and a pharmaceutically acceptable carrier.

10. The use of an effective amount of a compound according to Claim 1 for the preparation of a medicament for the inhibition of NOS in a mammal.

11. A pharmaceutical composition for treating or preventing a condition selected from the group consisting of migraine, inflammatory diseases, stroke, acute and chronic pain, hypovolemic shock, traumatic shock, reperfusion injury, Crohn's disease, ulcerative colitis, septic shock, multiple sclerosis, AIDS associated dementia, neurodegenerative diseases, neuron toxicity, Alzheimer's disease, chemical dependencies and addictions, emesis, epilepsy, anxiety, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, Parkinson's disease, glaucoma, macular degeneration, diabetic neuropathy, diabetic nephrosopathy and cancer in a mammal, comprising a NOS inhibiting effective amount of a compound according to claim 1 and a pharmaceutically acceptable carrier.

12. The use of an effective amount of a compound according to Claim 1 for the preparation of a medicament for the treatment or prevention of a condition selected from the group consisting of migraine, inflammatory diseases, stroke, acute and chronic pain, hypovolemic shock, traumatic shock, reperfusion injury, Crohn's disease, ulcerative colitis, septic shock, multiple sclerosis, AIDS associated dementia, neurodegenerative diseases, neuron toxicity, Alzheimer's disease, chemical dependencies and addictions, emesis, epilepsy, anxiety, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, Parkinson's disease, glaucoma, macular degeneration, diabetic neuropathy, diabetic nephropathy and cancer in a mammal.

13. A compound of the formula 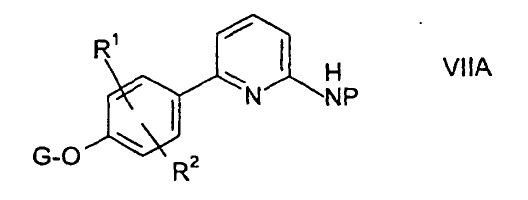 wherein R¹ and R² are selected, independently, from hydrogen, halo, hydroxy, ( C₁-C₆)alkoxy, (C₁-C₇)alkyl, (C₂-C₆)alkenyl, and (C₂ - C₁₀)alkoxyalkyl; and
G is selected from hydrogen, (C₁-C₆)alkyl, (C₁-C₆)alkoxy-(C₁-C₃)alkyl, aminocarbonyl-(C₁-C₃)alkyl-, (C₁-C₃) alkylaminocarbonyl -(C₁-C₃) alkyl-, di-[(C₁-C₃)alkyl]aminocarbonyl-(C₁-C₃)alkyl-, and N(R³)(R⁴)(C₀-C₄)alkyl-, wherein R³ and R⁴ are selected, independently, from hydrogen, (C₁-C₇) alkyl, tetrahydronaphthalene and aralkyl, wherein the aryl moiety of said aralkyl is phenyl or naphthyl and the alkyl moiety is straight or branched and contains from 1 to 6 carbon atoms, and wherein said (C₁-C₇) alkyl and said tetrahydronaphthalene and the aryl moiety of said aralkyl may optionally be substituted with from one to three substituents, that are selected, independently, from halo, nitro, hydroxy, cyano, amino, (C₁-C₄) alkoxy, and (C₁-C₄) alkylamino;
or R³ and R⁴ form, together with the nitrogen to which they are attached, a piperazine, piperidine, azetidine or pyrrolidine ring or a saturated or unsaturated azabicyclic ring system containing from 6 to 14 ring members, from 1 to 3 of which are nitrogen, from zero to two of which are oxygen, and the rest of which are carbon;
and wherein said piperazine, piperidine, azetidine and pyrrolidine rings and said azabicyclic ring systems may optionally be substituted with one or more substituents, preferably with from zero to two substituents, that are selected, independently, from (C₁-C₆)alkyl, amino, (C₁-C₆) alkylamino, [di-(C₁-C₆)alkyl]amino, phenyl substituted 5 to 6 membered heterocyclic rings containing from 1 to 4 ring nitrogen atoms, benzoyl, benzoylmethyl, benzylcarbonyl, phenylaminocarbonyl, phenylethyl and phenoxycarbonyl, and wherein the phenyl moieties of any of the foregoing substituents may optionally be substituted with one or more substituents, that are selected, independently, from halo, (C₁-C₃)alkyl, (C₁-C₃)alkoxy, nitro, amino, cyano, CF₃ and OCF₃;
and wherein said piperazine, piperidine, azetidine and pyrrolidine rings and said azabicyclic ring systems may be attached to -(C₀-C₄)alkyl-O- (wherein the oxygen of said -(C₀-C₄)alkyl-O- is the oxygen atom depicted in structural formula I) at a nitrogen atom of the NR³R⁴ ring or at any other atom of such ring having an available bonding site;
or G is a group of the formula A 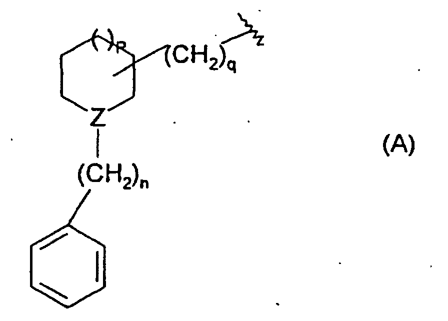 wherein Z is nitrogen or CH, n is zero or one, q is zero, one, two or three and p is zero, one or two;
and wherein the 2-amino pyridine ring depicted in structure I above may optionally be replaced with a 2-aminopiperidine, as shown below 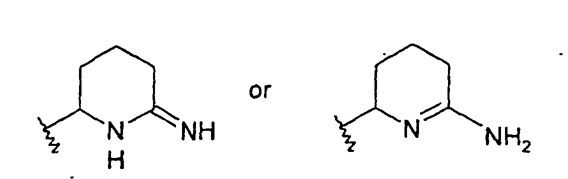 and P is a nitrogen protecting group.

14. A compound of the formula 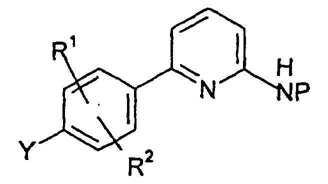 wherein Y is fluoro or benzyloxy;
R¹ and R² are selected, independently, from hydrogen, halo, hydroxy, ( C₁-C₆)alkoxy, (C₁-C₇)alkyl, (C₂-C₆)alkenyl, and (C₂ - C₁₀)alkoxyalkyl; and
and P is a nitrogen protecting group.

## Patentansprüche

1. Verbindung der Formel 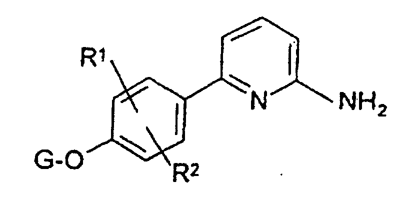 wobei R¹ und R² unabhängig voneinander ausgewählt werden unter Wasserstoff, (C₁-C₆)-Alkyl, (C₂-C₆)-Alkenyl, (C₁-C₆)-Alkoxy-(C₁-C₃)-alkyl, Halo, Hydroxy, (C₁-C₆)-Alkoxy, (C₁-C₇)-Alkyl und (C₂-C₁₀)-Alkoxyalkyl und
G ausgewählt wird unter Wasserstoff, Aminocarbonyl-(C₁-C₃)-alkyl-, (C₁-C₃)-Alkylaminocarbonyl-(C₁-C₃)-alkyl, Di[(C₁-C₃)-Alkyl]-aminocarbonyl-(C₁-C₃)-alkyl- und N(R³)(R⁴)(C₀₋C₄)-Alkyl-, wobei R³ und R⁴ unabhängig voneinander ausgewählt werden unter Wasserstoff, (C₁-C₇)-Alkyl, Tetrahydronaphthalin und Aralkyl, wobei der Arylanteil des Aralkyls Phenyl oder Naphthyl ist und der Alkylanteil gerade, cyclisch oder verzweigt ist und 1 bis 6 Kohlenstoffatome enthält und wobei das (C₁-C₇)-Alkyl und das Tetrahydronaphthalin und der Arylanteil des Aralkyls wahlweise durch einen bis drei Substituenten, bevorzugt null bis zwei Substituenten substituiert sein kann, der bzw. die unabhängig voneinander ausgewählt wird bzw. werden unter Halo, Nitro, Hydroxy, Cyano, Amino, (C₁-C₄)-Alkoxy und (C₁-C₄)-Alkylamino,
oder R³ und R⁴ zusammen mit dem Stickstoff, an den sie angeknüpft sind, einen Piperazin-, Piperidin-, Morpholin-, Azetidin- oder Pyrrolidinring oder ein gesättigtes oder ungesättigtes azabicyclisches Ringsystem, das 6 bis 14 Ringglieder aufweist, bilden, von denen 1 bis 3 Stickstoff, null bis zwei Sauerstoff und der Rest Kohlenstoff sind,
und wobei die Piperazin-, Piperidin-, Azetidin- und Pyrrolidinringe und die azbicyclischen Ringsysteme wahlweise mit einem oder mehreren Substituenten, bevorzugt null bis zwei Substituenten substituiert sein können, der bzw. die unabhängig voneinander ausgewählt wird bzw. werden unter (C₁-C₆)-Alkyl, Amino, (C₁-C₆)-Alkylamino, [Di-(C₁-C₆)-alkyl]-amino, phenylsubstituierten 5- bis 6-gliedrigen heterocyclischen Ringen, die 1 bis 4 Ringstickstoffatome enthalten, Benzoyl, Benzoylmethyl, Benzylcarbonyl, Phenylaminocarbonyl, Phenylethyl und Phenoxycarbonyl und wobei die Phenylanteile irgendeiner der vorhergehenden Substituenten wahlweise durch einen oder mehrere Substituenten, bevorzugt null bis zwei Substituenten substituiert sein können, der bzw. die unabhängig voneinander ausgewählt wird bzw. werden unter Halo, (C₁-C₃)-Alkyl, (C₁-C₃)-Alkoxy, Nitro, Amino, Cyano, CF₃ und OCF₃,
und wobei die Piperazin-, Piperidin-, Azetidin- und Pyrrolidinringe und die azabicyclischen Ringsysteme an - (C₀-C₄)-Alkyl-O- (wobei der Sauerstoff des -(C₀-C₄)-Alkyl)-O-s das in der strukturellen Formel I gezeigte Sauerstoffatom ist) bei einem Stickstoffatom des NR³R⁴-Rings oder irgendeinem anderen Atom des Rings angeknüpft sein können, der eine verfügbare Bindungsstelle aufweist, oder G für eine Gruppe der Formel A 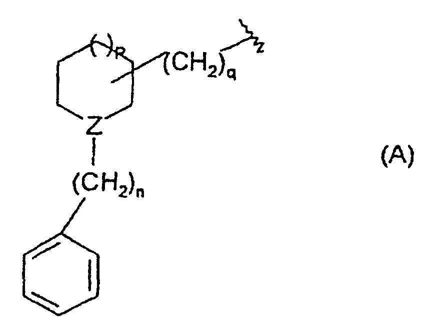 steht, wobei Z für Sauerstoff oder CH steht, n null oder eins beträgt, q null, eins, zwei oder drei beträgt und p null, eins oder zwei beträgt,
und wobei der in der Struktur I oben gezeigte 2-Aminopyridinring wahlweise durch einen 2-Aminopiperidinring, wie er unten gezeigt ist, 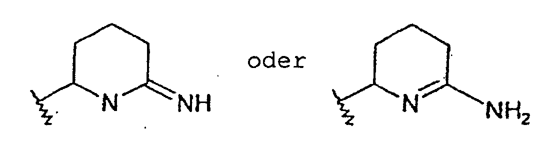 ersetzt werden kann, oder ein pharmazeutisch akzeptables Salz einer derartigen Verbindung.

2. Verbindung nach Anspruch 1, wobei G für NR³R⁴(C₀-C₄)-Alkyl steht und NR³R⁴ für einen Piperidin-, Piperazinoder Pyrrolidinring steht.

3. Verbindung nach Anspruch 1, wobei R¹ und R² unter Wasserstoff und (C₁-C₂)-Alkyl ausgewählt werden.

4. Verbindung nach Anspruch 1, wobei G für NR³R⁴(C₀-C₄)Alkyl steht und NR³R⁴ für eine Gruppe der Formel 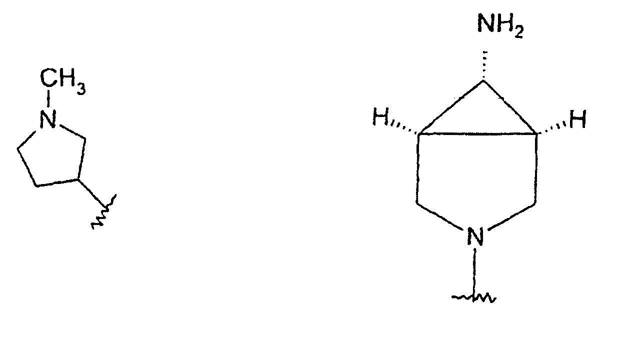 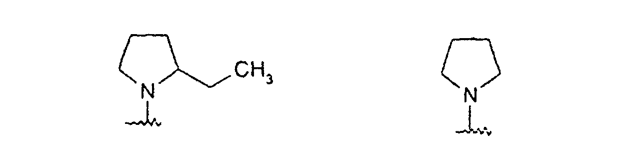 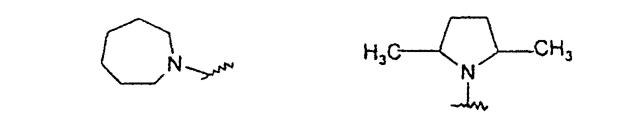 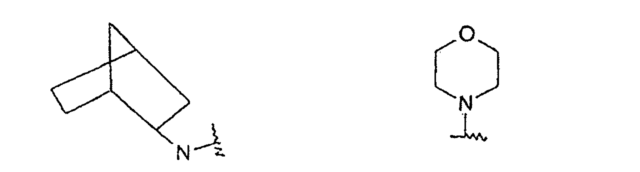 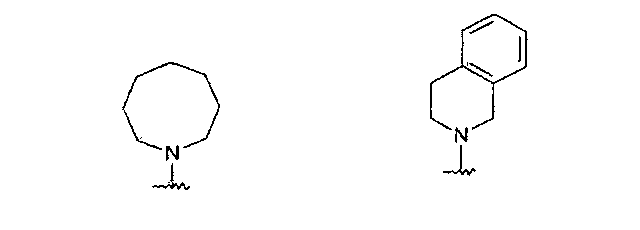 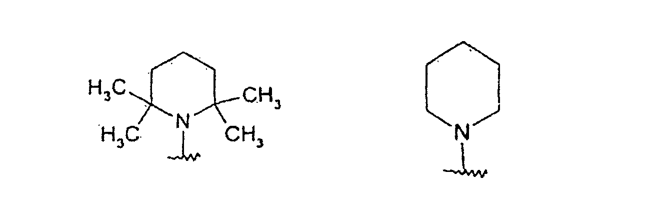 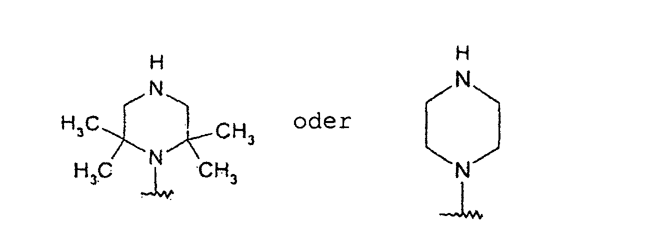 steht.

5. Verbindung nach Anspruch 1, wobei G für eine Gruppe der Formel A und Z für Stickstoff steht.

6. Verbindung nach Anspruch 1, wobei G für eine Gruppe der Formel A steht, Z für Stickstoff steht, jedes von n und p eins und q zwei beträgt.

7. Pharmazeutische Zusammensetzung für die Behandlung oder Verhinderung eines krankhaften Zustands, der aus der Gruppe ausgewählt wird bestehend aus Migräne, entzündlichen Krankheiten, Schlaganfall, akuten und chronischen Schmerzen, hypovolämischem Schock, traumatischem Schock, Reperfusionsschäden, Crohn-Krankheit, Colitis ulcerosa, septischem Schock, multipler Sklerose, mit AIDS assoziierter Demenz, neurodegenerativen Krankheiten, Neuronentoxizität, Alzheimer Krankheit, Drogenabhängigkeit und -süchtigkeit, Emesis, Epilepsie, Angstzuständen, Psychose, Kopftrauma, Schocklunge (ARDS), morphininduzierter Toleranz und Entzugserscheinungen, entzündlicher Darmerkrankung, Osteoarthrose, rheumatoider Arthritis, Ovulation, dilatierter Myokardiopathie, akuter Rückenmarksverletzung, Chorea Huntingdon, Parkinsonsche Krankheit, Glaukom, Makuladegeneration, diabetischer Neuropathie, diabetischer Nephropathie und Krebs bei einem Säuger, umfassend eine Menge einer Verbindung nach Anspruch 1, die bei der Behandlung oder Verhinderung eines derartigen krankhaften Zustands wirksam ist, sowie einen pharmazeutisch akzeptablen Träger.

8. Verwendung einer wirksamen Menge einer Verbindung nach Anspruch 1 für die Zubereitung eines Medikaments für die Behandlung oder Verhinderung eines krankhaften Zustands, der aus der Gruppe ausgewählt wird bestehend aus Migräne, entzündlichen Krankheiten, Schlaganfall, akuten und chronischen Schmerzen, hypovolämischem Schock, traumatischem Schock, Reperfusionsschäden, Crohn-Krankheit, Colitis ulcerosa, septischem Schock, multipler Sklerose, mit AIDS assoziierter Demenz, neurodegenerativen Krankheiten, Neuronentoxizität, Alzheimer Krankheit, Drogenabhängigkeit und -süchtigkeit, Emesis, Epilepsie, Angstzuständen, Psychose, Kopftrauma, Schocklunge (ARDS), morphininduzierter Toleranz und Entzugserscheinungen, entzündlicher Darmerkrankung, Osteoarthrose, rheumatoider Arthritis, Ovulation, dilatierter Myokardiopathie, akuter Rückenmarksverletzung, Chorea Huntingdon, Parkinsonsche Krankheit, Glaukom, Makuladegeneration, diabetischer Neuropathie, diabetischer Nephropathie und Krebs bei einem Säuger

9. Pharmazeutische Zusammensetzung für das Hemmen der Stickstoffoxidsynthase (SOS) bei einem Säuger nach Anspruch 1, umfassend eine für das Hemmen der SOS wirksame Menge einer Verbindung nach Anspruch 1 und einen pharmazeutisch akzeptablen Träger.

10. Verwendung einer wirksamen Menge einer Verbindung nach Anspruch 1 für die Zubereitung eines Medikaments für das Hemmen von SOS bei einem Säuger.

11. Pharmazeutische Zusammensetzung für die Behandlung oder Verhinderung eines krankhaften Zustands, der aus der Gruppe ausgewählt wird bestehend aus Migräne, entzündlichen Krankheiten, Schlaganfall, akuten und chronischen Schmerzen, hypovolämischem Schock, traumatischem Schock, Reperfusionsschäden, Crohn-Krankheit, Colitis ulcerosa, septischem Schock, multipler Sklerose, mit AIDS assoziierter Demenz, neurodegenerativen Krankheiten, Neuronentoxizität, Alzheimer Krankheit, Drogenabhängigkeit und -süchtigkeit, Emesis, Epilepsie, Angstzustände, Psychose, Kopftrauma, Schocklunge (ARDS), morphininduzierter Toleranz und Entzugserscheinungen, entzündlicher Darmerkrankung, Osteoarthrose, rheumatoider Arthritis, Ovulation, dilatierter Myokardiopathie, akuter Rückenmarksverletzung, Chorea Huntingdon, Parkinsonsche Krankheit, Glaukom, Makuladegeneration, diabetischer Neuropathie, diabetischer Nephropathie und Krebs bei einem Säuger, umfassend eine Menge einer Verbindung nach Anspruch 1, die bei der Verhinderung von SOS wirksam ist, sowie einen pharmazeutisch akzeptablen Träger.

12. Verwendung einer wirksamen Menge einer Verbindung nach Anspruch 1 für die Zubereitung eines Medikaments für die Behandlung oder Verhinderung eines krankhaften Zustands, der aus der Gruppe ausgewählt wird bestehend aus Migräne, entzündlichen Krankheiten, Schlaganfall, akuten und chronischen Schmerzen, hypovolämischem Schock, traumatischem Schock, Reperfusionsschäden, Crohn-Krankheit, Colitis ulcerosa, septischem Schock, multipler Sklerose, mit AIDS assoziierter Demenz, neurodegenerativen Krankheiten, Neuronentoxizität, Alzheimer Krankheit, Drogenabhängigkeit und -süchtigkeit, Emesis, Epilepsie, Angstzustände, Psychose, Kopftrauma, Schocklunge (ARDS), morphininduzierter Toleranz und Entzugserscheinungen, entzündlicher Darmerkrankung, Osteoarthrose, rheumatoider Arthritis, Ovulation, dilatierter Myokariopathie, akuter Rückenmarksverletzung, Chorea Huntingdon, Parkinsonsche Krankheit, Glaukom, Makuladegeneration, diabetischer Neuropathie, diabetischer Nephropathie und Krebs bei einem Säuger.

13. Verbindung der Formel 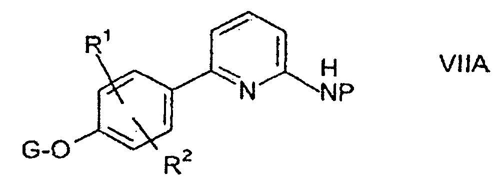 wobei R¹ und R² unabhängig voneinander ausgewählt werden unter Wasserstoff, Halo, Hydroxy, (C₁-C₆)-Alkoxy, (C₁-C₇)-Alkyl, (C₂-C₆)-Alkenyl und (C₂-C₁₀)-Alkoxyalkyl und G ausgewählt wird unter Wasserstoff, (C₁-C₆)-Alkyl, (C₁-C₆)-Alkoxy- (C₁-C₃)-alkyl, Aminocarbonyl-(C₁-C₃)-alkyl-, (C₁-C₃)-Alkylaminocarbonyl-(C₁-C₃)-alkyl, Di[(C₁-C₃)-alkyl]-aminocarbonyl-(C₁-C₃)-alkyl- und N(R³)(R⁴) (C₀₋C₄)-Alkyl-, wobei R³ und R⁴ unabhängig voneinander ausgewählt werden unter Wasserstoff, (C₁-C₇)-Alkyl, Tetrahydronaphthalin und Aralkyl, wobei der Arylanteil des Aralkyls Phenyl oder Naphthyl ist und der Alkylanteil gerade oder verzweigt ist und 1 bis 6 Kohlenstoffatome enthält, und wobei das (C₁-C₇)-Alkyl und das Tetrahydronaphthalin und der Arylanteil des Aralkyls wahlweise durch einen bis drei Substituenten substituiert sein kann, der bzw. die unabhängig voneinander ausgewählt wird bzw. werden unter Halo, Nitro, Hydroxy, Cyano, Amino, (C₁-C₄)-Alkoxy und (C₁-C₄)-Alkylamino, oder R³ und R⁴ zusammen mit dem Stickstoff, an das sie angeknüpft sind, einen Piperazin-, Piperidin-, Azetidinoder Pyrrolidinring oder ein gesättigtes oder ungesättigtes azabicyclisches Ringsystem, das 6 bis 14 Ringglieder aufweist, bilden, von denen 1 bis 3 Stickstoff, null bis zwei Sauerstoff und der Rest Kohlenstoff sind,
und wobei die Piperazin-, Piperidin-, Azetidin- und Pyrrolidinringe und die azabicyclischen Ringsysteme wahlweise mit einem oder mehreren Substituenten, bevorzugt null bis zwei Substituenten substituiert sein können, der bzw. die unabhängig voneinander ausgewählt wird bzw. werden unter (C₁-C₆)-Alkyl, Amino, (C₁-C₆)-Alkylamino, [Di-(C₁-C₆)-alkyl]-amino, phenylsubstituierten 5- bis 6-gliedrigen heterocyclischen Ringen, die 1 bis 4 Ringstickstoffatome enthalten, Benzoyl, Benzoylmethyl, Benzylcarbonyl, Phenylaminocarbonyl, Phenylethyl und Phenoxycarbonyl und wobei die Phenylanteile irgendeiner der vorherigen Substituenten wahlweise durch einen oder mehrere Substituenten substituiert sein können, der bzw. die unabhängig voneinander ausgewählt wird bzw. werden unter Halo, (C₁-C₃)-Alkyl, (C₁-C₃)-Alkoxy, Nitro, Amino, Cyano, CF₃ und OCF₃,
und wobei die Piperazin-, Piperidin-, Azetidin- und Pyrrolidinringe und die azabicyclischen Ringsysteme an (C₀-C₄)-Alkyl-O- (wobei der Sauerstoff des (C₀-C₄)-Alkyl-O-s das in der strukturellen Formel I gezeigte Sauerstoffatom ist) bei einem Stickstoffatom des NR³R⁴-Rings oder irgendeinem anderen Atom eines derartigen Rings angeknüpft sein können, der eine verfügbare Bindungsstelle aufweist,
oder G einer Gruppe der Formel A ist 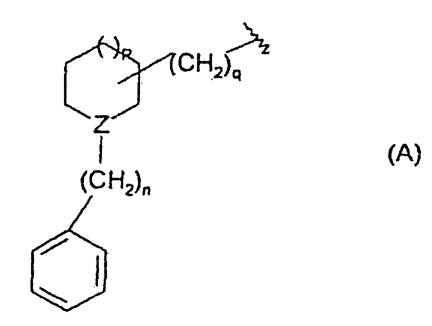 wobei Z für Sauerstoff oder CH steht, n null oder eins beträgt, q null, ein, zwei oder drei beträgt und p null, eins oder zwei beträgt,
und wobei der in der Struktur I oben gezeigte 2-Aminopyridinring wahlweise durch einen 2-Aminopiperidinring, wie er unten gezeigt ist, 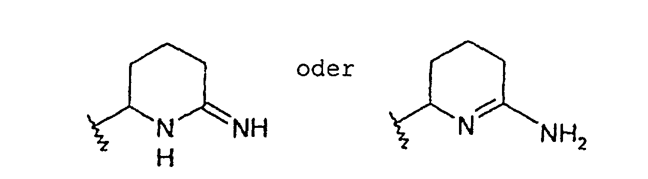 ersetzt werden kann und P für eine Stickstoffschutzgruppe steht.

14. Verbindung der Formel 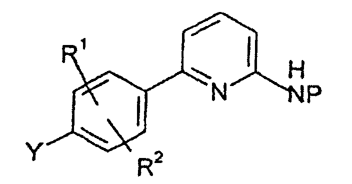 wobei Y für Fluor oder Benzyloxy steht,
R¹ und R² unabhängig voneinander ausgewählt werden unter Wasserstoff, Halo, Hydroxy, (C₁-C₆)-Alkoxy, (C₁-C₇)-Alkyl, (C₂-C₆)-Alkenyl und (C₂-C₁₀)-Alkoxyalkyl und
P für eine Stickstoffschutzgruppe steht.

## Revendications

1. Composé de la formule 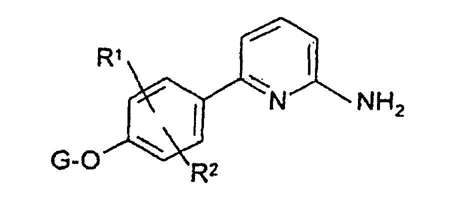 dans laquelle R¹ et R² sont sélectionnés, indépendamment, parmi l'hydrogène, les radicaux (C₁-C₆)alkyle, (C₂-C₆)alcényle, (C₁-C₆) alkoxy-(C₁-C₃)alkyle, halo, hydroxy, (C₁-C₆)alkoxy, (C₁-C₇)alkyle, et (C₂-C₁₀)alkoxyalkyle ; et
G est sélectionné parmi l'hydrogène, les radicaux aminocarbonyl-(C₁-C₃)alkyle, (C₁-C₃)alkylaminocarbonyl-(C₁-C₃)alkyle, di- [(C₁-C₃)alkyl] aminocarbonyl- (C₁-C₃)alkyle et N(R³)(R⁴)(C₀-C₄)alkyle, où R³ et R⁴ sont sélectionnés, indépendamment, parmi l'hydrogène, les radicaux (C₁-C₇)alkyle, tétrahydronaphthalène et aralkyle, où la fraction aryle dudit radical aralkyle est un radical phényle ou naphtyle et la fraction alkyle est linéaire, cyclique ou ramifiée et contient de 1 à 6 atomes de carbone et où ledit radical (C₁-C₇)alkyle et ledit tétrahydronaphthalène et la fraction aryle dudit radical aralkyle peuvent être substitués en option par de un à trois substituants, de préférence, de zéro à deux substituants, qui sont sélectionnés, indépendamment, parmi les radicaux halo, nitro, hydroxy, cyano, amino, (C₁-C₄)alkoxy, et (C₁-C₄)alkylamino ;
ou R³ et R⁴ forment, ensemble avec l'azote auquel ils sont attachés, un cycle pipérazine, pipéridine, morpholine, azétidine ou pyrrolidine ou un système cyclique azabicyclique, saturé ou insaturé, contenant de 6 à 14 membres cycliques, dont de 1 à 3 sont l'azote, dont de zéro à deux sont l'oxygène, et dont le reste sont le carbone ;
et dans laquelle lesdits cycles pipérazine, pipéridine, azétidine et pyrrolidine et lesdits systèmes cycliques azabicycliques peuvent être substitués en option par un ou plusieurs substituants, de préférence, de zéro à deux substituants, qui sont sélectionnées, indépendamment, parmi les radicaux (C₁-C₆)alkyle, amino, (C₁-C₆)alkylamino, [di-(C₁-C₆)alkyl]amino, des cycles hétérocycliques substitués avec un radical phényle, ayant de 5 to 6 membres, contenant de 1 à 4 atomes d'azote cyclique, les radicaux benzoyle, benzoylméthyle, benzylcarbonyle, phénylaminocarbonyle, phényléthyle et phénoxycarbonyle, et où les fractions phényles de l'un quelconque des substituants ci-dessus peuvent être substituées en option par un ou plusieurs substituants, de préférence, de zéro à deux substituants, qui sont sélectionnés, indépendamment parmi les radicaux halo, (C₁-C₃)alkyle, (C₁-C₃)alkoxy, nitro, amino, cyano, CF₃ et OCF₃ ;
et dans laquelle lesdits cycles pipérazine, pipéridine, azétidine et pyrrolidine et lesdits systèmes cycliques azabicycliques peuvent être attachés au radical -(C₀-C₄)alkyl-O- (dans lequel ledit oxygène dudit radical -(C₀-C₄)alkyl-O- est l'atome d'oxygène illustré dans la formule structurelle I) à un atome d'azote du cycle NR³R⁴ ou à tout autre atome du cycle ayant un site de liaison disponible ;
ou G est un groupement de la formule A : 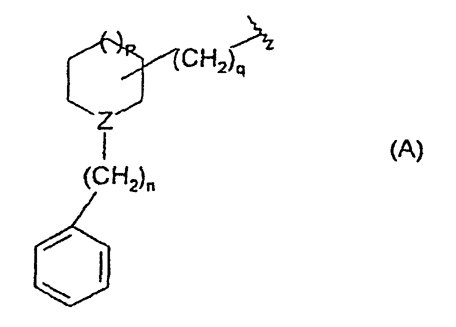 dans laquelle Z est l'azote ou CH, n est zéro ou un, q est zéro, un, deux ou trois et p est zéro, un ou deux ;
et dans laquelle le cycle 2-aminopyridine, illustré dans la structure I, ci-dessus peut être remplacé en option par un cycle 2-aminopipéridine, comme indiqué ci-dessous 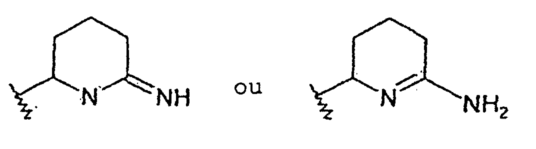 ou un sel, acceptable du point de vue pharmaceutique, d'un composé de ce genre.

2. Composé selon la revendication 1, dans lequel G est le radical NR³R⁴(C₀-C₄)alkyle et dans lequel NR³R⁴ est un cycle pipéridine, pipérazine ou pyrrolidine.

3. Composé selon la revendication 1, dans laquelle R¹ et R² sont sélectionnés parmi l'hydrogène et le radical (C₁-C₂)alkyle.

4. Composé selon la revendication 1, dans lequel G est le radical NR³R⁴(C₀-C₄)alkyle et dans lequel NR³R⁴ est un groupement de la formule

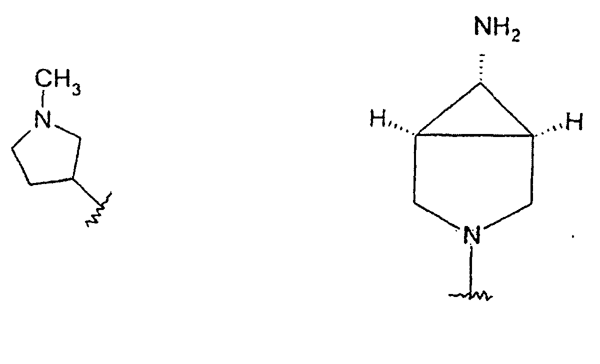

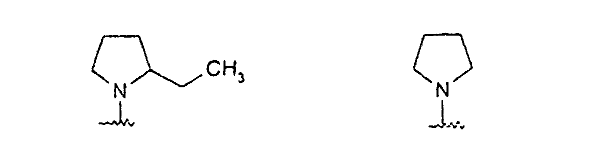

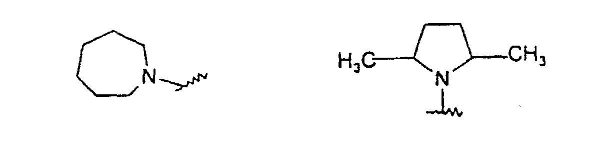

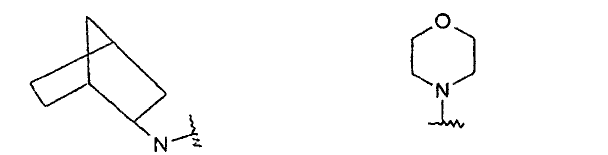

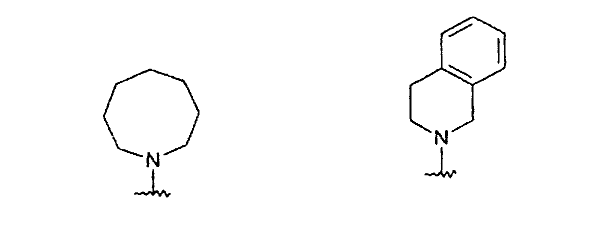

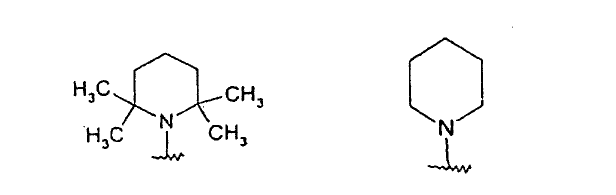

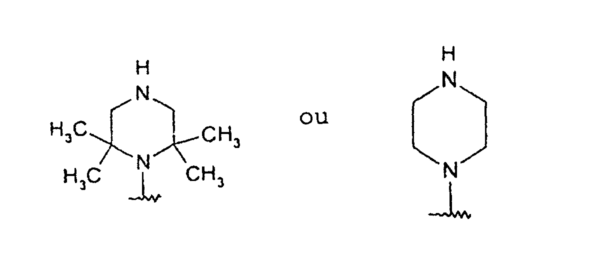

5. Composé selon la revendication 1, dans lequel G est un groupement de la formule A et Z est l'azote.

6. Composé selon la revendication 1, dans lequel G est un groupement de la formule A, Z est l'azote et chacun de n et de p est un, et q est deux.

7. Composition pharmaceutique pour le traitement ou la prévention d'un trouble sélectionné parmi le groupe constitué de la migraine, des troubles inflammatoires, de l'apoplexie, des douleurs aiguës et chroniques, du choc hypovolémique, du choc traumatique, des lésions consécutives à la perfusion, de la maladie de Crohn, de la rectocolite hémorragique, du choc septique, de la sclérose en plaques, de la démence associée au SIDA, des maladies neuro dégénératives, de la toxicité neuronale, de la maladie d'Alzheimer, des dépendances et des addictions chimiques, des vomissements, de l'épilepsie, de l'anxiété, de la psychose, du traumatisme crânien, du syndrome de détresse respiratoire adulte (abrégé en anglais par ARDS), de la tolérance induite par la morphine et des symptômes de sevrage ; de la maladie intestinale inflammatoire, de l'ostéoarthrite, de l'arthrite rhumatoïde, de l'ovulation, de la cardiomyopathie dilatée, des lésions aiguës de la moelle épinière, de la maladie de Huntington, de la maladie de Parkinson, du glaucome, de la dégénérescence maculaire, de la neuropathie diabétique, de la néphropathie diabétique et du cancer chez un mammifère, comprenant une quantité d'un composé selon la revendication 1, qui est efficace dans le traitement ou la prévention d'un trouble de ce genre et un excipient acceptable du point de vue pharmaceutique.

8. Utilisation d'une quantité efficace d'un composé selon la revendication 1, en vue de la préparation d'un médicament pour le traitement ou la prévention d'un trouble sélectionné parmi le groupe constitué de la migraine, des troubles inflammatoires, de l'apoplexie, des douleurs aiguës et chroniques, du choc hypovolémique, du choc traumatique, des lésions consécutives à la perfusion, de la maladie de Crohn, de la rectocolite hémorragique, du choc septique, de la sclérose en plaques, de la démence associée au SIDA, des maladies neuro dégénératives, de la toxicité neuronale, de la maladie d'Alzheimer, des dépendances et des addictions chimiques, des vomissements, de l'épilepsie, de l'anxiété, de la psychose, du traumatisme crânien, du syndrome de détresse respiratoire adulte (ARDS), de la tolérance induite par la morphine et des symptômes de sevrage, de la maladie intestinale inflammatoire, de l'ostéoarthrite, de l'arthrite rhumatoïde, de l'ovulation, de la cardiomyopathie dilatée, des lésions aiguës de la moelle épinière, de la maladie de Huntington, de la maladie de Parkinson, du glaucome de la dégénérescence maculaire, de la neuropathie diabétique, de la néphropathie diabétique et du cancer chez un mammifère.

9. Composition pharmaceutique en vue de l'inhibition de la synthase de l'oxyde nitrique (NOS) chez un mammifère, selon la revendication 1, comprenant une quantité efficace, inhibant la NOS, d'un composé selon la revendication 1, et un excipient acceptable du point de vue pharmaceutique.

10. Utilisation d'une quantité efficace d'un composé selon la revendication 1, en vue de la préparation d'un médicament pour l'inhibition de la NOS chez un mammifère.

11. Composition pharmaceutique pour le traitement ou la prévention d'un trouble sélectionné du groupe constitué de la migraine, des troubles inflammatoires, de l'apoplexie, des douleurs aiguës et chroniques, du choc hypovolémique, du choc traumatique, des lésions consécutives à la perfusion, de la maladie de Crohn, de la rectocolite hémorragique, du choc septique, de la sclérose en plaques, de la démence associée au SIDA, des maladies neuro dégénératives, de la toxicité neuronale, de la maladie d'Alzheimer, des dépendances et des addictions chimiques, des vomissements, de l'épilepsie, de l'anxiété, de la psychose, du traumatisme crânien, du syndrome de détresse respiratoire adulte (ARDS), de la tolérance induite par la morphine et des symptômes de sevrage, de la maladie intestinale inflammatoire, de l'ostéoarthrite, de l'arthrite rhumatoïde, de l'ovulation, de la cardiomyopathie dilatée, des lésions aiguës de la moelle épinière, de la maladie de Huntington, de la maladie de Parkinson, du glaucome, de la dégénérescence maculaire, de la neuropathie diabétique, de la néphropathie diabétique et du cancer chez un mammifère, comprenant une quantité efficace, inhibibant la NOS, d'un composé selon la revendication 1 et un excipient, acceptable du point de vue pharmaceutique.

12. Utilisation d'une quantité efficace d'un composé selon la revendication 1, en vue de la préparation d'un médicament pour le traitement ou la prévention d'un trouble sélectionné du groupe constitué de la migraine, des troubles inflammatoires, de l'apoplexie, des douleurs aiguës et chroniques, du choc hypovolémique, du choc traumatique, des lésions consécutives à la perfusion, de la maladie de Crohn, de la rectocolite hémorragique, du choc septique, de la sclérose en plaques, de la démence associée au SIDA, des maladies neuro dégénératives, de la toxicité neuronale, de la maladie d'Alzheimer, des dépendances et des addictions chimiques, des vomissements, de l'épilepsie, de l'anxiété, de la psychose, du traumatisme crânien, du syndrome de détresse respiratoire adulte (ARDS), de la tolérance induite par la morphine et des symptômes de sevrage, de la maladie intestinale inflammatoire, de l'ostéoarthrite, de l'arthrite rhumatoïde, de l'ovulation, de la cardiomyopathie dilatée, des lésions aiguës de la moelle épinière, de la maladie de Huntington, de la maladie de Parkinson, du glaucome, de la dégénérescence maculaire, de la neuropathie diabétique, de la néphropathie diabétique et du cancer chez un mammifère.

13. Composé de la formule 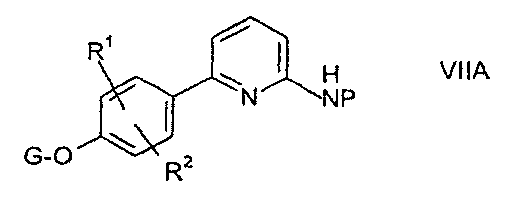 dans laquelle R¹ et R² sont sélectionnés, indépendamment, parmi l'hydrogène, les radicaux halo, hydroxy, (C₁-C₆)alkoxy, (C₁-C₇)alkyle, (C₂-C₆)alcényle, et (C₂-C₁₀)alkoxyalkyle ; et
G est sélectionnée parmi l'hydrogène, les radicaux (C₁-C₆)alkyle, (C₁-C₆) alkoxy-(C₁-C₃)alkyle, aminocarbonyl-(C₁-C₃)alkyle, (C₁-C₃)alkylaminocarbonyl-(C₁-C₃)alkyle, [di-[(C₁-C₃)alkyl]aminocarbonyl-(C₁-C₃)alkyle, et N(R³) (R⁴) (C₀-C₄)alkyl-, où R³ et R⁴ sont sélectionnés, indépendamment, parmi l'hydrogène, les radicaux (C₁-C₇)alkyle, tétrahydronaphthalène et aralkyle, où la fraction aryle dudit radical aralkyle est un radical phényle ou naphtyle et la fraction alkyle est linéaire ou ramifiée et contient de 1 à 6 atomes de carbone et où ledit radical (C₁-C₇)alkyle et ledit tétrahydronaphthalène et la fraction aryle dudit radical aralkyle peuvent être substitués en option par de un à trois substituants, qui sont sélectionnés, indépendamment, parmi les radicaux halo, nitro, hydroxy, cyano, amino, (C₁-C₄)alkoxy, et (C₁-C₄)alkylamino ;
ou R³ et R⁴ forment, pris ensemble avec l'azote auquel ils sont attachés, un cycle pipérazine, pipéridine, azétidine ou pyrrolidine ou un système cyclique azabicyclique, saturé ou non saturé, contenant de 6 à 14 membres cycliques, dont de 1 à 3 sont l'azote, de zéro à deux sont l'oxygène et dont le restant sont le carbone ;
et dans laquelle lesdits cycles pipérazine, pipéridine, azétidine et pyrrolidine et lesdits systèmes cycliques azabicycliques peuvent être substitués en option par un ou plusieurs substituants, de préférence, de zéro à deux substituants, qui sont sélectionnés, indépendamment, parmi les radicaux (C₁-C₆)alkyle, amino, (C₁-C₆) alkylamino, [di-(C₁-C₆)alkyl] amino, des cycles hétérocycliques, substitués avec un radical phényle, ayant de 5 to 6 membres, contenant de 1 à 4 atomes d'azote cyclique, les radicaux benzoyle, benzoylméthyle, benzylcarbonyle, phénylaminocarbonyle, phényléthyle et phénoxycarbonyle, et où les fractions phényles de l'un quelconque des substituants ci-dessus peuvent être substituées en option par un ou plusieurs substituants, qui sont sélectionnés, indépendamment, parmi les radicaux halo, (C₁-C₃)alkyle, (C₁-C₃)alkoxy, nitro, amino, cyano, CF₃ et OCF₃ ;
et dans laquelle lesdits cycles pipérazine, pipéridine, azétidine et pyrrolidine et lesdits systèmes cycliques azabicycliques peuvent être attachés au radical -(C₀-C₄)alkyl-O- (dans lequel l'oxygène dudit radical -(C₀-C₄)alkyl-O- est l'atome d'oxygène illustré dans la formule structurelle I) à un atome d'azote du cycle NR³R⁴ ou à tout autre atome d'un cycle de ce genre ayant un site de liaison disponible ;
ou G est un groupement de la formule A 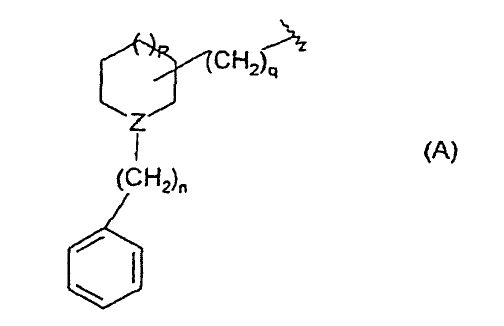 dans laquelle Z est l'azote ou CH, n est zéro ou un, q est zéro, un, deux ou trois, et p est zéro, un ou deux ;
et dans laquelle le cycle 2-aminopyridine, illustré dans la structure I ci-dessus peut être remplacé en option par une 2-aminopipéridine, comme indiqué ci-dessous 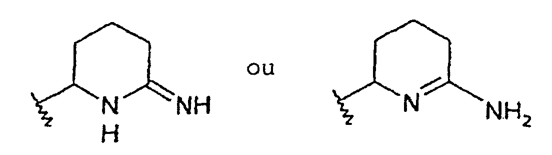 et P est un groupement protecteur de l'azote.

14. Composé de la formule 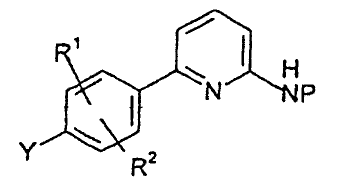 dans laquelle Y est le radical fluoro ou benzyloxy ;
R¹ et R² sont sélectionnés, indépendamment, parmi l'hydrogène, les radicaux halo, hydroxy, (C₁-C₆)alkoxy, (C₁-C₇)alkyle, (C₂-C₆)alcényle, et (C₂-C₁₀)alkoxyalkyle ; et
P est un groupement protecteur de l'azote.
